(19) **Europäisches Patentamt**
**European Patent Office**
**Office européen des brevets**

(11) **EP 3 474 832 B1**

(12) **EUROPEAN PATENT SPECIFICATION**

(45) Date of publication and mention
of the grant of the patent:
**18.03.2026 Bulletin 2026/12**

(21) Application number: **17736843.8**

(22) Date of filing: **23.06.2017**

(51) International Patent Classification (IPC):
**A61K 9/72** *(2006.01)*  **A61K 9/16** *(2006.01)*
**A61P 11/00** *(2006.01)*  **A61K 31/685** *(2006.01)*

(52) Cooperative Patent Classification (CPC):
**A61K 9/0082; A61K 9/0075; A61K 9/1617;
A61K 9/1623; A61K 9/1658; A61P 11/00;
A61P 11/06; A61P 11/16; A61P 31/04; A61P 31/18;
A61P 43/00**

(86) International application number:
**PCT/US2017/039094**

(87) International publication number:
**WO 2017/223502 (28.12.2017 Gazette 2017/52)**

(54) **SURFACTANT FORMULATIONS FOR INHALATION**

TENSIDFORMULIERUNGEN ZUR INHALATION

FORMULATIONS TENSIOACTIVES DESTINÉES À L'INHALATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **24.06.2016 US 201662354382 P
11.11.2016 US 201662420932 P**

(43) Date of publication of application:
**01.05.2019 Bulletin 2019/18**

(73) Proprietor: **Merz Pharmaceuticals, LLC
Raleigh, NC 27615 (US)**

(72) Inventors:
• **LIPP, Michael, M.
Framingham, MA 01710 (US)**
• **KAMERKAR, Abhijit
Salem, MA 01970 (US)**
• **GILANI, Fahad
Wayland, MA 01778 (US)**
• **CHAN, Holly
Braintree, MA 02148 (US)**
• **TAUBER, Michael
Allston, MA 02134 (US)**

(74) Representative: **Latscha Schöllhorn Partner AG
Grellingerstrasse 60
4052 Basel (CH)**

(56) References cited:
**WO-A1-01/58423    WO-A1-2009/026434
WO-A1-97/26863    WO-A2-01/13891
WO-A2-01/13893**

• **KAIALY WASEEM ED - BLANCO-PRIETO MARIA
J ET AL: "A review of factors affecting
electrostatic charging of pharmaceuticals and
adhesive mixtures for inhalation",
INTERNATIONAL JOURNAL OF
PHARMACEUTICS, ELSEVIER, NL, vol. 503, no.
1, 2 February 2016 (2016-02-02), pages 262 - 276,
XP029475923, ISSN: 0378-5173, DOI: 10.1016/
J.IJPHARM.2016.01.076**

**Description**

<u>RELATED APPLICATIONS</u>

This application claims the benefit of U.S. Provisional Application No. 62/354,382, filed on June 24, 2016 and U.S. Provisional Application No. 62/420,932, filed on November 11, 2016.

<u>BACKGROUND OF THE INVENTION</u>

Endogenous pulmonary lung surfactant (LS) reduces surface tension at the air-liquid interface of the alveolar lining, preventing the lungs from collapsing at end expiration. Surfactant deficiency is a common disorder in premature infants and causes respiratory distress syndrome (RDS), which can be effectively treated with preparations which are lipid extracts of minced mammalian lung or lung lavage. Said preparations are known as modified natural surfactants and they are mainly composed of phospholipids (PLs) such as phosphatidylcholine (PC), phosphatidylethanolamine (PE) and phosphatidylglycerol (PG) and the hydrophobic surfactant proteins B and C (SP-B and SP-C). Such preparations are formulated as intratracheal suspensions for administration via an endotracheal tube in the infant patient.

A list of PLs cited in this patent application follows:

- phosphatidylcholine: PC,
- phosphatidylethanolamine: PE,
- phosphatidylglycerol: PG,
- phosphatidylinositol: PI,
- phosphatidylserine: PS,
- sphingomyelin: SM,
- 1,2-dipalmitoyl-sn-glycero-3-phosphoglycerol, generally known as dipalmitoyl-phosphatidylglycerol: DPPG,
- 1,2-dipalmitoyl-sn-glycero-3-phosphocholine, generally known as dipalmitoylphosphatidylcholine: DPPC,
- 1-palmitoyl-2-oleyl-sn-glycero-3-phosphoglycerol, generally known as palmitoyl-oleyl-phosphatidylglycerol: POPG,
- 1-palmitoyl-2-oleyl-sn-glycero-3-phosphocholine generally known as palmitoyl-oleyl-phosphatidylcholine: POPC,
- 1,2-dioleyl-sn-glycero-3-phosphoglycerol generally known as dioleyl-phosphatidylglycerol: DOPG,
- 1-palmitoyl-2-linoleyl-sn-glycero-3-phosphocholine, generally known as palmitoyl-linoleyl-phosphatidylcholine: PLPC,
- 1-stearoyl-2-arachidonoyl-sn-glycero-3-phosphocholine, generally known as stearoyl-arachidonoyl-phosphocholine (SAPC),
- 1-palmitoyl-2-arachidonoyl-sn-glycero-3-phosphocholine (PAPC),
- 1,2-dipalmitoyl-sn-glycero-3-phosphoethanolamine, generally known as dipalmitoyl-phosphatidylethanolamine: DPPE,
- 1,2-distearoyl-sn-glycero-3-phosphoethanolamine, generally known as distearoyl-phosphatidylethanolamine: DSPE,
- 1,2-dipalmitoyl-sn-glycero-3-phospho-L-serine, generally known as dipalmitoyl-phosphatidylserine: DPPS.

The glycerol moieties of the phospholipids are mainly esterified with long chain fatty acids ($C_{14}$-$C_{20}$) which in turn can be saturated (e.g., myristic, palmitic and stearic acid), monounsaturated (e.g., oleic acid) or polyunsaturated (e.g., linoleic and arachidonic acid). Phospholipids containing as the characterizing residues neutral or zwitter-ionic moieties such as glycerol (PG), inositol (PI) and serine (PS) are known as acidic phospholipids. Other examples of acidic phospholipids are DPPG, POPG and DPPS.

Surfactants are usually administered to premature infants in the form of aqueous suspensions by instillation into the lungs through the trachea. They can also be administered to adults affected by various pathologies involving a severe pulmonary insufficiency such as adult respiratory distress syndrome (ARDS).

One of the most important lipid components of surfactant preparations is 1,2-dipalmitoyl-sn-glycero-3-phosphocholine (DPPC) as it forms a monomolecular film at the air-liquid interface during compression, probably undergoing phase transition (solidification) during surface compression, thereby stabilizing a system of alveoli with different sizes. It is also generally recognized that acidic phospholipids are of paramount importance in order to obtain a good activity since they favor the spreading of DPPC.

Surfactant preparations obtained from animal tissues present some drawbacks, like their availability in limited amounts, the complexity of the production and sterilization processes and the relevant production costs: as a consequence, many efforts have been made to prepare synthetic surfactants. "Artificial" surfactants are devoid of surfactant proteins and simply consist of mixtures of synthetic compounds, primarily phospholipids and other lipids that are formulated to mimic the lipid composition and behavior of natural surfactant. "Reconstituted" surfactants are artificial surfactants to which have been added surfactant proteins isolated from animals or manufactured through recombinant technology. Patent document WO 97/26863 discloses a process for the production of powdered pulmonary surfactant formulations.

Another major drawback of most prior art surfactant preparations is that they must be delivered to the patient via an endotracheal tube. It would be desirable to provide a surfactant formulation that is suitable for pulmonary delivery via inhalation and without the need for intubation to an infant or adult with comprised lung function.

## SUMMARY OF THE INVENTION

The present invention is directed respirable, dry powder particle formulations of lung surfactants that comprise surfactant proteins and that are formulated for delivery to the pulmonary system via inhalation.

The formulations of the invention comprise active agents selected from the group consisting of one or more surfactant proteins and/or one or more lung surfactants. Preferably the formulations of the invention are free of additional active agents.

The respirable, dry powder particle surfactant formulation for pulmonary delivery comprises:
i) at least 30% DPPC by weight of the particle; ii) between 0.1% to 3% NaCl by weight of the particle; iii) between 1% to 10% by weight of the particle of a surfactant protein selected from the group consisting of: SP-A, SP-B, SP-C and SP-D or any active fragment thereof, wherein the active fragment contains at least a portion of a lipid associating region and maintains the functionality of the native protein; and iv) between 10% to 30% by weight of the particle of a hydrogenated starch hydrolysate (HSH); wherein all components of the dry powder particles amount to 100 weight percent; and wherein the fine particle fraction less than 3.4 microns (FPF<3.4 $\mu$m) of the powder is 30% or more.

Preferably the formulation further comprises any one or more of DOPC, POPC, DPPE, DPPG POPG or any salt thereof (e.g. DPPG-Na or POPG-Na).

Optionally, the formulation further comprises a fatty acid derived from a vegetable or animal fat. Preferred fatty acids include lauric, myristic, palmitic (PA), stearic, oleic, linoleic and linolenic fatty acids. Preferably the fatty acid is palmitic acid (PA).

The formulation further comprises 1% to 10% and preferably about 5% by weight of a surfactant protein selected from the group consisting of: SP-A, SP-B, SP-C and SP-D; SEQ ID Nos: 1-21 or any active fragment thereof, wherein the active fragment contains at least a portion of a lipid associating region and maintains the functionality of the native protein or any amino acid sequence homologous to any of the foregoing amino acid sequences with at least 90% sequence identity at the amino acid level.

Preferably, Leucine is L-leucine.

Weight percent is intended to reflect the total amount of solids, lipids, and/or excipients in the dry particles without regard to residual water, solvent or impurities. All of the components of the dry particles amount to 100 wt %.

## DESCRIPTION OF THE DRAWINGS

FIG.1 is a surface pressure isotherm of formulation 3.
FIG.2 is a surface pressure isotherm of formulation 4.
FIG.3 is a surface pressure isotherm of formulation 5.
FIG.4 is a surface pressure isotherm of formulation 7.
FIG.5 is a surface pressure isotherm of formulation 8.
FIG.6 is a surface pressure isotherm of formulation 11.
FIG.7 is a surface pressure isotherm of formulation 12.
FIG.8 is a surface pressure isotherm of formulation 13.
FIG.9 is a surface pressure isotherm of formulation 14.
FIG.10 is a surface pressure isotherm of formulation 15.
FIG.11 is a surface pressure isotherm of formulation 16.
FIG.12 is a surface pressure isotherm of formulation 17.
FIG.13 is a surface pressure isotherm of formulation 18.
FIG.14 is a surface pressure isotherm of formulation 20.
FIG.15 is a surface pressure isotherm of formulation 21.
FIG.16 is a surface pressure isotherm of formulation 22.
FIG. 17 is a surface pressure isotherm of formulation 23.
FIG. 18 is a surface pressure isotherm of formulation 25.
FIG. 19 is a surface pressure isotherm of formulation 29.

FIG.20 is a surface pressure isotherm of formulation 56.

FIG.21 is a surface pressure isotherm of formulation 59.

FIG.22 is a surface pressure isotherm of formulation 60.

FIG.23 is scanning electron microscopy (SEM) image of formulation 31-4.

FIG.24 is scanning electron microscopy (SEM) image of formulation 31-4.

FIG.25 is a cross polarized microscopic image of formulation 31-2 in oil at 50X.

FIG.26 is an SEM image of Formulation 29.

FIG.27 is an SEM image of Formulation 29.

FIG. 28 is cross -polarized microscopic image of Formulation 32 in oil at 5x.

FIG. 29 is cross -polarized microscopic image of Formulation 32 in oil at 20x.

FIG. 30 is an SEM image of formulation 37-16 produced with Niro PSD-1.

FIG. 31 is an SEM image of formulation 37-16 produced with Niro PSD-1.

FIG. 32 is an SEM image of Formulation 33.

FIG. 33 is an SEM image of Formulation 33.

FIG. 34 is an SEM image of Formulation 34.

FIG. 35 is an SEM image of Formulation 35.

FIG. 36 is an SEM image of Formulation 1.

FIG. 37 is an SEM image of Formulation 1.

FIG. 38 is an SEM image of Formulation 47.

FIG. 39 is an SEM image of Formulation 47.

FIG. 40 is a polarized image of Formulation 2 in oil at 5x.

FIG. 41 is an SEM image of Formulation 2.

FIG. 42 is an SEM image of Formulation 2.

FIG. 43 is an image of the LFAC engines size 00 (left) and size 0 (right).

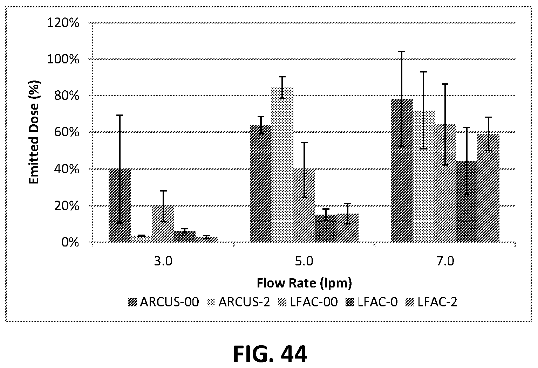

FIG. 44 is a graph showing the averaged emitted dose (ED) from two Arcus inhalers and three LFAC engines after five, 1.0 second actuations wherein N=3 inhales per flow rate.

FIG. 45 is a graph showing emitted doses as a function of actuations for ARCUS-00 inhaler.

FIG. 46 is a graph showing emitted doses as a function of actuations for ARCUS-2 inhaler.

FIG. 47 is a graph showing emitted doses as a function of actuations for LFAC-00 engine.

FIG. 48 is a graph showing emitted doses as a function of actuations for LFAC-0 engine.

FIG. 49 is a graph showing emitted doses as a function of actuations for LFAC-2inhaler.

FIG. 50 is an image of a Constant Flow Emitted Dose Setup.

FIG. 51 is an image of a Breath Simulator Emitted Dose Setup.

FIG. 52 is an image of an Assisted Flow Emitted Dose Setup.

FIG. 53 is a graph showing yield percentage as a function of batch size for Formula 71.

## DETAILED DESCRIPTION OF THE INVENTION

The terms "a", "an" and "the" as used herein are defined to mean "one or more" and include the plural unless the context is inappropriate.

The term "comprising" as used herein which is synonymous with "including," "containing," or "characterized by," is inclusive or open-ended and does not exclude additional, unrecited elements of a composition or method steps. The term "consisting of" excludes any element, step, or ingredient that is not otherwise specified. The term "consisting essentially of" limits the scope of a composition or method to the specified materials or steps and those that do not materially affect the basic and novel characteristic(s) of the specified composition or method.

The term "dry powder" as used herein refers to a composition that contains finely dispersed respirable dry particles that are capable of being dispersed in an inhalation device and subsequently inhaled by a subject. Such dry powder or dry particle may contain up to about 15% water or other solvent, preferably up to about 10% water or other solvent, or preferably be substantially free of water or other solvent, or preferably be anhydrous.

The term "dry particles" as used herein refers to respirable particles that may contain up to about 15% water or other solvent, preferably up to 10% water or other solvent or preferably be substantially free of water or other solvent, or preferably be anhydrous.

The term "respirable" as used herein refers to dry particles or dry powders that are suitable for delivery to the respiratory tract (e.g., pulmonary delivery) in a subject by inhalation. Respirable dry powders or dry particles have a mass median aerodynamic diameter (MMAD) of less than about 10 microns, preferably about 5 microns and more preferably about 3 microns or less. The "mass median aerodynamic diameter" (MMAD) is also referred to herein as "aerodynamic diameter". Experimentally, aerodynamic diameter can be determined by employing a gravitational settling method, whereby the time for an ensemble of powder particles to settle a certain distance is used to infer directly the aerodynamic diameter of the particles. An indirect method for measuring the mass median aerodynamic diameter (MMAD) is the multistage liquid impinger (MSLI). The aerodynamic diameter, $d_{aer}$, can be calculated from the equation:

$$d_{aer} = d_g \sqrt{\rho_{tap}}$$

where $d_g$ is the geometric diameter, for example the MMGD, and $\rho$ is the powder density.

As used herein, the terms "administration" or "administering" of respirable dry particles refers to introducing respirable dry particles to the respiratory tract of a subject.

As used herein, the term "respiratory tract" includes the upper respiratory tract (e.g., nasal passages, nasal cavity, throat, pharynx), respiratory airways (e.g., larynx, tranchea, bronchi, bronchioles) and lungs (e.g., respiratory bronchioles, alveolar ducts, alveolar sacs, alveoli). The deep lung, or alveoli, are typically the desired target of inhaled therapeutic formulations for systemic drug delivery. In one embodiment of the invention, most of the mass of particles deposit in the deep lung or alveoli. In another embodiment of the invention, delivery is primarily to the central airways. In other embodiments, delivery is to the upper airways.

"Pulmonary delivery," as that term is used herein refers to delivery to the respiratory tract. Pulmonary delivery includes inhalation by a patient that is capable of independent inhalation or inhalation via a ventilation system such as a mechanical ventilation (MV) system or a non-invasive mechanical ventilation system (NIMV) such as via a continuous positive airway pressure (CPAP) system.

The term "working density" as used herein is interchangeable with the term "bulk density" and is defined herein as the weight of the powder (m) divided by the volume it occupies (*Vo*) and is expressed herein as grams per liter (g/L) as determined by measurement in a graduated cylinder. Briefly, a graduated cylinder is first weighed, filled with powder without compacting, leveled if necessary without compacting and weighed again. The unsettled apparent volume is read to the nearest graduated unit. The working density is calculated by the formula *m/Vo*. Working density may also be expressed for example in grams per cubic centimeter (g/cm$^3$). In one embodiment, the working density is less than 0.1 g/cm$^3$. In one embodiment, the working density ranges from about 0.02 g/cm$^3$ to about 0.05 g/cm$^3$. In one embodiment, the capsules contain powder with a working density between about 0.03 g/cm$^3$ to about 0.06 g/cm$^3$. In another embodiment, the capsules contain powder with a working density between about 0.04 g/cm$^3$ to about 0.05 g/cm$^3$. In a further embodiment, the capsules contain powder with a working density of about 0.04 g/cm$^3$. In a further embodiment, the capsules contain powder with a working density of about 0.045 g/cm$^3$. In a further embodiment, the capsules contain powder with a working density of about 0.05 g/cm$^3$. In a further embodiment, the capsules contain powder with a working density of about 0.035 g/cm$^3$. In a further embodiment, the capsules contain powder with a working density of about 0.03 g/cm$^3$. In one embodiment, the capsules contain powder with a working density between about 0.03 g/cm$^3$ to about 0.05 g/cm$^3$. In another embodiment, the capsules contain powder with a working density between about 0.04 g/cm$^3$ to about 0.06 g/cm$^3$. In another embodiment, the capsules contain powder with a working density between about 0.05 g/cm$^3$ to about 0.06 g/cm$^3$. In another embodiment, the capsules contain powder with a working density between about 0.06 g/cm$^3$ to about 0.07 g/cm$^3$.

The term "dispersible" is a term of art that describes the characteristic of a dry powder or dry particles to be dispelled into a respirable aerosol. One way of measuring the dispersibility of a dry powder or dry particles is expressed herein as the quotient of the volume median geometric diameter (VMGD) measured at a dispersion (i.e., regulator) pressure of 1 bar divided by the VMGD measured at a dispersion (i.e., regulator) pressure of 4 bar, or VMGD at 0.5 bar divided by the VMGD at 4 bar as measured by HELOS/RODOS. These quotients are referred to herein as "¼ bar," and "0.5/4 bar," respectively, and dispersibility correlates with a low quotient. For example, ¼ bar refers to the VMGD of respirable dry particles or powders emitted from the orifice of a RODOS dry powder disperser (or equivalent technique) at about 1 bar, as measured by a HELOS or other laser diffraction system, divided the VMGD of the same respirable dry particles or powders measured at 4 bar by HELOS/RODOS. Thus, a highly dispersible dry powder or dry particles will have a ¼ bar or 0.5/4 bar ratio that is close to 1.0. Highly dispersible powders have a low tendency to agglomerate, aggregate or clump together and/or, if agglomerated, aggregated or clumped together, are easily dispersed or de-agglomerated as they emit from an inhaler and are breathed in by the subject. Dispersibility can also be assessed by measuring the size emitted from an inhaler as a function of flowrate.

Another way of measuring dispersibility that is of particular relevance to this application is to measure the emitted dose (ED) as a function of decreasing flow rate from a dry powder inhaler (DPI) or related device. Preferably, the LS powders of this invention are capable of being dispersed from a simplified version of a DP device (hereafter referred to as low flow aerosolization chamber, or LFAC, device) consisting of a simple cylindrical chamber with one or more holes at one end that can accommodate a perforated capsule containing powder) at extremely low flow rates (10 liters per minute (lpm) or less) with little resistance provided to aid in dispersion. Powder dispersibility can be quantified via calculating the ratio of the ED of a powder at a relatively low flow rate (i.e., 20, 15, 10 or 5 lpm, etc.) to the ED measured at a standard flow rate of 28.3 lpm. Thus, the LS powders disclosed herein are capable of being dispersed and deagglomerated at greatly decreased energies (a function of device resistance and flow rate through the device) as compared to traditional DP formulations administered via conventional DPI devices.

A high degree of dispersibility is a key advantageous aspect of the LS powders disclosed herein. Traditional dry powder (DP) formulations for pulmonary delivery are administered via a dry powder inhaler (DPI) with a relatively moderate to high degree of resistance incorporated into the device which acts to facilitate the dispersion and deagglomeration of the powder as patients inhale through the device at flow rates ranging from 20 to 60 liters per minute (lpm).

The terms "FPF (<5.6)," "FPF (<5.6 microns)," and "fine particle fraction of less than 5.6 microns" as used herein, refer to the fraction of a sample of dry particles that have an aerodynamic diameter of less than 5.6 microns. For example, a two- or three-stage collapsed ACI can be used to measure FPF < 5.6 microns. The two-stage collapsed ACI consists of only the top stage (S0) and the filter stage of the eight-stage ACI and allows for the collection of two separate powder fractions. Specifically, a two-stage collapsed ACI is calibrated so that the fraction of powder that is collected on S0 is composed of non-respirable dry particles that have an aerodynamic diameter of greater than 5.6 microns. The fraction of powder passing S0 and depositing on the filter stage is thus composed of respirable dry particles having an aerodynamic diameter of less than 5.6 microns. The airflow at such a calibration is approximately 60 L/min. This parameter may also be identified as "FPF TD(<5.6)," where TD means total dose. A similar measurement can be conducted using an eight-stage ACI. The eight-stage ACI cutoffs are different at the standard 60 L/min flowrate, but the FPF TD(<5.6) can be extrapolated from the eight-stage complete data set. The eight-stage ACI result can also be calculated by the USP method of using the dose collected in the ACI instead of what was in the capsule to determine FPF.

The terms "FPF (<3.4)," "FPF (<3.4 microns)," and "fine particle fraction of less than 3.4 microns" as used herein, refer to the fraction of a mass of respirable dry particles that have an aerodynamic diameter of less than 3.4 microns. For example, three-stage collapsed ACI can be used to measure both FPF < 5.6 microns and < 3.4 microns. The three-stage collapsed ACI consists of collection stage S0, S2 and the filter stage and provides fractions of powder of an aerodynamic diameter greater than 5.6 microns, less than 5.6 microns and less than 3.4 microns. This parameter may also be identified as "FPF TD(<3.4)," where TD means total dose. A similar measurement can be conducted using an eight-stage ACI. The eight-stage ACI result can also be calculated by the USP method of using the dose collected in the ACI instead of what was in the capsule to determine FPF. Other cutoff values for FPF (i.e., < 5.0 microns, etc.) can be utilized in a similar manner either via utilizing different stage configurations for the ACI or else extrapolating from the results obtained for a specific set of stages and cutoff diameters.

As used herein, the term "emitted dose" or "ED" refers to an indication of the delivery of a drug formulation from a suitable inhaler device after a firing or dispersion event. More specifically, for dry powder formulations, the ED is a measure of the percentage of powder that is drawn out of a unit dose package and that exits the mouthpiece of an inhaler device. The ED is defined as the ratio of the dose delivered by an inhaler device to the "nominal dose" (i.e., the mass of powder per unit dose placed into a suitable inhaler device prior to firing). The ED is an experimentally-measured parameter, and can be determined using the method of USP Section 601 Aerosols, Metered-Dose Inhalers and Dry Powder Inhalers, Delivered-Dose Uniformity, Sampling the Delivered Dose from Dry Powder Inhalers, United States Pharmacopia convention, Rockville, Md., 13th Revision, 222-225, 2007. This method utilizes an *in vitro* device set up to mimic patient dosing.

The term "capsule emitted powder mass" or "CEPM" as used herein refers to the amount of dry powder formulation emitted from a capsule or dose unit container during an inhalation maneuver. CEPM is measured gravimetrically, typically by weighing a capsule before and after the inhalation maneuver to determine the mass of powder formulation removed. CEPM can be expressed either as the mass of powder removed, in milligrams, or as a percentage of the initial filled powder mass in the capsule prior to the inhalation maneuver.

The term "effective amount," as used herein, refers to the amount of agent needed to achieve the desired effect, such as an amount that is sufficient to increase surface and/or bulk viscoelasticy of the respiratory tract mucus (e.g., airway lining fluid), increase gelation of the respiratory tract mucus (e.g., at the surface and/or bulk gelation), increase surface tension of the respiratory tract mucus, increasing elasticity of the respiratory tract mucus (e.g., surface elasticity and/or bulk elasticity), increase surface viscosity of the respiratory tract mucus (e.g., surface viscosity and/or bulk viscosity), reduce the amount of exhaled particles, reduce pathogen (e.g., bacteria, virus) burden, reduce symptoms (e.g., fever, coughing, sneezing, nasal discharge, diarrhea and the like), reduce occurrence of infection, reduce viral replication, or improve or prevent deterioration of respiratory function (e.g., improve forced expiratory volume in 1 second FEV1 and/or forced expiratory volume in 1 second FEV1 as a proportion of forced vital capacity FEV1/FVC) and/or reduce bronchoconstriction. The actual effective amount for a particular use can vary according to the particular dry powder or dry particle, the mode of administration, and the age, weight, general health of the subject, and severity of the symptoms or condition being treated. Suitable amounts of dry powders and dry particles to be administered, and dosage schedules, for a particular patient can be determined by a clinician of ordinary skill based on these and other considerations.

Preferred "excipients" as that term is used herein are those excipients that can be taken into the lungs with no significant adverse toxicological effects on the lungs. Such excipients are generally regarded as safe (GRAS) by the U.S. Food and Drug Administration. Such excipients include water.

The term "patient" or "subject" as used interchangeably herein are individuals to whom the compositions of the invention may be administered. Examples of such individuals include adult humans and pediatric humans. Pediatric humans include individuals aged from birth up to 18 years of age. Pediatric aged children may also include the following subgroups including but not limited to, neonates comprising newborn individuals up to about 28 days of age or 1 month of age; infants comprising individuals aged from the neonatal period up to 12 months of age; toddlers comprising individuals of ages 1-3 years old; preschool children comprising individuals of ages 3-5 years old, school-aged children comprising individuals of ages 6 to 10 years old and adolescents comprising individuals of ages 11-14 years. Pediatric children may also be referred to having the following age ranges of about 6 to about 11 years of age, about 12 to about 17 years of age, or about 6 to about 17 years of age. Premature human children (preemies) include individuals who are less than about 37 weeks of gestational age.

The term "compromised patients" as used herein includes individuals who do not or cannot breathe hard or have a compromised lung function. Examples of such individuals include premature infants and/or newborns suffering from respiratory distress syndrome (RDS), adults suffering from acute respiratory distress (ARDS), and adults and children suffering from other disease states associated with compromised lung function (cystic fibrosis, COPD, etc.). Generally, the individual will have a peak inspiratory flow rate (PIFR) of less than about 30 liters per minute. In one embodiment, the patient will have a PIFR of about 15 liters per minute or less. Alternatively or additionally, the compromised patient has an inspiration volume of less than 2 liters, such as less than about 1.5 liters, including less than about 1 liter, such as about 0.75 liters, such as about 0.5 liters, such as about 0.2 liters, or such as about 0.1 liters or less.

The term "peak inspiratory flow rate" (PFIR) as used herein refers to a patient's maximum speed of inhalation as conventionally assessed via a pulmonary flow meter.

The term "hydrogenated starch hydrolysate" (HSH) or "polyglycitol" as used interchangeably herein refers to the broad group of polyols that contain substantial quantities of hydrogenated oligo- and polysaccharides in addition to any monomeric or dimeric polyols (sorbitol, mannitol or maltitol, respectively). HSH are produced by the partial hydrolysis of corn, wheat or potato starch and subsequent hydrogenation of the hydrolysate at high temperature under pressure. The end product is an ingredient composed of sorbitol, maltitol and higher hydrogenated saccharides (maltitritol and others). By varying the conditions and extent of hydrolysis, the relative occurrence of various mono-, di-, oligo- and polymeric hydrogenated saccharides in the resulting product can be obtained.

Sequences similar to or homologous to (e.g., at least about 70% sequence identity) the sequences disclosed herein are also part of the invention. In some embodiments, the sequence identity at the amino acid level can be about 70%, 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

Calculations of "homology" or "sequence identity" or "similarity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences. In the case of circularly related proteins, the sequence of one of the partners needs to be appropriately split and aligned in two sections to achieve optimal alignment of the functionally equivalent residues necessary to calculate the percent identity.

Amino acid and nucleotide sequence alignments and homology, similarity or identity, as defined herein are preferably prepared and determined using the algorithm BLAST 2 Sequences, using default parameters (Tatusova, T. A. et al., FEMS Microbiol Lett, 174:187-188 (1999)). Alternatively, the BLAST algorithm (version 2.0) is employed for sequence alignment, with parameters set to default values. BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, 1990, Proc. Natl. Acad. Sci. USA 87(6):2264-8.

There are several important limitations related to the potential implementation of LS delivery therapies for the treatment of neonatal RDS in the developing world are related to the costs, resources, equipment and infrastructure required to deliver replacement LS in liquid form via intratracheal instillation to premature infants as such formulations cannot be delivered effectively via passive nebulization. A dry powder formulation of LS could instead be delivered in a noninvasive manner, such as via incorporation of a dry powder LS formulation and delivery system into a noninvasive ventilation system such as a continuous positive airway pressure (CPAP) system. Such a formulation and delivery system could thus provide an inexpensive and efficacious alternative for use in developing areas where intratracheal liquid installation is not possible and where LS formulations must be in a more stable and portable dry powder form versus in a liquid form that requires refrigeration.

The invention provides respirable dry powder particles that comprise one or more lung surfactants (LS), one or more phospholipids, and optionally one or more lung surfactant proteins as active ingredients. Preferably the formulations are free of additional active agents.

The respirable dry powder particles of the invention are particularly formulated for inhalation by patients having compromised lung function also referred to herein as "compromised patients" such as those patients suffering from surfactant deficiency resulting from a disease condition. The dry powder particles of the invention are capable of being inhaled through, for example a dry powder inhaler, or via a system that provides inhalation through a ventilator.

The respirable, dry powder particle surfactant formulation for pulmonary delivery comprises:
i) at least 30% DPPC by weight of the particle; ii) between 0.1% to 3% NaCl by weight of the particle; iii) between 1% to 10% by weight of the particle of a surfactant protein selected from the group consisting of: SP-A, SP-B, SP-C and SP-D or any active fragment thereof, wherein the active fragment contains at least a portion of a lipid associating region and maintains the functionality of the native protein; and iv) between 10% to 30% by weight of the particle of a hydrogenated starch hydrolysate (HSH); wherein all components of the dry powder particles amount to 100 weight percent; and wherein the fine particle fraction less than 3.4 microns (FPF<3.4 $\mu$m) of the powder is 30% or more.

Preferably the formulation further comprises one or more of additional phospholipids, such as DOPC, POPC, DPPE, DPPG POPG or any salt thereof (e.g. DPPG-Na or POPG-Na). Preferably, the formulation comprises a combination of DPPC, POPG, and/or POPC or salts thereof. In one embodiment the combination is DPPC and POPG or a salt thereof. POPG-Na is a preferred additional phospholipid.

The total phospholipids (DPPC and additional phospholipid, if any) is at least 30% by weight, preferably at least about 40% by weight, more preferably at least 50% by weight, preferably at least about 60% by weight, more preferably at least 70% by weight, such as about 80% by weight. The ratio of DPPC to additional phospholipids is preferably at least 1:1. For example, the ratio of DPPC: additional phospholipids (e.g., POPG or POPG-Na) can be between about 1:1 to 4:1 (e.g.,1:1, 2:1, 3:1, 4:1). Preferably, the ratio is about 4.1. Preferably, the ratio is about 4.1 or less. For example, the ratio of DPPC: POPG-Na can be more than about 7:3, such as between about 7:3 to 3:1.

Optionally, the formulation further comprises a fatty acid derived from a vegetable or animal fat. Preferred fatty acids include lauric, myristic, palmitic (PA), stearic, oleic, linoleic and linolenic fatty acids. Preferably the fatty acid is palmitic acid (PA).

The formulation further comprises 1% to 10% and preferably about 5% by weight of a surfactant protein selected from the group consisting of: SP-A, SP-B, SP-C and SP-D; SEQ ID NOs: 1-21, or any active fragment thereof, wherein the active fragment contains at least a portion of a lipid associating region and maintains the functionality of the native protein or any amino acid sequence homologous to any of the foregoing amino acid sequences with at least 70%, 80% 85% 90% or 90% sequence identity at the amino acid level.

Preferably, Leucine is L-leucine.

Weight percent is intended to reflect the total amount of solids, lipids, and/or excipients in the dry particles without regard to residual water, solvent or impurities. All of the components of the dry particles amount to 100 wt %.

The respirable dry particles of the invention preferably have an MMAD of about 10 microns or less, such as an MMAD of about 0.5 micron to about 10 microns.
Preferably, the dry particles of the invention have an MMAD of about 7 microns or less (e.g., about 0.5 micron to about 7 microns), preferably about 1 micron to about 7 microns, or about 2 microns to about 7 microns, or about 3 microns to about 7 microns, or about 4 microns to about 7 microns, about 5 microns to about 7 microns, about 1 micron to about 6 microns, about 1 micron to about 5 microns, about 2 microns to about 5 microns, about 2 microns to about 4 microns, or about 3 microns.

The fine particle fraction less than 5.6 microns, or FPF<5.6 of a powder corresponds to the percentage of particles in the powder that have an aerodynamic diameter of less than 5.6 $\mu$m. The FPF<5.6 of a powder of the invention is preferably about 40% or more. In certain embodiments, the FPF<5.6 of the powder is at least about 50%, 60% or 70%. In one embodiment, the FPF<5.6 is about 30% to about 90%. In one embodiment, the FPF<5.6 is about 70% to about 95%. In one embodiment, the FPF<5.6 is about 70% to about 90%. In one embodiment, the FPF<5.6 is about 70% to about 85% or about 70% to about 80%.

The fine particle fraction less than 3.4 microns, or FPF<3.4, of a powder corresponds to the percentage of particles in the powder that have an aerodynamic diameter of less than 3.4 $\mu$m. In one embodiment, the FPF<3.4 of a powder of the invention is 30% or more. In one embodiment, the FPF<3.4 of the powder is at least about 40% or 50%. In one embodiment, the FPF<3.4 is 30% to 60%.

Preferably, the powders of the invention have a tap density of less than about 0.4 g/cm$^3$. For example, the powders have a tap density between 0.02 and 0.20 g/cm$^3$, between 0.02 and 0.15 g/cm$^3$, between 0.03 and 0.12 g/cm$^3$,

between 0.05 and 0.15 g/cm$^3$, or less than about 0.15 g/cm$^3$, or a tap density less than about 0.10 g/cm$^3$, a tap density less than about 0.15 g/cm$^3$. In one embodiment, the powders of the invention have a tap density of less than about 0.2 g/cm$^3$. Preferably, the tap density is from about 0.02 to 0.175 g/cm$^3$. Preferably, the tap density is from about 0.06 to 0.175 g/cm$^3$.

Tap density can be measured by using instruments known to those skilled in the art such as the Dual Platform Microprocessor Controlled Tap Density Tester (Vankel, N.C.) or a GEOPYC ™ instrument (Micrometrics Instrument Corp., Norcross, GA, 30093). Tap density is a standard measure of the envelope mass density. Tap density can be determined using the method of USP Bulk Density and Tapped Density, United States Pharmacopia convention, Rockville, Md., 10$^{th}$ Supplement, 4950-4951, 1999. Features which can contribute to low tap density include irregular surface texture and porous structure. The envelope mass density of an isotropic particle is defined as the mass of the particle divided by the minimum sphere envelope volume within which it can be enclosed. In one embodiment of the invention, the particles have an envelope mass density of less than about 0.4 g/cm$^3$.

Preferably, the respirable dry powders and dry particles formulations of the invention have a water or solvent content of less than about 15% by weight, less than about 13% by weight, less than about 11.5% by weight, less than about 10% by weight, less than about 9% by weight, less than about 8% by weight, less than about 7% by weight, less than about 6% by weight, less than about 5% by weight, less than about 4% by weight, less than about 3% by weight, less than about 2% by weight, less than about 1% by weight or be anhydrous.

Preferably, the dry particle formulations of the invention can have a water or solvent content of less than about 6% and greater than about 1%, less than about 5.5% and greater than about 1.5%, less than about 5% and greater than about 2%, about 2%, about 2.5%, about 3%, about 3.5%, about 4%, about 4.5%, or about 5%.

Preferably, the HSH powder can be, for example the polyglycitol, STABILITE™ SD30 or SD60 (INNOVA, Muscatine, Iowa). The polyol distribution for STABILITE™ SD30 is about 2% sorbitol by weight and about 6% maltitol by weight and the polyol distribution for STABILITE™ SD60 is about 1% sorbitol and about 3.5% maltitol. Other general characteristics of the STABILITE™ family of products are listed in Table A.

See, https://www.scribd.com/document/239956170/STABILITE-SD-polyglycitol-powder-fact-sheet.

Table A

| General Characteristics of the STABILITE ™ Products | | |
|---|---|---|
| | STABILITE SD30 | STABILITE SD60 |
| Moisture | 8% maximum | 8% maximum |
| Ash | 0.1% maximum | 0.1 % maximum |
| pH of 20% solution | 4.0-5.0 | 4.0-5.0 |
| Reducing Sugars | 1.0% maximum | 1.0% maximum |
| Viscosity of 50% | about 90 cP | about 280 cP |
| solution at 25° C. | | |
| Osmolality of 20% | About 278 mOsm | About 185 mOsm |
| solution | | |
| Polyol distribution | HP 1 (sorbitol) ~2% | HP 1 (sorbitol) ~1% |
| | HP 2 (maltitol) ~6% | HP 2 (maltitol) ~3.5% |
| | HP 3 and above ~92% | HP 3 and above ~95.5% |

Preferably, HSH powder is a mixed polyol composition where no one polyol is present in a quantity greater than 50% by weight. Preferably the HSH (preferably, SD30) is less than 30% by weight, preferably less than about 25% by weight in a formulation. Preferred ranges of HSH (e.g., SD30) include, 10 to 30% HSH by weight and about 15 to about 25% by weight.

Any salt is suitable for use in the invention. Preferably less than about 10% by weight, preferably less than about 5% by weight, preferably less than about 3% by weight, preferably less than about 1% by weight, preferably less than about 1 % by weight and preferably between about 0.1% to about 2 % by weight of a salt is present in a formulation of the invention. Preferred ranges of salts include, but are not limited to, about 0.1 to about 10% salt by weight, about 0.1 to about 3% salt by weight and about 0.1 to about 3% by weight. Preferably the particles of the invention comprise about 2% by weight of salts. Preferred salts include, but are not limited to sodium salts, potassium salts, lithium salts and calcium salts. A preferred salt is sodium chloride (NaCl). Preferably the dry particles comprise about 2% by weight NaCl. The claims demand the presence of 0.1% to 3% NaCl by weight of the particle.

The particles of the invention can optionally comprise a fatty acid in an amount ranging from about 1% by weight to about 10% by weight, more preferably from about 1% by weight to about 5% by weight, and more preferably about 1 % by weight, 2% by weight, 3% by weight, 4% by weight or 5% by weight.

Surfactant proteins (SP) are included in the dry powder surfactant formulations of the invention. Preferably, surfactant proteins include to SP-A, SP-B, SP-C, SP-D, SEQ ID NOS: 1-21 or any active fragment thereof, wherein the active fragment contains at least a portion of a lipid associating region and maintains the functionality of the native protein. SP-A and SP-B are believed to have roles in the production of the surfactant monolayer in the lungs. SP-A and SP-D are also believed to have rules are believed to have roles in binding pathogens and other organic materials such as pollens and dust mite antigens that may be present in the lungs.

The structural features of the full-length mature SP-A, SP-B, and SP-C proteins are well known and reported as Genbank Accession Nos. L10123, BC111570, BC111571, BC026229, NM-006926, and NM 005411 for SP-A; L11573, AF400074, BC032785, NM-000542, and NM-198843 for SP-B; and J03890, U02948, AY357924, AY337315, BC005913, and NM-003018 for SP-C. When fragments of the mature SP-A, SP-B, and/or SP-C are employed in the surfactant compositions of the present invention, it is preferable to utilize fragments thereof that contain at least a portion of a lipid associating region. Lipid associating regions are those portions of the mature protein that are capable of molecular interaction with lipids (either native glycerophospholipids or synthetic phospholipase-resistant lipids) to promote surface activity of the resulting composition in which they are introduced. Such fragments include, without limitation, fragments of SP-A that contain an amphipathic or hydrophobic region capable of associating with lipids, fragments of SP-B that contain an amphipathic or hydrophobic region capable of associating with lipids, fragments of SP-C that contain an amphipathic or hydrophobic region capable of associating with lipids, as well as any number of synthetic peptides or combinations thereof. One exemplary SP-B peptide family is designated the "Mini-B Family" (Protein Data Bank Coordinate accession number 1SSZ). Exemplary peptides from the Mini-B Family include, but are not limited to SEQ ID NOS: 1-21.

Examples of preferable peptide derivatives of SP-B and SP-C include, but are not limited to those described in U.S. Pat. No. 8,563,683 including but not limited to the following exemplary peptides of Table B:

Table B

| |
|---|
| CWFCRFFFKRFFFFFPKGGRFFPFFFCRFFFRCS (SEQ ID NO: 1), |
| CWFCRAFIKRFQAMIPKGGRMLPQLVCRLVLRCS (SEQ ID NO: 2), |
| CWLCRALIKRIQAMIPKGGRMFPQFFCRFFFRCS (SEQ ID NO: 3), |
| CWFCRAFIKRFQAMIPKGGRMFPQFFCRFFFRCS (SEQ ID NO: 4), |
| CWFCRAFIKRFQAMIPKGERMLPQLVCRLVLRCS (SEQ ID NO: 5), |
| CWLCRALIKRIQAMIPKGERMFPQFFCRFFFRCS (SEQ ID NO: 6), |
| CWFCRAFIKRFQAMIPKGERMFPQFFCRFFFRCS (SEQ ID NO: 7), |
| CWLCRALIKRIQAMIPCGGRMLPQLVCRLVLRCS (SEQ ID NO: 8), |
| FPIPLPYCWLCRALIKRIQAMIPKGGRMLPQLVCRLVLRCS (SEQ ID NO: 9), |
| FPCPLPYCWLCRALIKRIQAMIPKGGRMLPQLVCRLVLRCS (SEQ ID NO: 10), |
| CPIPLPYCWLCRALIKRIQAMIPKGGRMLPQLVCRLVLRCS (SEQ ID NO: 11), |
| CWLCRALIKRIQAMIPKGALAVAVAQVCRVVPLVAGGICQALAERYS |
| VILLDTLLGRMLPQLVCRLVLRCS (SEQ ID NO: 12), |
| CWLCRALIKRIQAMIPKGALAVAVAQVCRVVPLVAGGICQFLAERYSV |
| ILLDTLLGRMLPQLVCRLVLRCS (SEQ ID NO: 13), |
| FPIPLPYCWLCRALIKRIQAMIPKGALAVAVAQVCRVVPLVVGGICQYL |
| AERYSVILLDTLLGRMLPQLVCRLVLRCS (SEQ ID NO: 14), |
| CPIPLPYCWLCRALIKRIQAMIPKGALAVAVAQVCRVVPLVVGGICQY |
| LAERYSVILLDTLLGRMLPQLVCRLVLRCS (SEQ ID NO: 15), |
| FPCPLPYCWLCRALIKRIQAMIPKGALAVAVAQVCRVVPLVVGGICQY |
| LAERYSVILLDTLLGRMLPQLVCRLVLRCS (SEQ ID NO: 16), |
| FGIPFFPVHLKRLLVVVVVVVLVVVVIVGALLMGL (SEQ ID NO: 17), |
| FGIPFFPVHLKRLLVPVVVVVLVVVVIVGALLMGL (SEQ ID NO: 18), |

(continued)

| FGIPFFPVHLKRLLVVVVVPVLVVVVIVGALLMGL (SEQ ID NO: 19), |
| FGIPFFPVHLKRLLVVVVVVVLVPVVIVGALLMGL (SEQ ID NO: 20), |
| FGIPFFPVHLKRLLVVVVVVVVLVVVVIPGALLMGL (SEQ ID NO: 21), |

and any amino acid sequences homologous to any of the foregoing peptides with at least 70%, preferably at least 80%, preferably at least 85%, preferably at least 90%, preferably at least 95% sequence identity at the amino acid level.

A preferred SP-B peptide for use in the surfactant compositions of the invention comprises the amino acid sequence of SEQ ID NO: 9 or any amino acid sequences homologous thereto with at least 70%, preferably at least 80%, preferably at least 90%, preferably at least 95% sequence identity at the amino acid level.

The dry powder particle formulations of the invention can be administered with low inhalation energy such as to compromised patients. In order to relate the dispersion of powder at different inhalation flow rates, volumes, and from inhalers of different resistances, the energy required to perform the inhalation maneuver can be calculated Inhalation energy can be calculated from the equation $E=R^2Q^2V$ where E is the inhalation energy in Joules, R is the inhaler resistance in $kPa^{1/2}/LPM$, Q is the steady flow rate in L/min and V is the inhaled air volume in L.

The dry powder particle formulations of the invention are characterized by a high emitted dose (e.g., CEPM of at least 75%, at least 80%, at least 85%, at least 90%, at least 95%, preferably at least 100%) from a dry powder inhaler when a total inhalation energy of less than about 2 Joules or less than about 1 Joule, or less than about 0.8 Joule, or less than about 0.5 Joule, or less than about 0.3 Joule is applied to the dry powder inhaler. For example, an emitted dose of at least 75%, at least 80%, at least 85%, at least 90%, at least 95% CEPM of a formulation of the invention contained in a unit dose container, containing about 5 to about 50 mg, preferably about 10 mg to about 40 mg, preferably about 25 mg to about 50 mg, preferably about 40 mg, or preferably about 50 mg of the appropriate formulation, in a dry powder inhaler can be achieved when a total inhalation energy of less than about 1 Joule (e.g., less than about 0.8 Joule, less than about 0.5 Joule, less than about 0.3 Joule) is applied to the dry powder inhaler. An emitted dose of at least about 70% CEPM of respirable dry powder contained in a unit dose container, containing about 50 mg or about 40 mg of the respirable dry powder, in a dry powder inhaler can be achieved when a total inhalation energy of less than about 0.28 Joule is applied to the dry powder inhaler. The dry powder can fill the unit dose container, or the unit dose container can be at least 40% full, at least 50% full, at least 60% full, at least 70% full, at least 80% full, or at least 90% full. The unit dose container can be a capsule (e.g., size 000, 00, 0E, 0, 1, 2, 3, and 4, with respective volumetric capacities of 1.37 ml, 950 $\mu$l, 770 $\mu$l, 680 $\mu$l, 480 $\mu$l, 360 $\mu$l, 270 $\mu$l, and 200 $\mu$l). Alternatively, the unit dose container can be a blister. The blister can be packaged as a single blister, or as part of a set of blisters, for example, 7 blisters, 14 blisters, 28 blisters, or 30 blisters.

Alternatively, the dry powder formulations of the invention may be administered to patients indirectly via a ventilator. For example, premature infants are often on some kind of ventilation such as invasive mechanical ventilation, or non-invasive ventilation which does not require the use of an endotracheal tube. In such cases, inhalable drugs such as the dry powder formulations of the invention may be administered via a system wherein an inhaler or other device configured to release the dry powder of the invention is incorporated into the ventilation system and is activated to deliver the inhalable drug to the respiratory system of the patient in conjunction with the action of the ventilation system. The formulations of the invention are particularly useful in conjunction with non-invasive ventilation, such as a continuous positive airway pressure (CPAP) system.

An advantage of the invention is the production of powders that disperse well across a wide range of flowrates and are relatively flowrate independent. The dry particles and powders of the invention enable the use of a simple, passive DPI for a wide patient population or alternatively, for use with ventilators of all types and particularly non-invasive ventilation.

The methods that are disclosed, but not claimed, comprise administering an effective amount of a surfactant dry powder formulation of the invention to a patient who is need of restoration of pulmonary function as the result of disease characterized by insufficient lung surfactant production. Preferably, the method is effective for treatment of newborns, infants, children, and adults who suffer from any condition caused by insufficient surfactant production.

Abnormalities of surfactant production have been described in various lung diseases including but not limited to asthma, bronchitis, chronic obstructive pulmonary disease, and following lung transplantation. Abnormal surfactant production has also been seen in infectious and suppurative lung diseases, such as cystic fibrosis, pneumonia, and AIDS. Finally, insufficient surfactant also characterizes diseases such as acute respiratory distress syndrome (ARDS), pulmonary edema, interstitial lung diseases, pulmonary alveolar proteinosis, following cardiopulmonary bypass, and in smokers.

The respirable dry particles and dry powder formulations of the invention can be administered to the respiratory tract of a subject in need thereof using any suitable method, such as instillation techniques, and/or an inhalation device, such as a dry powder inhaler (DPI) or metered dose inhaler (MDI). A number of DPIs are available, such as, the ARCUS inhalers, (e.g., the inhalers described in US Patent No. 94687288), the inhalers disclosed in U.S. Pat. Nos. 4,995,385 and 4,069,819, SPINHALER® (Fisons, Loughborough, U.K.), ROTAHALERS®, DISKHALER® and DISKUS® (GlaxoSmithKline, Research Triangle Technology Park, N.C.), FLOWCAPSS® (Hovione, Loures, Portugal), INHALATORS® (Boehringer-Ingelheim, Germany), AEROLIZER® (Novartis, Switzerland), and others known to those skilled in the art.

Generally, inhalation devices (e.g., DPIs) are able to deliver a maximum amount of dry powder or dry particles in a single inhalation, which is related to the capacity of the blisters, capsules (e.g. size 000, 00, 0E, 0, 1, 2, 3, and 4, with respective volumetric capacities of 1.37 ml, 950 $\mu$l, 770 $\mu$l, 680 $\mu$l, 480 $\mu$l, 360 $\mu$l, 270 $\mu$l, and 200 $\mu$l) or other means that contain the dry particles or dry powders within the inhaler. Accordingly, delivery of a desired dose or effective amount may require two or more inhalations. Preferably, each dose that is administered to a subject in need thereof contains an effective amount of respirable dry particles or dry powder and is administered using no more than about 4 inhalations. For example, each dose of respirable dry particles or dry powder can be administered in a single inhalation or 2, 3, or 4 inhalations. The respirable dry particles and dry powders, are preferably administered in a single, breath-activated step using a breath-activated DPI. When this type of device is used, the energy of the subject's inhalation both disperses the respirable dry particles and draws them into the respiratory tract.

The respirable dry particles or dry powders can be delivered by inhalation to a desired area within the respiratory tract, as desired. It is well-known that particles with an aerodynamic diameter of about 1 micron to about 3 microns, can be delivered to the deep lung. Larger aerodynamic diameters, for example, from about 3 microns to about 5 microns can be delivered to the central and upper airways.

Any one of the above inhalers may also be used in conjunction with an invasive mechanical ventilation (MV) or noninvasive mechanical ventilation (NIMV) such as that which is generally used for premature babies and other individuals suffering from compromised lung function. A noninvasive mechanical ventilation (NIMV) system includes a CPAP system. There is currently no commercially available system designed specifically for inhalation therapy during NIMV. However, the formulations of the present invention are capable of high emitted doses in conjunction with a ventilation system and therefore studies have shown that various inhalers may be configured for use with and to achieve delivery of therapeutically effective amounts to the lung of the patient.

Preferably, a therapeutically effective dosage comprises about 10 mg surfactant formulation per kg of body weight to about 200 mg surfactant formulation per kg of body weight.

Preferred surfactant formulations are shown in Table C Only formulations # 72, 75, 77 are according to the claims.

Table C

| Formulation # | Description |
|---|---|
| 1 | DPPC:DOPC:POPC:SD-30:NaCl (50:10:10:28:2) |
| 2 | DPPC:DOPC:POPC:DPPE:DPPG-Na:POPG-Na:SD-30:NaCl (32.8:13.3:13.3:2.6:4.0:4.0:28:2.0) |
| 3 | DPPC:DPPG-Na:POPG-Na:CaCl2:SD-30:NaCl (50:10:10:6.9:21.1:2) |
| 4 | DPPC:POPC:POPG-Na:SD-30:NaCl (42:14:14:28:2) |
| 5 | DPPC:POPC:POPG-Na:CaCl2:SD-30:NaCl (42:14:14:5:23:2) |
| 6 | DPPC:POPC:POPG-Na:Mg Lactate:NaCl (42:14:14:28:2) |
| 7 | DPPC:POPC:POPG-Na:SD-30:NaCl (40:24:16:18:2) |
| 8 | DPPC:POPC:POPG-Na:Leu:NaCl (40:24:16:18:2) |
| 9 | DPPC:POPC:POPG-Na:SD-30:NaCl (44:18:18:18:2) |
| 10 | DPPC:POPC:DPPG-Na:POPG-Na:SD-30:NaCl (44:18:9:9:18:2) |
| 11 | DPPC:POPC:POPG-Na:Leu:NaCl (44:18:18:18:2) |
| 12 | DPPC:POPC:DPPG-Na:POPG-Na:Leu:NaCl (44:18:9:9:18:2) |
| 13 | DPPC:POPG-Na:SD-30:NaCl (56:24:18:2) |
| 14 | DPPC:DPPG-Na:POPG-Na:SD-30:NaCl (56:8:16:18:2) |
| 15 | DPPC:POPG-Na:Leu:NaCl (56:24:18:2) |
| 16 | DPPC:DPPG-Na:POPG-Na:Leu:NaCl (56:8:16:18:2) |
| 17 | DPPC:DPPE:DPPG-Na:POPG-Na:SD-30:NaCl (40:24:8:8:18:2) |
| 18 | DPPC:DPPE:DPPG-Na:POPG-Na:Leu:NaCl (40:24:8:8:18:2) |
| 19 | DPPC:POPC:Leucine:NaCl (60:20:18:2) |

(continued)

| Formulation # | Description |
|---|---|
| 20 | DPPC:Trehalose:NaCl (80:18:2) |
| 21 | DPPC:DPPG:POPG:Albumin*:NaCl (58:10:25:5:2) |
| 22 | DPPC:POPG:Albumin* (80:15:5) |
| 23 | DPPC:POPG:Albumin* (70:25:5) |
| 24 | DPPC:POPG:Albumin* (60:35:5) |
| 25 | DPPC:POPG:Albumin*:NaCl (78:15:5:2) |
| 26 | DPPC:POPG:Albumin*:NaCl (68:25:5:2) |
| 27 | DPPC:POPG:Albumin*:NaCl (58:35:5:2) |
| 28 | DPPC:SD-30:Leu:NaCl (40:40:18:2) |
| 29 | DPPC:SD-30:NaCl (80:18:2) |
| 30 | DPPC:SD-30:NaCl (60:38:2) |
| 31 | DPPC: L-leu:NaCl (80:18:2) |
| 32 | DPPC: L-leu:NaCl (60:38:2) |
| 33 | DPPC:DOPC:Leu:NaCl (70:10:18:2) |
| 34 | DPPC:DOPC:SD-30:Leu:NaCl (60:10:20:8:2) |
| 35 | DPPC:DOPC:SD-30:Leu:NaCl (60:20:10:8:2) |
| 36 | DPPC:DOPC:SD-30:NaCl (80:10:8:2) |
| 37 | DPPC:Leu:NaCl (80:18:2) |
| 40 | DPPC:Albumin*:NaCl (93:5:2) |
| 41 | DPPC:Lactose:NaCl (80:18:2) |
| 42 | DPPC:Mannitol:NaCl (80:18:2) |
| 43 | DPPC:POPC:NaCl (70:28:2) |
| 45 | DPPC:POPG:Albumin* (50:45:5) |
| 65 | DPPC:POPG-Na:SD-30:NaCl (49:21:28:2) |
| 66 | DPPC:POPG-Na:Leu:NaCl (49:21:28:2) |
| 67 | DPPC:POPC:SD-30:NaCl (49:21:28:2) |
| 68 | DPPC:POPC:Leu:NaCl (49:21:28:2) |
| 69 | DPPC:POPC:SD-30:NaCl (56:24:18:2) |
| 70 | DPPC:POPC:Leu:NaCl (56:24:18:2) |
| 71 | DPPC:POPG-Na:SD-30:Albumin*:NaCl (49:21:25:3:2) |
| 72 | DPPC:POPG-Na:SD-30:SP-B:NaCl (49:21:25:3:2) |
| 73 | DPPC:POPG-Na:Palmitic Acid:SD-30:NaCl 49:21:5:23:2 |
| 74* | DPPC:POPG-Na:Palmitic Acid:Albumin*:SD-30:NaCl 49:21:5:3:20:2 |
| 75 | DPPC:POPG-Na:Palmitic Acid:SP-B:SD-30:NaCl (49:21:5:3:20:2) |
| 76 | DPPC:POPG-Na:POPC:SD-30:NaCl (49:21:7:21:2) |
| 77 | DPPC:POPG:POPC:SP-B:SD-30:NaCl (49:21:7:3:18:2) |
| 78 | DPPC:POPG:POPC:Albumin*: SD-30:NaCl (49:21:7:3:18:2) |

*Albumin is preferably replaced with a surfactant protein, e.g., SEQ ID No. 9, in these formulations.

Preferably, the surfactant formulations of the present invention are selected from Formulations 1-45 and 65-72 and 73-77. Preferably the surfactant formulations of the invention are selected from Formulations 1-27 and 65-72 and 73-77. Preferably Formulations 1-45 and 65-71 and 72, 73, 74, 76 and 77 (further include a surfactant protein in addition to the named components, or instead of one or more of the named components, for example as shown in Formulas 72, 75 and 77. Preferably, the surfactant protein is present in an amount of about 5 weight percent or less of the particle. Preferably the surfactant protein is derived from SP-B including but not limited to SEQ ID NOS: 1-21. Preferably the surfactant protein comprises SEQ ID NO: 9 or a homologous amino acid sequence with at least about 70%, preferably at least about 80%, preferably at least about 85%, preferably at least about 90%, or preferably at least about 95% sequence identity at the amino acid level.

A preferred surfactant formulation comprising a surfactant protein is DPPC:POPG-Na:SD-30:*surfactant protein*:NaCL (49:21:25:3:2). Preferably the surfactant protein is SP-B including but not limited to SEQ ID NOS: 1-21. Preferably the surfactant protein comprises SEQ ID NO: 9 or a homologous amino acid sequence with at least about 70%, preferably at least about 80%, preferably at least about 85%, preferably at least about 90%, or preferably at least about 95% sequence identity at the amino acid level. A preferred surfactant formulation comprising a surfactant protein in accordance with the invention is Formula 72.

Preferably, the percentage of excipient when present in the particle is lowered or eliminated in order to accommodate the additional presence of surfactant protein. Preferably in those formulations comprising albumin (Formulations 21-27, 45 and 71) at least one surfactant protein, preferably SP-B or fragment, derivative or modification thereof (e.g. SEQ ID NOS: 1-21), replaces the 5% by weight albumin in the formulation. Alternatively, the ratio of the excipients can be maintained and the surfactant protein added thereto. Preferably less than about 25% by weight, preferably less than about 15% by weight, preferably less than about 10% by weight, and/or less than or equal to about 5% by weight of a surfactant protein is present in a formulation of the invention. Preferred ranges of surfactant protein include, but are not limited to, about 0.1 to about 25% by weight, about 1 to about 10% by weight and about 2 to about 7% by weight. Preferably the particles of the invention comprise about 3%, 4%, 5%, 6% or 7% by weight of surfactant protein.

Other surfactant containing formulations are shown in Table D:

**Table D**

| Formulation # | Description |
|---|---|
| 38 | DPPC:POPC (70:30) |
| 39 | DPPC:Albumin* (95:5) |
| 44 | DPPC:POPC (70:30) |
| 46 | DPPC:DOPC:Leu:NaCl (60:20:18:2) |
| 47 | DPPC:DOPC (95:5) |
| 48 | DPPC:DOPC:Leucine:NaCl (60:20:18:2) |
| 49 | DPPC:POPG:Albumin*:NaCl (48:45:5:2) |
| 50 | DPPC:DPPE:POPG:NaCl (68:10:20:2) |
| 51 | DPPC:DPPG:POPG:NaCl (68:10:20:2) |
| 52 | DPPC:SD-30:NaCl (40:58:2) |
| 53 | DPPC:SD-30:NaCl (20:78:2) |
| 54 | DPPC: L-leu:NaCl (40:58:2) |
| 55 | DPPC: L-leu:NaCl (20:78:2) |
| 56 | DPPC:SD-30:Oregon Green Dye: NaCl (80:17.5:0.5:2) |
| 57 | DPPC:SD-30:Fluorescein Dye (FD):NaCl (80:17.5:0.5:2) |
| 58 | DPPC:SD-30:FD:NaCl (80:16:2:2) |
| 59 | DPPC:POPC:POPG-Na:SD-30:FD:NaCl (42:14:14:27.5:0.5:2) |
| 60 | DPPC:POPC:POPG-Na:SD-30:FD:NaCl (40:24:16:17.5:0.5:2) |
| 61 | DPPC:DOPC:SD-30 (85:5:10) |
| 62 | DPPC:DPPG:POPG-Na:Albumin*) (60:10:25:5) |
| 63 | DPPC:POPG-Na (70:30) |

(continued)

| Formulation # | Description |
|---|---|
| 64 | DPPC:POPG-Na:NaCl (68:30:2) |

*Albumin is preferably replaced with a surfactant protein in these formulations

Methods for Preparing Dry Powders and Dry Particles

The respirable dry particles and dry powders can be prepared using any suitable method. Many suitable methods for preparing respirable dry powders and particles are conventional in the art, and include single and double emulsion solvent evaporation, spray drying, milling (e.g., jet milling), blending, solvent extraction, solvent evaporation, phase separation, simple and complex coacervation, interfacial polymerization, suitable methods that involve the use of supercritical carbon dioxide ($CO_2$), and other suitable methods. Respirable dry particles can be made using methods for making microspheres or microcapsules known in the art. These methods can be employed under conditions that result in the formation of respirable dry particles with desired aerodynamic properties (e.g., aerodynamic diameter and geometric diameter). If desired, respirable dry particles with desired properties, such as size and density, can be selected using suitable methods, such as sieving.

The respirable dry particles are preferably spray dried. Suitable spray-drying techniques are described, for example, by K. Masters in "Spray Drying Handbook", John Wiley & Sons, New York (1984). Generally, during spray-drying, heat from a hot gas such as heated air or nitrogen is used to evaporate a solvent from droplets formed by atomizing a continuous liquid feed. If desired, the spray drying or other instruments, e.g., jet milling instrument, used to prepare the dry particles can include an inline geometric particle sizer that determines a geometric diameter of the respirable dry particles as they are being produced, and/or an inline aerodynamic particle sizer that determines the aerodynamic diameter of the respirable dry particles as they are being produced.

For spray drying, solutions, emulsions or suspensions that contain the components of the dry particles to be produced in a suitable solvent (e.g., aqueous solvent, organic solvent, aqueous-organic mixture or emulsion) are distributed to a drying vessel via an atomization device. For example, a nozzle or a rotary atomizer may be used to distribute the solution or suspension to the drying vessel. Examples of suitable spray dryers that can be outfitted with either a rotary atomizer or a nozzle, include, Mobile Minor Spray Dryer or the Model PSD-1, both manufactured by Niro, Inc. (Denmark). Actual spray drying conditions will vary depending, in part, on the composition of the spray drying solution or suspension and material flow rates.

The person of ordinary skill will be able to determine appropriate conditions based on the compositions of the solution, emulsion or suspension to be spray dried, the desired particle properties and other factors. In general, the inlet temperature to the spray dryer is about 50° C to about 200° C., and preferably is about 60° C to about 150° C. The spray dryer outlet temperature will vary depending upon such factors as the feed temperature and the properties of the materials being dried. Generally, the outlet temperature is about 40° C to about 150° C, preferably about 50° C to about 120° C, or about 60° C to about 90° C. If desired, the respirable dry particles that are produced can be fractionated by volumetric size, for example, using a sieve, or fractioned by aerodynamic size, for example, using a cyclone, and/or further separated according to density using techniques known to those of skill in the art.

To prepare the respirable dry particles of the invention, generally, a solution, emulsions or suspension that contains the desired components of the dry powder (i.e., a feed stock) is prepared and spray dried under suitable conditions. Preferably, the dissolved or suspended solids concentration in the feed stock is at least about 1 g/L, at least about 2 g/L, at least about 5 g/L, at least about 10 g/L, at least about 15 g/L, at least about 20 g/L, at least about 30 g/L, at least about 40 g/L, at least about 50 g/L, at least about 60 g/L, at least about 70 g/L, at least about 80 g/L, at least about 90 g/L, or at least about 100 g/L. The feed stock can be provided by preparing a single solution or suspension by dissolving or suspending suitable components (e.g., salts, excipients, other active ingredients) in a suitable solvent. The solvent, emulsion or suspension can be prepared using any suitable methods, such as bulk mixing of dry and/or liquid components or static mixing of liquid components to form a combination. For example, a hydrophilic component (e.g., an aqueous solution) and a hydrophobic component (e.g., an organic solution) can be combined using a static mixer to form a combination. The combination can then be atomized to produce droplets, which are dried to form respirable dry particles. Preferably, the atomizing step is performed immediately after the components are combined in the static mixer.

The feed stock, or components of the feed stock, can be prepared using any suitable solvent, such as an organic solvent, an aqueous solvent or mixtures thereof. Suitable organic solvents that can be employed include but are not limited to alcohols such as, for example, ethanol, methanol, propanol, isopropanol, butanols, and others. Other organic solvents include but are not limited to perfluorocarbons, dichloromethane, chloroform, ether, ethyl acetate, methyl tert-butyl ether and others. Co-solvents that can be employed include an aqueous solvent and an organic solvent, such as, but not limited to, the organic solvents as described above. Aqueous solvents include water and buffered solutions.

The feed stock or components of the feed stock can have any desired pH, viscosity or other properties. If desired, a pH buffer can be added to the solvent or co-solvent or to the formed mixture. Generally, the pH of the mixture ranges from about 3 to about 8.

Respirable dry particles and dry powders can be fabricated and then separated, for example, by filtration or centrifugation by means of a cyclone, to provide a particle sample with a preselected size distribution. For example, greater than about 30%, greater than about 40%, greater than about 50%, greater than about 60%, greater than about 70%, greater than about 80%, or greater than about 90% of the respirable dry particles in a sample can have a diameter within a selected range. The selected range within which a certain percentage of the respirable dry particles fall can be, for example, any of the size ranges described herein, such as between about 0.1 to about 3 microns VMGD.

The diameter of the respirable dry particles, for example, their VMGD, can be measured using an electrical zone sensing instrument such as a Multisizer lie, (Coulter Electronic, Luton, Beds, England), or a laser diffraction instrument such as a HELOS system (Sympatec, Princeton, N.J.). Other instruments for measuring particle geometric diameter are well known in the art. The diameter of respirable dry particles in a sample will range depending upon factors such as particle composition and methods of synthesis. The distribution of size of respirable dry particles in a sample can be selected to permit optimal deposition within targeted sites within the respiratory system.

Experimentally, aerodynamic diameter can be determined using time of flight (TOF) measurements. For example, an instrument such as the Model 3225 Aerosizer DSP Particle Size Analyzer (Amherst Process Instrument, Inc., Amherst, Mass.) can be used to measure aerodynamic diameter. The Aerosizer measures the time taken for individual respirable dry particles to pass between two fixed laser beams.

Aerodynamic diameter also can be experimentally determined directly using conventional gravitational settling methods, in which the time required for a sample of respirable dry particles to settle a certain distance is measured. Indirect methods for measuring the mass median aerodynamic diameter include the Andersen Cascade Impactor and the multi-stage liquid impinger (MSLI) methods. The methods and instruments for measuring particle aerodynamic diameter are well known in the art.

Tap density is a measure of the envelope mass density characterizing a particle. The envelope mass density of a particle of a statistically isotropic shape is defined as the mass of the particle divided by the minimum sphere envelope volume within which it can be enclosed. Features which can contribute to low tap density include irregular surface texture and porous structure. Tap density can be measured by using instruments known to those skilled in the art such as the Dual Platform Microprocessor Controlled Tap Density Tester (Vankel, N.C.), a GEOPYC™ instrument (Micrometrics Instrument Corp., Norcross, Ga.), or SOTAX Tap Density Tester model TD2 (SOTAX Corp., Horsham, Pa.). Tap density can be determined using the method of USP Bulk Density and Tapped Density, United States Pharmacopia convention, Rockville, Md., 10th Supplement, 4950-4951, 1999.

Fine particle fraction can be used as one way to characterize the aerosol performance of a dispersed powder. Fine particle fraction describes the size distribution of airborne respirable dry particles. Gravimetric analysis, using a Cascade impactor, is one method of measuring the size distribution, or fine particle fraction, of airborne respirable dry particles. The Andersen Cascade Impactor (ACI) is an eight-stage impactor that can separate aerosols into nine distinct fractions based on aerodynamic size. The size cutoffs of each stage are dependent upon the flow rate at which the ACI is operated. The ACI is made up of multiple stages consisting of a series of nozzles (i.e., a jet plate) and an impaction surface (i.e., an impaction disc). At each stage an aerosol stream passes through the nozzles and impinges upon the surface. Respirable dry particles in the aerosol stream with a large enough inertia will impact upon the plate. Smaller respirable dry particles that do not have enough inertia to impact on the plate will remain in the aerosol stream and be carried to the next stage. Each successive stage of the ACI has a higher aerosol velocity in the nozzles so that smaller respirable dry particles can be collected at each successive stage.

If desired, a two- or three-stage collapsed ACI can also be used to measure fine particle fraction. The two-stage collapsed ACI consists of only the top stage (S0) and the filter stage of the eight-stage ACI and allows for the collection of two separate powder fractions. Specifically, a two-stage collapsed ACI is calibrated so that the fraction of powder that is collected on S0 is composed of non-respirable dry particles that have an aerodynamic diameter of greater than 5.6 microns. The fraction of powder passing S0 and depositing on the filter stage is thus composed of respirable dry particles having an aerodynamic diameter of less than 5.6 microns. The airflow at such a calibration is approximately 60 L/min. Similarly, the three-stage collapsed ACI consists of collection stage S0, S2 and the filter stage and provides fractions of powder of an aerodynamic diameter greater than 5.6 microns, less than 5.6 microns and less than 3.4 microns. The FPF (<5.6) has been demonstrated to correlate to the fraction of the powder that is able to make it into the lung of the patient, while the FPF(<3.4) has been demonstrated to correlate to the fraction of the powder that reaches the deep lung of a patient. These correlations provide a quantitative indicator that can be used for particle optimization.

An ACI can be used to approximate the emitted dose, which herein is called gravimetric recovered dose and analytical recovered dose. "Gravimetric recovered dose" is defined as the ratio of the powder weighed on all stage filters of the ACI to the nominal dose. "Analytical recovered dose" is defined as the ratio of the powder recovered from rinsing all stages, all stage filters, and the induction port of the ACI to the nominal dose. The FPF _TD(<5.0) is the ratio of the interpolated amount of powder depositing below 5.0 μm on the ACI to the nominal dose. The FPF _RD(<5.0) is the ratio of the interpolated amount of powder depositing below 5.0 μm on the ACI to either the gravimetric recovered dose or the analytical recovered dose.

Another way to approximate emitted dose is to determine how much powder leaves its container, e.g., capture or blister, upon actuation of a dry powder inhaler (DPI). This takes into account the percentage leaving the capsule, but does not take into account any powder depositing on the DPI. The emitted dose is the ratio of the weight of the capsule with the dose before inhaler actuation to the weight of the capsule after inhaler actuation. This measurement can also be called the capsule emitted powder mass (CEPM).

A Multi-Stage Liquid Impinger (MSLI) is another device that can be used to measure fine particle fraction. The Multi-stage liquid Impinger operates on the same principles as the ACI, although instead of eight stages, MSLI has five. Additionally, each MSLI stage consists of an ethanol-wetted glass frit instead of a solid plate. The wetted stage is used to prevent particle bounce and re-entrainment, which can occur when using the ACI.

## EXAMPLES

The present invention will be better understood in connection with the following Examples. However, it should be understood that these examples are for illustrative purposes only and are not meant to limit the scope of the invention.

## Example 1- Materials and Methods

### ABBREVIATIONS:

DOPC: 1,2-di-(9Z-octadecenoyl)-sn-glycero-3-phosphocholine
DPPC: Dipalmitoylphosphatidylcholine
DPPE: 1,2-dihexadecanoyl-sn-glycero-3-phosphoethanolamine
DPPG-Na: 1,2-Dipalmitoyl-sn-glycero-3-phosphoglycerol, sodium salt
gPSD: Average geometric particle size (d50)
L-leu or Leu: Leucine
Mg Lactate: Magnesium lactate trihydrate
PA: palmitic acid
n.c.: Not calculated
n.d.: Not detected
POPC: 1-palmitoyl-2-oleoyl-sn-glycero-3-phosphocholine
POPE: 1-palmitoyl-2-oleoyl-sn-glycero-3-phosphoethanolamine
POPG-Na: 1-palmitoyl-2-oleoyl-sn-glycero-3-phospho-(1'-rac-glycerol) (sodium salt)
SC-D: Semicrystalline due to lipid bilayer
SC-DE: Semicrystalline due to lipid bilayer and excipients
SD-30: Polyglycitol
TGA-120: Total weight loss or volatiles from heating a sample up to 120 °C at a heating ramp of 20 °C/ min
Um or μm: micrometer
XRD: X-Ray powder diffraction

Formulation numbers that include a dash (e.g. Formula 31-1, Formula 31-2 and so on) indicate that the components of the formula and the ratios of the components of the formula are identical to those of the parent formula (e.g. Formula 31), however, formulations containing dashes (e.g. Formula 31-1) are not from the same lot as the parent formulation and may also have been processed using different process parameters as compared to the parent formulation lot.

REAGENTS:

1. Polyglycitol Stabilite SD-30 (Grain Processing Corp., Muscatine, IA, USA)
2. L-leucine (Ajinomoto Aminosciences LLC, Raleigh, NC, USA)
3. DPPC (Lipoid GmbH, Steinhausen, Switzerland)
4. POPG-Na (Avanti Polar Lipids, Alabaster, AL, USA)

5. POPC (Lipoid GmbH, Steinhausen, Switzerland)
6. Sodium chloride (VWR, Radnor, PA, USA)
7. Ethanol, 200 Proof (Pharmco Products Inc, Brookfield, CT, USA)
8. Civitas Purified Water
9. DOPC
10. DPPE
11. DPPG-Na
12. Albumin
13. Mg Lactate
14. Lactose
15. Trehalose.

EQUIPMENT:

1. GEA Niro PSD, Size 1 (GEA Process Engineering, Soborg, Denmark).
2. Glove box equipped with N2 supply and accordion gloves (Bel-art products, Wayne, NJ).
3. Buchi B-290 spray dryer.

ANALYSIS:

Selected formulations were tested for the following parameters:

1. gPSD (Average geometric particle size in microns (d50));
2. Fine particle fraction of the total dose < 5.6 um and/or 3.4 um (grav.);
3. XRPD (X-ray powder diffraction);
4. Low T1 (Characteristic temperature(s) of the first thermal event(s) observed during a DSC scan at 20 °C/min);
5. Low T2 (Characteristic temperature(s) of the second set of thermal event(s) observed during a DSC scan at 20 °C/min); and
6. TGA-120 (%) (Volatiles loss by 120 °C).

## Example 2-Spray Drying Process Parameters and Determining Properties of Powders.

**2.1 DPPC: SD-30: Leucine:NaCl (40:40:18:2)** (not according to the claims)

The first powder spray dried with Niro PSD-1 is Formulation 28, containing 40% DPPC and 60% excipients. It is a semi-crystalline matrix with diffractions characteristic of DPPC and excipients (SC-DE). It has a high fine particle fraction as determined in Size 00 and Size 2 capsules. Table 1 summarizes the aerosol properties, Table 2 summarizes the solid state properties, and Table 3 lists the process parameters used for the spray drying operation. These process parameters were the default values used for subsequent formulations with Niro PSD-1 unless otherwise noted.

Table 1- Aerosol properties of DPPC: SD-30:Leucine:NaCl (40:40:18:2)

| Formula tion Number | Formulation components | | | | Fine particle fraction | | | | gPSD (VMG D 1 bar, um) |
|---|---|---|---|---|---|---|---|---|---|
| | DPPC | SD-30 | L-leu | NaCl | Size 00 | | Size 2 | | |
| | | | | | <5.6 um(%) | <3.4 um (%) | <5.6 um (%) | <3.4 um (%) | |
| 28 | 40% | 40% | 18% | 2% | 79 | 63 | 78 | 59 | 3.8 |

Table 2- Solid state properties of DPPC:SD-30:Leucine:NaCl (40:40:18:2)

| Formulation Number | Description | XRD | TGA-120 (%) | Low T1 (°C) | Low T2 (°C) |
|---|---|---|---|---|---|
| 28 | DPPC:SD-30:Leu:NaCl (40:40:18:2) | SC-DE | 2.05 | 46.5 | 65.8 |

**Table 3-** Initial default process parameters used to produce powder with Niro PSD-1

| Process Parameters- Niro PSD-1 | | | | |
|---|---|---|---|---|
| Total solid concentration (g/L) | 2 | | Aqueous Flow (mL/min) | 10 |
| Inlet Temperature (°C) | 62 | | Organic flow (mL/min) | 10 |
| Outlet Temperature (°C) | 40 | | Product filter purge gas (scfh) | 20 |
| Drying Gas Rate (kg/hr) | 125 | | Air cap # | 67147 |

**2.2 DPPC: Leucine:NaCl (80:18:2)- Buchi B-290** (not according to the claims)

Spray dried Formulation 31-4 (DPPC:Leucine:NaCl (80:18:2)) was produced with Buchi B-290 to compare with the powder made with Niro PSD-1. Buchi B-290 produced a powder with a smaller geometric size and a lower FPF. Table 4 shows the process parameters used to spray dry Formulation 31-4. Table 5 summarizes the aerosol properties for the powder produced for this evaluation.

**Table 4**- Primary process parameters for spray drying lipids with excipients using Buchi B-290

| Process Parameters- Buchi B-290 | | | | |
|---|---|---|---|---|
| Total solid concentration (g/L) | 4 | | Aqueous Flow (mL/min) | 5 |
| Inlet Temperature (°C) | 100 | | Organic flow (mL/min) | 5 |
| Outlet Temperature (°C) | 40 | | Product filter purge gas (scfh) | NA |
| Drying Gas Rate (kg/hr) | 17 | | Air cap # | 46375 |
| Atomization Gas Flow (mm) | 40 | | **Fluid** cap # | 44554 |

**Table 5**- Aerosol properties of DPPC: Leu:NaCl (80:18:2) produced with Buchi B290

| Formulation Number | Formulation components | | | Fine particle fraction | | | | gPSD (VMGD 1 bar, um) |
|---|---|---|---|---|---|---|---|---|
| | DPPC | L-leu | NaCl | Size 00 | | Size 2 | | |
| | | | | <5.6 um (%) | <3.4 um (%) | <5.6 um (%) | <3.4 um (%) | |
| 31-4 | 80% | 18% | 2% | 41 | 23 | 55 | 31 | 3.1 |

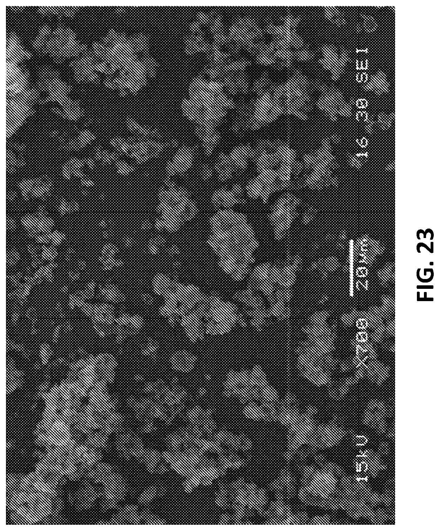

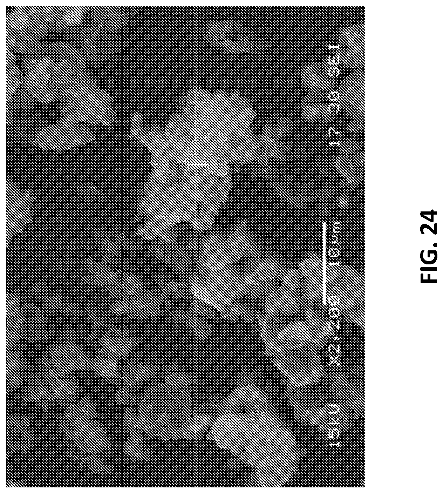

Figures 23 and 24 are each scanning electron microscopy (SEM) images of Formulation 31-4.

**2.3 DPPC:Leucine:NaCl (80:18:2)- Decreasing drying gas flow** (not according to the claims)

The process parameters were changed from the default values (Table 3) to determine the effect on the powder properties. Changing the drying gas flow and maintaining the outlet temperature at 40 °C did not affect the aerosol properties. Less drying gas led to more residual solvent in the powder.

Table 6 summarizes the aerosol properties and Table 7 summarizes the solid state properties for powders produced for this evaluation.

**Table 6**- Aerosol properties of DPPC; Leucine:NaCl (80:18:2)- Change in drying gas flow

| Formulation Number | Process Parameters | | Fine particle fraction | | | | gPSD (VMGD 1 bar, um) |
|---|---|---|---|---|---|---|---|
| | Inlet Temp (°C) | Drying Gas Rate (kg/hr) | Size 00 | | Size 2 | | |
| | | | <5.6 um (%) | <3.4 um (%) | <5.6 um (%) | <3.4 um (%) | |
| 31-1 | 63 | 125 | 71 | 48 | 74 | 47 | 3.8 |
| 31-2 | 68 | 100 | 71 | 49 | 75 | 53 | 3.7 |
| 31-3 | 77 | 75 | 64 | 42 | 78 | 53 | 3.8 |

**Table 7**- Solid state properties of DPPC:Leu:NaCl (80:18:2)- Change in drying gas flow

| Formulation Number | Description | XRD | TGA-120 (%) | Low T1 (°C) | Low T2 (°C) |
|---|---|---|---|---|---|
| 31-1 | Drying gas: 125 kg/hr | SC-DE | 1.3 | 38.5 | 75.7, 96.5, 114.1 |
| 31-2 | Drying gas: 100 kg/hr | SC-DE | 1.86 | 33.7 | 73.6, 97.1, 119.2 |
| 31-3 | Drying gas: 75 kg/hr | SC-DE | 2.24 | 35.8 | 73.9, 94.8, 116.2 |

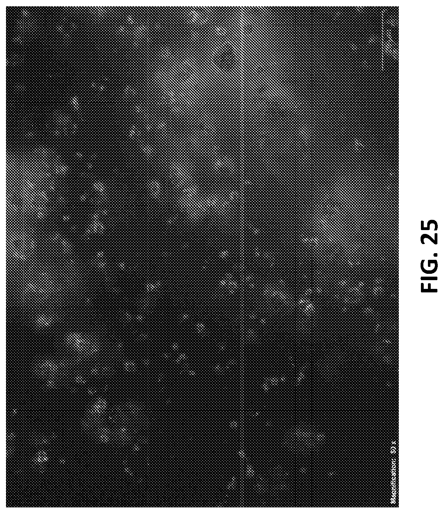

Figure 25 is a cross polarized microscopic image of Formulation 31-2 in oil at 50x.

**2.4 DPPC:Leucine:NaCl (80:18:2)- Increasing the feed flow rate** (not according to the claims)

The feed flow rates were changed from the initial default values (Table 3) to determine the effect on the powder properties. Increasing the total feed flow rate to 40 ml/min produced powder with a high FPF of 85 (Formulation 37-2). Therefore, this improvement is one preferred method for spray drying powders. Table 8 summarizes the process parameters and Table 10 summarizes the aerosol properties for powders produced for this evaluation.

Table 8- Changing the process parameters for spray drying DPPC: Leu:NaCl (80:18:2) from the initial default values in Table 3

| Formulation Number | Process Parameters | | | | |
|---|---|---|---|---|---|
| | Inlet Temp (°C) | Drying Gas Rate (kg/hr) | Aqueous Flow (mL/min) | Organic flow (mL/min) | Atomization gas (g/min) |
| 37-14 | 77 | 125 | 20 | 20 | 20 |
| 37-15 | 84 | 100 | 20 | 20 | 20 |
| 37-1 | 63 | 125 | 10 | 10 | 20 |
| 37-2 | 80 | 125 | 20 | 20 | 20 |
| 37-3 | 93 | 125 | 30 | 30 | 20 |
| 37-4 | 93 | 125 | 30 | 30 | 40 |

**Table 9**- Aerosol properties of DPPC: Leu:NaCl (80:18:2)- Increasing the feed flow rate

| Formulation Number | Fine particle fraction | | | | gPSD (VMGD 1 bar, um) |
|---|---|---|---|---|---|
| | Size 00 | | Size 2 | | |
| | <5.6 um (%) | <3.4 um (%) | <5.6 um (%) | <3.4 um (%) | |
| 37-14 | 58 | 37 | 73 | 48 | 3.7 |
| 37-15 | 60 | 37 | 68 | 50 | 3.8 |
| 37-1 | NA | NA | 74 | 47 | 5.8 |
| 37-2 | NA | NA | 85 | 61 | 5.6 |
| 37-3 | NA | NA | 76 | 49 | 7.9* |
| 37-4 | NA | NA | 67 | 43 | 6 |
| *Non-uniform particles | | | | | |

**Table 10**- Solid state properties of DPPC; Leu:NaCl (80:18:2)- Increasing the feed flow rate

| Formulation Number | Description | XRD | TGA-120 (%) | Low T1 (°C) | Low T2 (°C) |
|---|---|---|---|---|---|
| 37-14 | Aq:Or (20:20); 125 kg/ hr | SC-DE | 1.95 | 35.1 | 71.4, 99.3, 122.8 |

(continued)

| Formulation Number | Description | XRD | TGA-120 (%) | Low T1 (°C) | Low T2 (°C) |
|---|---|---|---|---|---|
| 37-15 | Aq:Or (20:20); 100 kg/ hr | SC-DE | 2.02 | n.c. | 69.8, 110.1, 135.1 |

**2.5 DPPC:Leucine:NaCl (80:18:2)- Changing aqueous: organic feed ratio** (not according to the claims)

The aqueous to organic feed ratio was changed from the initial default values (Table 3) to determine the effect on the powder properties. Table 11 summarizes the process parameters and Table 12 summarizes the aerosol properties for powders produced for this evaluation.

Table 11- Changing the process parameters for spray drying DPPC: Leu:NaCl (80:18:2) from the default values in Table 3

| Formulation Number | Process Parameters | | |
|---|---|---|---|
| | Inlet Temp (°C) Drying Gas Rate (kg/hr) | Aqueous Flow (mL/min) | Organic flow (mL/min) |
| 37-5 | 75 | 32 | 8 |
| 37-6 | 75 | 28 | 12 |
| 37-7 | 75 | 24 | 16 |
| 37-2 | 80 | 20 | 20 |
| 37-8 | 80 | 16 | 24 |
| 37-9 | 80 | 12 | 28 |
| 37-10 | 80 | 8 | 32 |

**Table 12**- Aerosol properties of DPPC: Leu:NaCl (80:18:2)-Changing the aqueous:organic feed ratio

| Formulati on Number | Fine particle fraction | | gPSD (VMGD 1 bar, um) |
|---|---|---|---|
| | Size 2 | | |
| | <5.6 um (%) | <3.4 um (%) | |
| 37-5 | 57 | 37 | 7.7 |
| 37-6 | 64 | 43 | 6.7 |
| 37-7 | 65 | 44 | 7.6 |
| 37-2 | 85 | 61 | 5.6 |
| 37-8 | 70 | 46 | 4.9 |
| 37-9 | 75 | 54 | 4 |
| 37-10 | 74 | 56 | 2.9 |

**2.6 DPPC:Leucine:NaCl (80:18:2)- Changing total solid concentration** (not according to the claims)

The total solid concentration was changed from the initial default values (Table 3) to determine the effect on the powder properties.

Table 13 summarizes the change in process parameters and aerosol properties for powders produced for this evaluation. Table 14 shows the revised process parameters for spray drying the formulation with Niro PSD-1 based on the aerosol performance.

Table 13- Aerosol properties of DPPC: Leu:NaCl (80:18:2)- Changing total solid concentration

| Formulation Number | Total solid concentration (g/L) | Fine particle fraction | | gPSD (VMGD 1 bar, um) |
|---|---|---|---|---|
| | | Size 2 | | |
| | | <5.6 um (%) | <3.4 um (%) | |
| 37-2 | 2 | 85 | 61 | 5.6 |
| 37-11 | 4 | 49 | 23 | 13.6 |
| 37-12 | 6 | 52 | 26 | 14.8 |
| 37-13 | 8 | 54 | 26 | 15.7 |

Table 14- Revised process parameters for spray drying formulation with Niro PSD-1

| Process Parameters- Niro PSD-1 | | | | |
|---|---|---|---|---|
| Total solid concentration (g/L) | 2 | | Aqueous Flow (mL/min) | 20 |
| Inlet Temperature (°C) | 80 | | Organic flow (mL/min) | 20 |
| Outlet Temperature (°C) | 40 | | Product filter purge gas (scfh) | 20 |
| Drying Gas Rate (kg/hr) | 125 | | Air cap # | 67147 |
| Atomization Gas Flow (g/min) | 20 | | Fluid cap # | 2850 |

## EXAMPLE 3-Spray Drying Process Parameters and Determining Properties of Powders

### 3.1 DPPC: SD-30:NaCl (Varying ratio of DPPC to SD-30) (not according to the claims)

Using SD-30 as the excipient, the DPPC composition was varied to determine the effect on the aerosol and solid state properties. The process parameters used to spray dry these powders are found in Table 3. As the DPPC concentration decreased and the SD-30 concentration increased, the geometric size of the particles decreased. A higher DPPC content led to a larger endothermic event at ~70 °C.

Table 15 summarizes the aerosol properties and summarizes the solid state properties for powders produced for this evaluation.

Table 15- Aerosol properties of DPPC; SD-30;NaCl (Varying ratio of DPPC to SD-30)

| Formulation Number | Formulation components | | | Fine particle fraction | | | | gPSD (VMGD 1 bar, um) |
|---|---|---|---|---|---|---|---|---|
| | DPPC | SD-30 | NaCl | Size 00 | | Size 2 | | |
| | | | | <5.6 um (%) | <3.4 um (%) | <5.6 um (%) | <3.4 um (%) | |
| 29 | 80% | 18% | 2% | 67 | 44 | 70 | 49 | 3.7 |
| 30 | 60% | 38% | 2% | 65 | 47 | 86 | 65 | 3.1 |
| 52 | 40% | 58% | 2% | 68 | 53 | 83 | 66 | 2.8 |
| 53 | 20% | 78% | 2% | 62 | 44 | 79 | 59 | 1.9 |

Table 16- Solid state properties of DPPC: SD-30:NaCl (Varying ratio of DPPC: SD-30)

| Formulation Number | DPPC:SD-30:NaCl (%) | XRD | TGA-120 (%) | Low T1 (°C) | Low T2 (°C) |
|---|---|---|---|---|---|
| 29 | (80:18:2) | SC-D | 2.1 | 42.8, 63.9 | 72.5 |
| 30 | (60:38:2) | SC-D | 2.1 | 40.7, 61.5 | 71 |
| 52 | (40:58:2) | SC-D | 2.4 | 43.6, 61.4 | 69.4 |
| 53 | (20:78:2) | SC-D | 2.24 | 44.4, 62.2 | 69.1 |

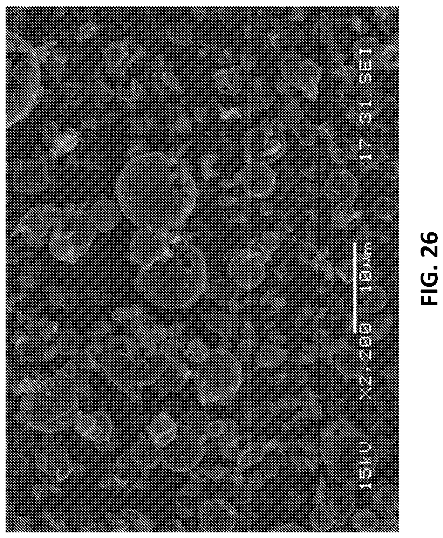

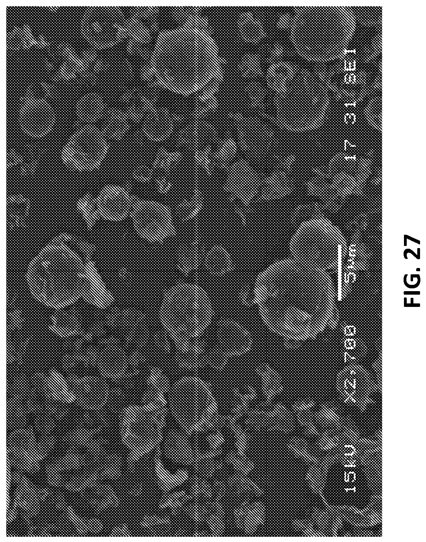

FIGS. 26 and 27 are each SEM images of Formulation 29.

**3.2 DPPC: Leucine:NaCl (Varying ratio of DPPC to Leucine)** (not according to the claims)

Using leucine as the excipient, the DPPC composition was varied to determine the effects on the aerosol and solid state properties. The process parameters used to spray dry these powders are found in Table 3. As the DPPC concentration decreased and the leucine concentration increased, the geometric size of the particles decreased. Higher leucine content produced drier powders. Compared to SD-30, leucine formulations contained more high temperature endothermic events, which may decrease the powder's physical stability. Table 17 summarizes the aerosol properties and Table 18 summarizes the solid state properties for powders produced for this evaluation.

**Table 17**- Aerosol properties of DPPC: Leu:NaCl (Varying ratio of DPPC to Leucine)

| Formulation Number | Formulation components | | | Fine particle fraction | | | | gPSD (VMGD 1 bar, um) |
|---|---|---|---|---|---|---|---|---|
| | DPPC | L-leu | NaCl | Size 00 | | Size 2 | | |
| | | | | <5.6 um (%) | <3.4 um (%) | <5.6 um (%) | <3.4 um (%) | |
| 31 | 80% | 18% | 2% | 57 | 40 | 73 | 48 | 5.1 |
| 32 | 60% | 38% | 2% | 72 | 50 | 79 | 56 | 2.9 |
| 54 | 40% | 58% | 2% | 74 | 57 | 76 | 53 | 2.9 |
| 55 | 20% | 78% | 2% | 73 | 54 | 79 | 60 | 2.6 |

**Table 18**- Solid state properties of DPPC: Leu:NaCl (Varying ratio of DPPC to Leu)

| Formulation Number | DPPC: L-leu:NaCl (%) | XRD | TGA-120 (%) | Low T1 (°C) | Low T2 (°C) |
|---|---|---|---|---|---|
| 31 | (80:18:2) | SC-DE | 1.91 | 34.8 | 73.8, 80.1, 101.2, 126.1 |
| 32 | (60:38:2) | SC-DE | 1.23 | 35.1 | 73.8, 80.3, 101.3, 121.5 |
| 54 | (40:58:2) | SC-DE | 0.95 | 35.2 | 73.2, 79.8, 102, 125.2 |
| 55 | (20:78:2) | SC-DE | 0.54 | 34.9 | 73.2, 79.8, 102.5, 125.1 |

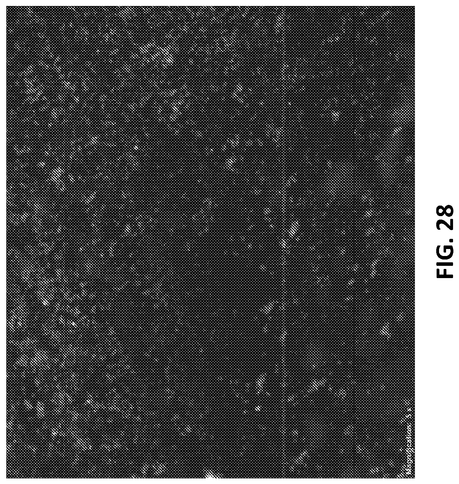

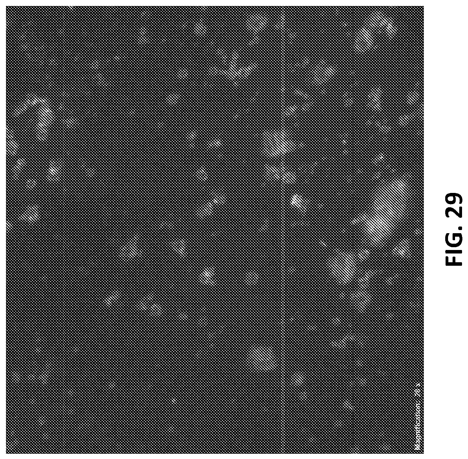

FIGS 28 and 29 are each cross-polarized microscopic images of Formulation 32 in oil at 5x and 20x, respectively.

**3.3 DPPC:Leucine:NaCl (80:18:2)** (not according to the claims)

DPPC:Leucine:NaCl (80:18:2) was spray dried with the process parameters shown in Table 3 in order to produce enough powder for inhaler device measurements. Compared with the Buchi powder (FIGs. 26 and 27), the Niro PSD-1 seemed to produce powder with less agglomeration.
Table 19 summarizes the aerosol properties for the powder produced for this evaluation.

**Table 19-** Aerosol properties of DPPC: Leucine:NaCl (80:18:2) produced with Buchi B-290

| Formulation Number | Formulation components | | | Fine particle fraction | | | | gPSD (VMGD 1 bar, um) |
|---|---|---|---|---|---|---|---|---|
| | DPPC | L-leu | NaCl | Size 00 | | Size 2 | | |
| | | | | <5.6 um (%) | <3.4 um (%) | <5.6 um (%) | <3.4 um (%) | |
| 37-16 | 80% | 18% | 2% | 58 | 35 | 72 | 44 | 7 |

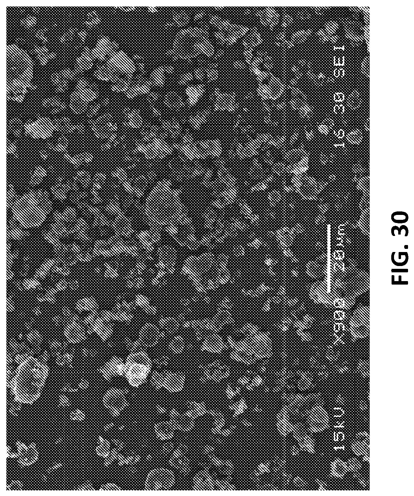

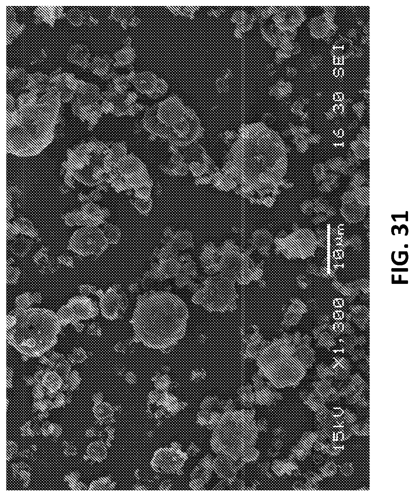

FIGS. 30 and 31 are each SEM images of Formulation 37-16 produced with Niro PSD-1.

**3.4 DPPC:DOPC:SD-30:Leucine:NaCl** (not according to the claims)

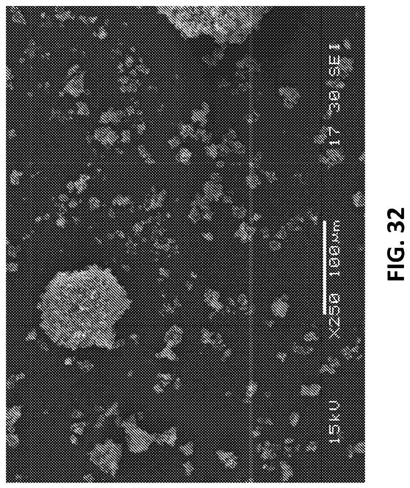

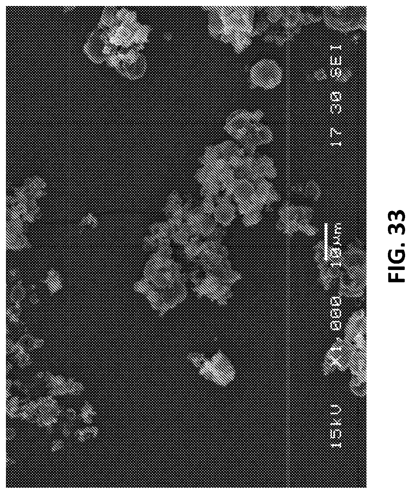

DOPC was introduced into the formulation of DPPC. The process parameters used to spray dry these powders are found in Table 3. The addition of DOPC increased the geometric size and lowered the FPF. Formulation 33 appears to contain small particles that are cohesive and tend to form large conglomerates (FIGS 32 and 33.).

Table 20 summarizes the aerosol properties and Table 21 summarizes the solid state properties for powders produced for this evaluation.

Table 20- Aerosol properties of DPPC:DOPC:SD-30:Leucine:NaCl

| Formulation Number | Formulation components | | | | | Fine particle fraction | | | | gPSD (VMGD 1 bar, um) |
|---|---|---|---|---|---|---|---|---|---|---|
| | DPPC | DOPC | SD-30 | L-leu | NaCl | Size 00 | | Size 2 | | |
| | | | | | | <5.6 um (%) | <3.4 um (%) | <5.6 um (%) | <3.4 um (%) | |
| 33 | 70% | 10% | 0% | 18% | 2% | 42 | 24 | 52 | 30 | 6.6 |
| 46 | 60% | 20% | 0% | 18% | 2% | No powder produced | | | | |
| 34 | 60% | 10% | 20% | 8% | 2% | 53 | 38 | 78 | 48 | 5 |
| 35 | 60% | 20% | 10% | 8% | 2% | 55 | 30 | 60 | 36 | 5.2 |
| 36 | 80% | 10% | 8% | 0% | 2% | 50 | 32 | 63 | 43 | 6.8 |

Table 21- Solid state properties of DPPC:DOPC:SD-30:Leucine:NaCl

| Formulation Number | Description | XRD | TGA-120 (%) | Low T1 (°C) | Low T2 (°C) |
|---|---|---|---|---|---|
| 33 | DPPC:DOPC:Leu:NaCl (70:10:18:2) | SC-DE | 1.67 | n.c. | 69.9, 78.1, 88.3, 111.8 |
| 34 | DPPC:DOPC:SD-30:Leu:NaCl (60:10:20:8:2) | SC-D | 1.3 | 41.5 | 68.1, 74.7 |
| 35 | DPPC:DOPC:SD-30:Leu:NaCl (60:20:10:8:2) | SC-D | 1.25 | 53.1 | 67.1 |
| 36 | DPPC:DOPC:SD-30:NaCl (80:10:8:2) | SC-D | 1.31 | n.c. | 70.7, 96 |

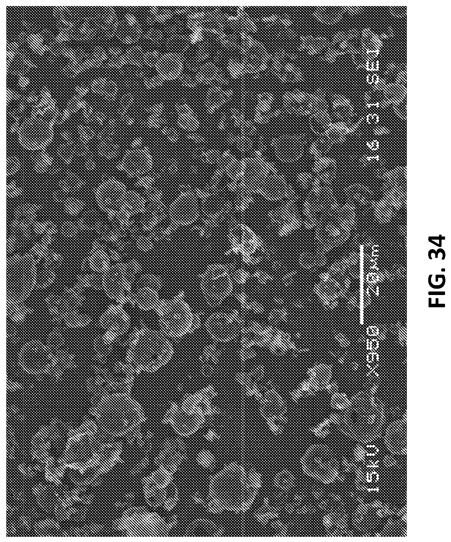

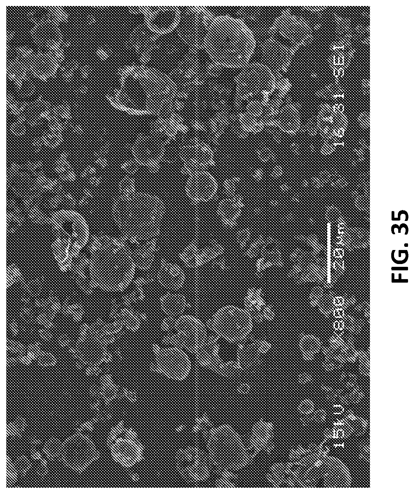

FIGs. 32 and 33 are each SEM images of Formulation 33. FIGS. 34 and 35 are each SEM images of Formulation 34.

**3.5 DPPC:DOPC:POPC:SD-30:NaCl (50:10:10:28:2)** (not according to the claims)

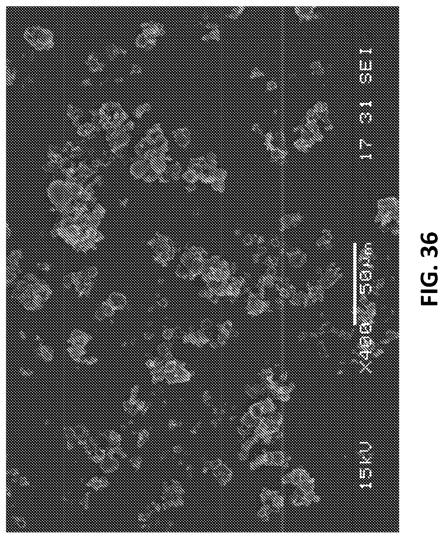

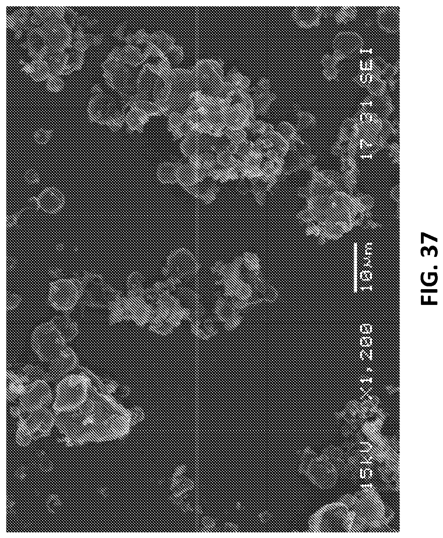

**POPC was introduced into the DPPC-based formulation. The process parameters used to spray dry these powders are found in Table 3. The powder contained particles that tend to stick together (FIGs. 36 and 37).**

Table 22 summarizes the aerosol properties and Table 23 summarizes the solid state properties for powders produced for this evaluation.

Table 22- Aerosol properties of DPPC:DOPC:POPC:SD-30:NaCl (50:10:10:28:2)

| Formulation Number | Formulation components | | | | | Fine particle fraction | | | | gPSD (VMGD 1 bar, um) |
|---|---|---|---|---|---|---|---|---|---|---|
| | DPPC | DOPC | POPC | SD-30 | NaCl | Size 00 | | Size 2 | | |
| | | | | | | <5.6 um (%) | <3.4 um (%) | <5.6 um (%) | <3.4 um (%) | |
| 1 | 50% | 10% | 10% | 28% | 2% | 60 | 40 | 73 | 50 | 5.5 |

**Table 23**- Solid state properties of DPPC:DOPC:POPC:SD-30:NaCl (50:10:10:28:2)

| Formulation Number | Description | XRD | TGA-120 (%) | Low T1 (°C) | Low T2 (°C) |
|---|---|---|---|---|---|
| 1 | DPPC:DOPC:POPC:SD-30:NaCl (50:10:10:28:2) | SC-D | 1.33 | 52.9 | 65.4 |

**Table 24**- Aerosol properties of DPPC:POPC (70:30)

| Formulation Number | Description | Fine particle fraction | | gPSD (VMGD 1 bar, um) |
|---|---|---|---|---|
| | | Size 2 | | |
| | | <5.6 um (%) | <3.4 um (%) | |
| 38 | DPPC:POPC (70:30) | 52 | 44 | 4.2* |
| *Non-uniform particles | | | | |

FIGs. 36 and 37 are each SEM images of Formulation 1.

**3.6 DPPC:DOPC:POPC:SD-30- Buchi B-290** (not according to the claims)

Process parameters are shown in Table 25 and Table 26. Table 27 summarizes the aerosol properties of the powder produced for this evaluation.

Table 25- Process parameters for spray drying Formulation 47

| Process Parameters- Buchi B-290 | | | | |
|---|---|---|---|---|
| Total solid concentration (g/L) | 1 | | Aqueous Flow (mL/min) | 0 |
| Inlet Temperature (°C) | 95 | | Organic flow (mL/min) | 30 |
| Outlet Temperature (°C) | 40 | | Product filter purge gas (scfh) | NA |
| Drying Gas Rate (kg/hr) | 23 | | Air cap # | 46375 |
| Atomization Gas Flow (mm) | 50 | | **Fluid** cap # | 44554 |

**Table 26**- Process parameters for spray drying Formulation 61

| Process Parameters- Buchi B-290 | | | | |
|---|---|---|---|---|
| Total solid concentration (g/L) | 1 | | Aqueous Flow (mL/min) | 6 |
| Inlet Temperature (°C) | 130 | | Organic flow (mL/min) | 24 |
| Outlet Temperature (°C) | 40 | | Product filter purge gas (scfh) | NA |
| Drying Gas Rate (kg/hr) | 23 | | Air cap # | 46375 |
| Atomization Gas Flow (mm) | 50 | | Fluid cap # | 44554 |

**Table 27**- Aerosol properties of DPPC:POPC (95:5) and DPPC:DOPC: SD-30 (85:5:10) produced with Buchi B-290

| Formula tion Number | Composition | | | | Fine particle fraction | | gPSD (VMGD 1 bar, um) |
|---|---|---|---|---|---|---|---|
| | DPPC | DOPC | POPC | SD-30 | Size 2 | | |
| | | | | | <5.6 um (%) | <3.4 um (%) | |
| 47 | 95% | 5% | 0% | 0% | 29 | 24 | 3.5 |
| 61 | 85% | 5% | 0% | 10% | 56 | 39 | 2.7 |
| 44 | 70% | 0% | 30% | 0% | 52 | 44 | 4.2 |

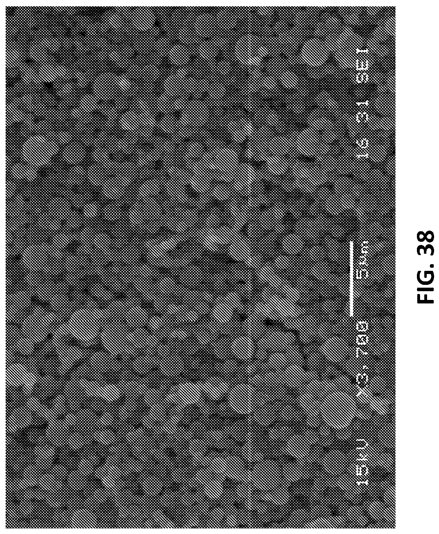

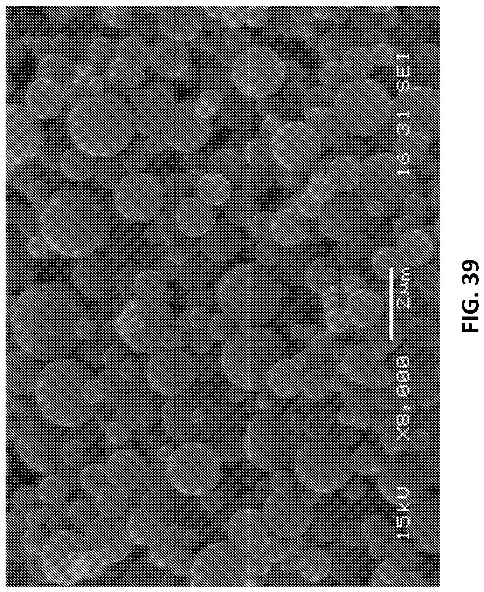

FIGs. 38 and 39 are each SEM images of Formulation 47.

### 3.7 DPPC:DOPC:POPC:DPPE:DPPG-Na:POPG-Na:SD-30:NaCl (not according to the claims)

A mixture of lipids was spray dried to replicate the components of bovine surfactant from lung lavage fluid. The process parameters used to spray dry these powders are found in Table 3. This resulted in a powder with a high FPF and sticky particles.

Table 28 summarizes the powder composition,

Table 29 summarizes the aerosol properties and Table 30 summarizes the solid state properties the powder produced for this evaluation.

Table 28- Composition of Formulation 2

| Component | Percent composition |
|---|---|
| DPPC | 32.8% |
| DOPC | 13.3% |
| POPC | 13.3% |
| DPPE | 2.6% |
| DPPG-Na | 4.0% |
| POPG-Na | 4.0% |
| Stabilite SD-30 | 28.0% |
| Sodium chloride | 2.0% |

Table 29- Aerosol properties of Formulation 2

| Formulation Number | Fine particle fraction | | | | gPSD (VMGD 1 bar, mm) |
|---|---|---|---|---|---|
| | Size 00 | | Size 2 | | |
| | <5.6 um (%) | <3.4 um (%) | <5.6 um (%) | <3.4 um (%) | |
| 2 | 67 | 51 | 76 | 57 | 7.4 |

Table 30- Solid state properties of Formulation 2

| Formulation Number | XRD | TGA-120 (%) | Low T1 (°C) | Low T2 (°C) |
|---|---|---|---|---|
| 2 | SC-D | 1.02 | 42.9, 60.5 | n/a |

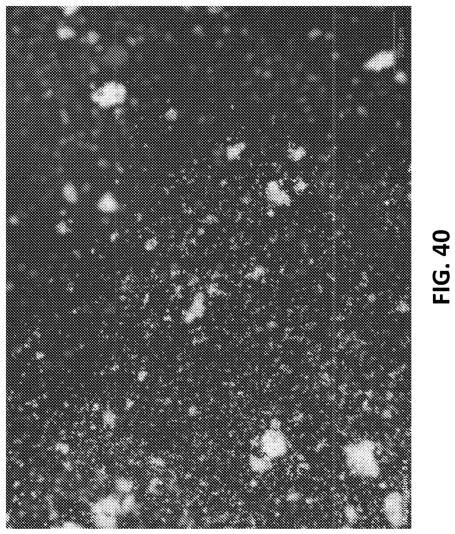

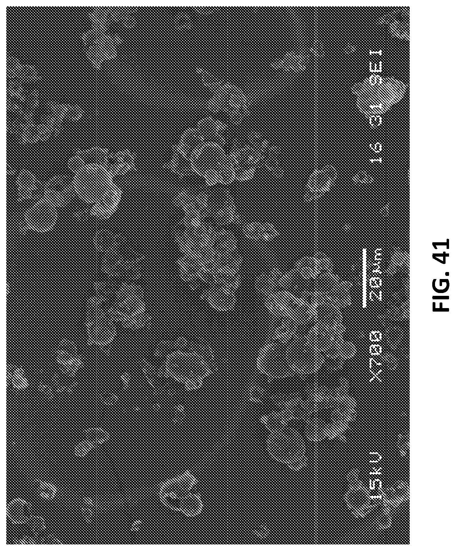

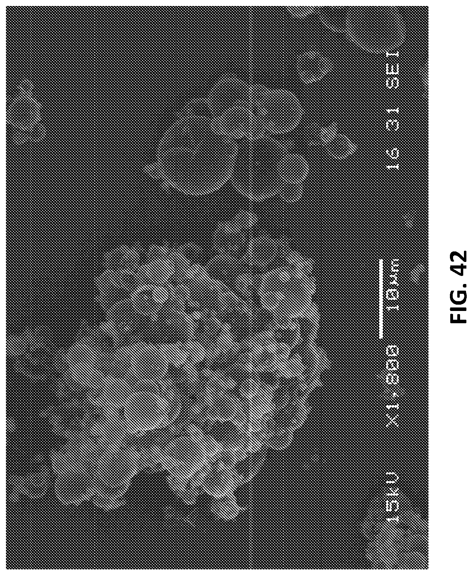

FIG. 40 is a cross polarized image of Formulation 2 in oil at 5x. Figs. 41 and 42 are each SEM images of Formulation 2.

### 3.8 DPPC:POPC:DPPG-Na:POPG-Na:CaCl$_2$:Mg Lactate:SD-30:NaCl (not according to the claims)

CaCl$_2$ and Mg lactate were incorporated into the DPPC-based formulation to determine whether the solid state properties and particle formation may be enhanced. The process parameters used to spray dry these powders are found in Table 3. This resulted in particles with a small geometric size. The powders contained multiple overlapping thermal events.

Table 31 summarizes the composition of powders,

Table 32 summarizes the aerosol properties and Table 33 summarizes the solid state properties the powders produced for this evaluation.

Table 31- Composition of Formulations 3, 5 and 6

| Formulation Number | Formulation components | | | | | | | |
|---|---|---|---|---|---|---|---|---|
| | DPPC (%) | POPC (%) | DPPG-Na (%) | POPG-Na (%) | CaCl$_2$ (%) | Mg Lactate (%) | SD-30 (%) | NaCl (%) |
| 3 | 50 | 0 | 10 | 10 | 6.9 | 0 | 21.1 | 2 |

(continued)

| Formulation Number | Formulation components | | | | | | | |
|---|---|---|---|---|---|---|---|---|
| | DPPC (%) | POPC (%) | DPPG-Na (%) | POPG-Na (%) | CaCl₂ (%) | Mg Lactate (%) | SD-30 (%) | NaCl (%) |
| 5 | 42 | 14 | 0 | 14 | 5 | 0 | 23 | 2 |
| 6 | 42 | 14 | 0 | 14 | 0 | 28 | 0 | 2 |

**Table 32**- Aerosol properties of Formulations 3, 5 and 6

| Formulation Number | Fine particle fraction | | | | gPSD (VMGD 1 bar, um) |
|---|---|---|---|---|---|
| | Size 00 | | Size 2 | | |
| | <5.6 um (%) | <3.4 um (%) | <5.6 um (%) | <3.4 um (%) | |
| 3 | 74 | 62 | 65 | 50 | 1.7 |
| 5 | 79 | 59 | 80 | 63 | 4 |
| 6 | 71 | 52 | 78 | 57 | 2.3 |

**Table 33**- Solid state properties of lots 224154, 224163, and 224164

| Formulation Number | TGA-120 (%) | Low T1 (°C) | Low T2 (°C) |
|---|---|---|---|
| 3 | 2.78 | 50.3 | 66.6, 102.4* |
| 5 | 1.16 | 53.9 | 61.5, 111.9* |
| 6 | 2.45 | 51 | 60.8, 75, 94 |
| *Multiple overlapping thermal events | | | |

### 3.9 DPPC-based formulations with fluorescent dye additive- Buchi B290 (not according to the claims)

Formulations 56, 57, 58, 59 and 60 were spray dried to supply powder for surface tension measurements that will allow for fluorescent dye: lipid content correlations. Table 34 summarizes the composition of powders for the powders produced for this evaluation.

**Table 34**- Compositions of lipid formulations containing fluorescent dye

| Formulation Number | Formulation components | | | | | | |
|---|---|---|---|---|---|---|---|
| | DPPC (%) | POPC (%) | POPG-Na (%) | Oregon Dye (%) | Fluorescein Dye (%) | SD-30 (%) | NaCl (%) |
| 56 | 80 | 0 | 0 | 0.5 | 0 | 17.5 | 2 |
| 57 | 80 | 0 | 0 | 0 | 0.5 | 17.5 | 2 |
| 58 | 80 | 0 | 0 | 0 | 2 | 16 | 2 |
| 59 | 42 | 14 | 14 | 0 | 0.5 | 27.5 | 2 |
| 60 | 40 | 24 | 16 | 0 | 0.5 | 17.5 | 2 |

### 3.10 DPPC:POPC:POPG-Na:Leucine:SD-30:NaCl (not according to the claims)

The effect of leucine and SD-30 as an excipient for various ratios of DPPC:POPC:POPG-Na was investigated. The process parameters used to spray dry these powders are found in Table 3. Formulation 7 has a high FPF but an abnormally high average geometric size. This suggests a large amount of small particles that agglomerated into larger masses. Table 35 summarizes the composition of powders, Table 36 summarizes the aerosol properties and Table 37 summarizes the solid state properties the powders produced for this evaluation.

**Table 35**- Composition of Formulations 8, 11, 7, 9, and 4,

| Formulation Number | Formulation components | | | | | |
|---|---|---|---|---|---|---|
| | DPPC | POPC | POPG-Na | L-leu | SD-30 | NaCl |
| 8 | 40% | 24% | 16% | 18% | 0% | 2% |
| 11 | 44% | 18% | 18% | 18% | 0% | 2% |
| 7 | 40% | 24% | 16% | 0% | 18% | 2% |
| 9 | 44% | 18% | 18% | 0% | 18% | 2% |
| 4 | 42% | 14% | 14% | 0% | 28% | 2% |

**Table 36-** Aerosol properties of Formulations 8, 11, 7, 9, and 4.

| Formulation Number | Fine particle fraction | | | | gPSD (VMGD 1 bar, um) |
|---|---|---|---|---|---|
| | Size 00 | | Size 2 | | |
| | <5.6 um (%) | <3.4 um (%) | <5.6 um (%) | <3.4 um (%) | |
| 8 | 53 | 38 | 64 | 42 | 10.3 |
| 11 | 62 | 46 | 69 | 47 | 13.5 |
| 7 | 71 | 56 | 78 | 59 | 42.8* |
| 9 | 78 | 59 | 79 | 64 | TBA |
| 4 | 73 | 56 | 75 | 58 | 6.5 |
| *This has been retested several times to confirm. | | | | | |

**Table 37**- Solid state properties of Formulations 8, 11, 7, 9, and 4.

| Formulation Number | XRD | TGA-120 (%) | Low T1 (°C) | Low T2 (°C) |
|---|---|---|---|---|
| 8 | SC-DE | 1.35 | 42.5 | 53, 58.7, 69.7 |
| 11 | SC-DE | 0.24 | 48.5 | 61.5, 65.3,72.4, 121.7 |
| 7 | SC-D | 0.88 | 38.6 | 56.2 |
| 9 | SC-D | 0* | 42.3 | 59.9 |
| 4 | SC-D | 1.07 | 41.6 | 58.1 |
| *Noisy baseline may interfere with actual value | | | | |

**3.11 DPPC:POPC:DPPG-Na:POPG-Na:Leucine:SD-30:NaC** (not according to the claims) 1

The effect of leucine and SD-30 as an excipient for various ratios of DPPC:POPC:DPPG-Na:POPG-Na was investigated. The process parameters used to spray dry these powders are found in Table 3. The formulation with SD-30 as the excipient has higher FPF values. Table 38 summarizes the composition of powders, Table 39 summarizes the aerosol properties and
Table 40 summarizes the solid state properties the powders produced for this evaluation.

**Table 38-** Composition of Formulation numbers 10 and 12.

| Formulation Number | Formulation components | | | | | | |
|---|---|---|---|---|---|---|---|
| | DPPC | POPC | DPPG-Na | POPG-Na | L-leu | SD-30 | NaCl |
| 10 | 44% | 18% | 9% | 9% | 0% | 18% | 2% |
| 12 | 44% | 18% | 9% | 9% | 18% | 0% | 2% |

**Table 39-** Aerosol properties of Formulations 10 and 12.

| Formulation Number | Fine particle fraction | | | | gPSD (VMGD 1 bar, um) bar, um) |
|---|---|---|---|---|---|
| | Size 00 | | Size 2 | | |
| | <5.6 um (%) | <3.4 um (%) | <5.6 um (%) | <3.4 um (%) | |
| 10 | 79 | 66 | 78 | 65 | 8.1 |
| 12 | 59 | 43 | 59 | 41 | 10.7 |

**Table 40-** Solid state properties of Formulations 10 and 12.

| Formulation Number | XRD | TGA-120 (%) | Low T1 (°C) | Low T2 (°C) |
|---|---|---|---|---|
| 10 | SC-D | 0.12 | 42.5 | 63.1 |
| 12 | SC-DE | 0.73 | n.c. | 64.8, 74.5, 121.1 |

**3.12 DPPC:POPG-Na:Leucine:SD-30:NaCl** (not according to the claims)

The effect of leucine and SD-30 as an excipient for various ratios of DPPC:POPG-Na was investigated. The process parameters used to spray dry these powders are found in Table 3. The formulation with SD-30 as the excipient has higher FPF values. Table 41 summarizes the composition of powders,

Table 42 summarizes the aerosol properties and Table 43 summarizes the solid state properties the powders produced for this evaluation.

**Table 41-** Formulation components of Formulations 13 and 15.

| Formulation Number | Formulation components | | | | |
|---|---|---|---|---|---|
| | DPPC | POPG-Na | L-leu | SD-30 | NaCl |
| 13 | 56% | 24% | 0% | 18% | 2% |
| 15 | 56% | 24% | 18% | 0% | 2% |

**Table 42-** Aerosol properties of Formulations 13 and 15.

| Formulation Number | Fine particle fraction | | | | gPSD (VMGD 1 bar, um) |
|---|---|---|---|---|---|
| | Size 00 | | Size 2 | | |
| | <5.6 um (%) | <3.4 um (%) | <5.6 um (%) | <3.4 um (%) | |
| 13 | 72 | 61 | 81 | 68 | 8.8 |
| 15 | 64 | 46 | 75 | 57 | 8.7 |

**Table 43-** Solid state properties of Formulations 13 and 15.

| Formulation Number | XRD | TGA-120 (%) | Low T1 (°C) | Low T2 (°C) |
|---|---|---|---|---|
| 13 | SC-D | 0.44* | 41.3 | 63.9 |
| 15 | SC-DE | 0.07 | 51.6 | 65.7, 75.5, 124.9 |
| *Noisy baseline may interfere with actual value | | | | |

**3.13 DPPC:DPPG-Na:POPG-Na:Leucine:SD-30:NaCl** (not according to the claims)

The effect of leucine and SD-30 as an excipient for various ratios of DPPC:DPPG-Na:POPG-Na was investigated. The process parameters used to spray dry these powders are found in Table 3. The formulation with SD-30 as the excipient has higher FPF values.

**Table 44 summarizes the composition of powders, Table 45 summarizes the aerosol properties and** Table 46 summarizes the solid state properties the powders produced for this evaluation.

**Table 44-** Composition of Formulations 14 and 16.

| Formulation Number | Formulation components | | | | | |
|---|---|---|---|---|---|---|
| | DPPC | DPPG-Na | POPG-Na | L-leu | SD-30 | NaCl |
| 14 | 56% | 8% | 16% | 0% | 18% | 2% |
| 16 | 56% | 8% | 16% | 18% | 0% | 2% |

**Table 45**- Aerosol properties

| Formulation Number | Fine particle fraction | | | | gPSD (VMGD 1 bar, um) |
|---|---|---|---|---|---|
| | Size 00 | | Size 2 | | |
| | <5.6 um (%) | <3.4 um (%) | <5.6 um (%) | <3.4 um (%) | |
| 14 | 77 | 69 | 85 | 75 | 8.4 |
| 16 | 63 | 46 | 78 | 59 | 8 |

**Table 46-** Solid state properties

| Formulation Number | XRD | TGA-120 (%) | Low T1 (°C) | Low T2 (°C) |
|---|---|---|---|---|
| 14 | SC-D | 0.31 | 41.9 | 66.7 |
| 16 | SC-DE | 1.15 | 49.1 | 65.2, 76.2, 122.9 |

**3.14 DPPC:DPPE:DPPG-Na:POPG-Na:Leucine:SD-30:NaCl** (not according to the claims)

The effect of leucine and SD-30 as an excipient for various ratios of DPPC:DPPE:DPPG-Na:POPG-Na was investigated. The process parameters used to spray dry these powders are found in Table 3. The formulation with SD-30 as the excipient has higher FPF values. Table 47 summarizes the composition of powders, Table 48 summarizes the aerosol properties and Table 49 summarizes the solid state properties the powders produced for this evaluation.

**Table 47**- Composition of Formulations 17 and 18

| Formulation Number | Formulation components | | | | | | |
|---|---|---|---|---|---|---|---|
| | DPPC | DPPE | DPPG-Na | POPG-Na | L-leu | SD-30 | NaCl |
| 17 | 40% | 24% | 8% | 8% | 0% | 18% | 2% |
| 18 | 40% | 24% | 8% | 8% | 18% | 0% | 2% |

**Table 48-** Aerosol properties

| Formulation Number | Fine particle fraction | | | | gPSD (VMGD 1 bar, um) |
|---|---|---|---|---|---|
| | Size 00 | | Size 2 | | |
| | <5.6 um (%) | <3.4 um (%) | <5.6 um (%) | <3.4 um (%) | |
| 17 | 70 | 51 | 64 | 40 | 8.6 |
| 18 | 50 | 33 | 46 | 29 | 12.6 |

**Table 49-** Solid state properties

| Formulation Number | XRD | TGA-120 (%) | Low T1 (°C) | Low T2 (°C) |
|---|---|---|---|---|
| 17 | SC | 0.18* | 36 | 61.7, 78.1 |
| 18 | SC-DE | 1.06 | 49 | 62.5, 72.2, 83.1 |
| *Noisy baseline may interfere with actual value | | | | |

**3.15 DPPC:DOPC:POPC:Leu:NaCl** (not according to the claims)

Phosphocholine-based formulations were spray dried. Since powder did not pulse off the filter bags, the powder was scraped off of the filter bag. The process parameters used to spray dry these powders are found in Table 14. Table 50 summarizes the composition of powders.

Table 51 summarizes the aerosol properties and Table 52 summarizes the solid state properties the powders produced for this evaluation.

**Table 50**- Composition of Formulations 48 and 19.

| Formulation Number | Formulation components | | | | | Note |
|---|---|---|---|---|---|---|
| | DPPC | DOPC | POPC | Leu | NaCl | |
| 48 | 60% | 20% | 0% | 18% | 2% | Scraped off filter bag |
| 19 | 60% | 0% | 20% | 18% | 2% | Scraped off filter bag |

**Table 51-** Aerosol properties

| Formulation Number | Fine particle fraction | | gPSD (VMGD 1 bar, um) |
|---|---|---|---|
| | Size 2 | | |
| | <5.6 um (%) | <3.4 um (%) | |
| 48 | 33 | 20 | 10.5* |
| 19 | 71 | 45 | 5.7 |
| *Nonuniform particles | | | |

**Table 52-** Solid state properties

| Formulation Number | XRD | TGA-120 (%) | Low T1 (°C) | Low T2 (°C) |
|---|---|---|---|---|
| 48 | SC-D | 1.69 | 46.9 | 64.8, 74.5, 109 |
| 19 | SC-DE | 1.42 | 49.4 | 67, 75.1, 90.3 |
| *Noisy baseline may interfere with actual value | | | | |

**3.16 DPPC:DPPG-Na:POPG-Na:Albumin:NaCl** (not according to the claims)

Albumin as an excipient for lipid-based formulations was evaluated. The incorporation of unsaturated lipids produced powders that had to be scraped off the filter bag. The process parameters used to spray dry these powders are found in Table 14. Table 53 summarizes the composition of powders, Table 54 summarizes the aerosol properties and Table 55 summarizes the solid state properties the powders produced for this evaluation.

**Table 53-** Composition of lots with albumin as the excipient

| Formulation Number | Formulation components | | | | | Note |
|---|---|---|---|---|---|---|
| | DPPC | DPPG-Na | POPG-Na | Albumin | NaCl | |
| 39 | 95% | 0% | 0% | 5% | 0% | NA |
| 40 | 93% | 0% | 0% | 5% | 2% | NA |
| 62 | 60% | 10% | 25% | 5% | 0% | Scraped off |

(continued)

| Formulation Number | Formulation components | | | | | Note |
|---|---|---|---|---|---|---|
| | DPPC | DPPG-Na | POPG-Na | Albumin | NaCl | |
| 21 | 58% | 10% | 25% | 5% | 2% | filter bag |
| 22 | 80% | 0% | 15% | 5% | 0% | |
| 23 | 70% | 0% | 25% | 5% | 0% | |
| 24 | 60% | 0% | 35% | 5% | 0% | |
| 45 | 50% | 0% | 45% | 5% | 0% | |
| 25 | 78% | 0% | 15% | 5% | 2% | |
| 26 | 68% | 0% | 25% | 5% | 2% | |
| 27 | 58% | 0% | 35% | 5% | 2% | NA |
| 49 | 48% | 0% | 45% | 5% | 2% | No powder produced |

**Table 54-** Aerosol properties of lots with albumin as the excinient

| Formulation Number | Fine particle fraction | | gPSD (VMGD 1 bar, um) |
|---|---|---|---|
| | Size 2 | | |
| | <5.6 um (%) | <3.4 um (%) | |
| 39 | 67 | 37 | 9.1* |
| 40 | 77 | 52 | 9.9* |
| 62 | 48 | 29 | 17.4* |
| 21 | 57 | 36 | 30.3* |
| 22 | 61 | 33 | 8 |
| 23 | 55 | 28 | 16.4 |
| 24 | 48 | 26 | 12.4* |
| 45 | 48 | 26 | 9.1 |
| 25 | 60 | 36 | 13.2* |
| 26 | 41 | 21 | 26.2 |
| 27 | 53 | 32 | 11.3 |
| 49 | *No powder produced* | | |
| *Nonuniform particles | | | |

**Table 55-** Solid state properties of lots with albumin as the excipient

| Formulation Number | XRD | TGA-120 (%) | Low T1 (°C) | Low T2 (°C) |
|---|---|---|---|---|
| 39 | SC-D | 0.91 | 34 | 73.4 |
| 40 | SC-D | 1.41 | n.c. | 74.7, 95.8, 119.7 |
| 62 | SC-D | 0.53 | n.c. | 64.5, 71.9 |
| 21 | SC-D** | 0.11* | n.c. | 62.2, 69.7, 121.5 |
| 22 | SC-D | 1.26 | n.c. | 67.7, 71.6 |
| 23 | SC-D | 0.54 | 37.2 | 73.6, 75.3 |
| 24 | *Not enough powder to test* | | | |
| 45 | *Not enough powder to test* | | | |

(continued)

| Formulation Number | XRD | TGA-120 (%) | Low T1 (°C) | Low T2 (°C) |
|---|---|---|---|---|
| 25 | SC-D** | 0.93 | n.c. | 70.5, 84.9, 116.8 |
| 26 | SC-D** | 0.71 | n.c. | 68.2, 70.3, 118 |
| 27 | SC-D** | 0.58 | n.c. | 67.5, 115.4 |
| 49 | No powder produced | | | |
| *Noisy baseline may interfere with actual value<br>**Diffraction observed at 32 2theta | | | | |

### 3.17 DPPC:Lactose:Mannitol:NaCl (not according to the claims)

Lactose and mannitol as an excipient for a DPPC-based formulation was evaluated. This resulted in powders with high FPF values. The process parameters used to spray dry these powders are found in Table 14. Table 56 summarizes the composition of powders, Table 57 summarizes the aerosol properties and Table 58 summarizes the solid state properties the powders produced for this evaluation.

**Table 56-** Composition of formulations 41 and 42.

| Formulation Number | Formulation components | | | |
|---|---|---|---|---|
| | DPPC | Lactose | Mannitol | NaCl |
| 41 | 80% | 18% | 0% | 2% |
| 42 | 80% | 0% | 18% | 2% |

**Table 57-** Aerosol properties

| Formulation Number | Fine particle traction | | gPSD (VMGD 1 bar, um) |
|---|---|---|---|
| | Size 2 | | |
| | <5.6 um (%) | <3.4 um (%) | |
| 41 | 85 | 70 | 9.1* |
| 42 | 86 | 75 | 7.4* |
| *Nonuniform particles | | | |

**Table 58-** Solid state properties

| Formulation Number | XRD | TGA-120 (%) | Low T1(°C) | Low T2 (°C) |
|---|---|---|---|---|
| 41 | SC-D | 0.95 | 40.4 | 61.6 |
| 42 | SC-D | 0.86 | 40.3 | 73 |

### 3.18 DPPC:Trehalose:NaCl (not according to the claims)

Trehalose as an excipient for a DPPC-based formulation was evaluated. The atomization gas flow rate was varied to determine the effects on the aerosol properties. As the atomization gas flow rate decreased, the FPF decreased slightly and the gPSD increased. The process parameters used to spray dry these powders are found in Table 14. Table 59 summarizes the composition of powders, Table 60 summarizes the aerosol properties and Table 61 summarizes the solid state properties the powders produced for this evaluation.

**Table 59-** Composition of lots with trehalose as the excipient

| Formulation Number | Formulation components | | | Atomization gas (g/min) | |
|---|---|---|---|---|---|
| | DPPC | Trehalose | NaCl | | Note |
| 20-1 | 80% | 18% | 2% | 20 | Scraped off filter bag |
| 20-2 | 80% | 18% | 2% | 15 | Scraped off filter bag |
| 20-3 | 80% | 18% | 2% | 10 | Scraped off filter bag |

**Table 60-** Aerosol properties of lots with trehalose as the excipient

| Formulation Number | Fine particle fraction | | gPSD (VMGD 1 bar, um) |
|---|---|---|---|
| | Size 2 | | |
| | <5.6 um (%) | <3.4 um (%) | |
| 20-1 | 83 | 68 | 4.4 |
| 20-2 | 77 | 56 | 5.8 |
| 20-3 | 74 | 49 | 8.1 |

**Table 61-** Solid state properties of lots with trehalose as the excipient

| Formulation Number | XRD | TGA-120 (%) | Low T1 (°C) | Low T2 (°C) |
|---|---|---|---|---|
| 20-1 | SC-D | 1.19 | 41.6 | 64.7 |
| 20-2 | *Not enough powder to test* | | | |
| 20-3 | *Not enough powder to test* | | | |

**3.19 Lipids:NaCl** (not according to the claims)

Lipids were spray dried with up to 2% NaCl. This resulted in waxy material which coated the stainless steel pipes. This emphasizes the need for excipients in order to produce powders with superior aerosol properties. The process parameters used to spray dry these powders are found in Table 14. There was not enough of Formulation 43 for solid state testing. Table 62 summarizes the aerosol properties and Table 63 summarizes the solid state properties the powders produced for this evaluation.

**Table 62-** Composition of lipid-based formulations

| Formulation Number | Formulation components | | | | | | |
|---|---|---|---|---|---|---|---|
| | DPPC | POPC | DPPE | DPPG-Na | POPG-Na | NaCl | Note |
| 43 | 70% | 28% | 0% | 0% | 0% | 2% | NA |
| 44 | 70% | 30% | 0% | 0% | 0% | 0% | No powder pro-duced- waxy ma-terial coated pipes |
| 50 | 68% | 0% | 10% | 0% | 20% | 2% | |
| 51 | 68% | 0% | 0% | 10% | 20% | 2% | |
| 63 | 70% | 0% | 0% | 0% | 30% | 0% | |
| 64 | 68% | 0% | 0% | 0% | 30% | 2% | |

**Table 63-** Aerosol properties of lipid-based formulations

| Formulation Number | Fine particle fraction | | gPSD (VMGD 1 bar, um) |
|---|---|---|---|
| | Size 2 | | |
| | <5.6 um (%) | <3.4 um (%) | |
| 43 | 7 | 2 | 25.1* |

**Example 4: Emitted Dose Testing (EDT): Constant Flow and Breath Simulation.**

A study was initiated to screen through various capsule based low flow aerosolization chamber (LFAC) designs to identify candidate designs that are capable of dispersing powders at the low flow rates and actuation times required for use in treating nRDS and other diseases of the lungs associated with reduced pulmonary function. The following aerosolization chamber designs were included within the scope of this study:

- ARCUS Size 00 Inhaler (Size 00 Capsule)
- Size 00 LFAC Engine (Size 00 Capsule)
- ARCUS Size 2 Inhaler (Size 2 Capsule)
- Size 2 LFAC Engine (Size 2 Capsule)
- Size 0 LFAC Engine ( Size 0 Capsule)

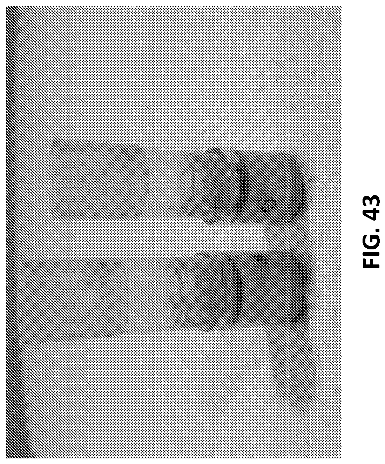

Model LS powders consisting of 80:18:2 DPPC:leucine:NaCl were used for this evaluation. The details of the powder, inhaler and LFAC engine lots utilized in this study are summarized in Table 66, with examples of LFAC engines (size 0 and 00) shown in Figure 43.

**Table 66: Inhalers Engines and Powders** (not according to the claims)

| Inhaler/Engine | Design Description | Inhaler Resistance $(( \frac{\sqrt{cmH_2O}}{LPM} ))$ | Powder Lot |
|---|---|---|---|
| ARCUS-00 | 4 vents, Capsule Size 00 | 0.208 | 80:18:2 DPPC:l-leucine Formulation 31 |
| LFAC-00 | 4 vents, Capsule Size 00 | 0.221 | 80:20 DPPC:l-leucine Formulation 37-16 |
| ARCUS-2 | 6 vents, Capsule Size 2 | 0.165 | 80:18:2 DPPC:l-leucine Formulation 31 |
| LFAC-2 | 6 vents, Capsule Size 2 | 0.164 | 80:20 DPPC:l-leucine Formulation 37-16 |
| LFAC-0 | 4 vents, Capsule Size 0 | 0.223 | 80:20 DPPC:l-leucine Formulation 37-16 |

Emitted Dose at varying flow rates through these devices was assessed. Filled capsule were weighed before actuation. For the case of the LFAC engines that do not possess a capsule puncturing mechanism, capsules were punctured using the corresponding sized inhaler. In particular, size 00 capsules and size 0 capsules were punctured using the Size 00 ARCUS inhaler and size 2 capsules were punctured using the Size 2 ARCUS inhaler. Flow was actuated for 1.0 seconds using a flow controller at various flow rates. Capsules were removed from the inhaler or the LFAC engine and weighed after the first actuation. The same capsule was put back in the inhaler or LFAC engine, actuated, and weighed (to prevent re-puncture of capsules for the inhaler test arms, the operator pinched the mouthpiece while snapping it to the body to prevent actuation of the staple puncturing mechanism). This process was repeated so that each capsule was actuated and weighed a total of five times. Finally, capsules were weighed after cleaning.

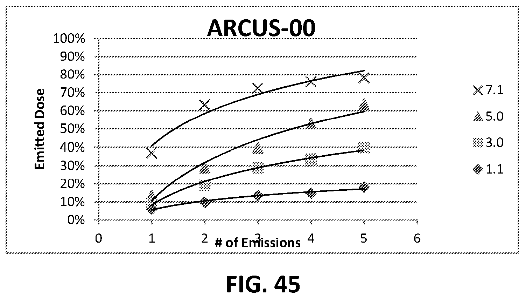

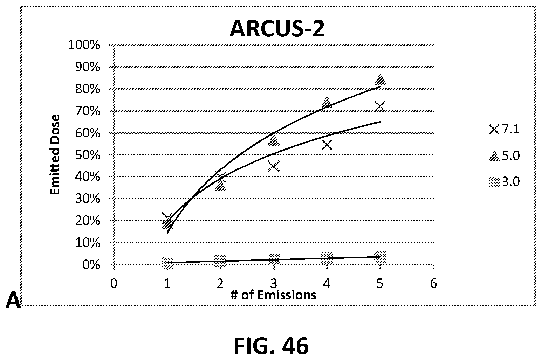

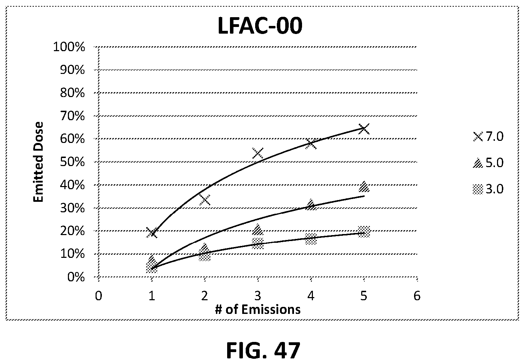

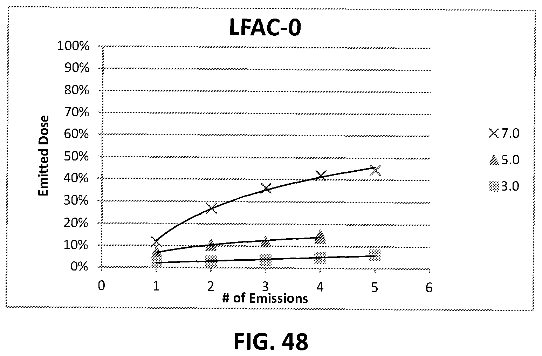

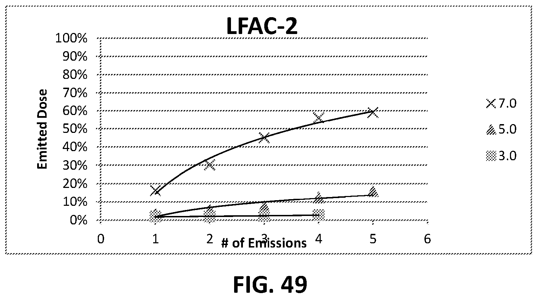

Results for this series of tests are summarized in Table 67 and Figures 45-49. At a flow rate of 7 lpm, both the ARCUS inhalers and the three LFAC engines performed well, with EDs approaching or exceeding 50% observed for all devices. At lower flow rates, higher EDs were observed for the ARCUS devices over the LFAC engines; however, for the lowest flowrate tested (3 lpm), a higher ED was observed for the LFAC 00 engine than for the ARCUS Size 2 device, with the LFAC-00 device performing relatively well in comparison to the ARCUS inhalers across all flowrates. With respect to the LFAC engines, these engines can be easily incorporated into conventional tubing systems associated with lung ventilation delivery systems (CPAP systems etc.) in contrast to the ARCUS inhalers, with the LFAC-00 engine also having the additional advantage of possessing the highest capsule fill weight capacity (approaching 100 mg of a low density LS DP powder) of the three LFAC devices. These results indicate that, even at extremely low flow rates (as low as 3 lpm) and short durations of actuation (1 second), the DP LS of this invention can be successfully emitted from both ARCUS and LFAC engine devices.

In terms of the application of these powders and devices to the delivery of a given dose of DP LS in a nRDS setting, delivery times required to administer 90% (i.e., 90% ED) of a given dose are shown can be estimated based on the slopes of the graphs shown in Figures 45-49. For the case of the ARCUS-00 device, it can be estimated that only 7 seconds would be required to emit 90% of the dose at 7 lpm and 12 seconds at 3 lpm. Similarly, for the LFAC-00 device, approximately 7 seconds would be required at 7 lpm, 12 seconds at 5 lpm and 53 seconds at 3 lpm. Thus, these results further indicate that the LS DP powders of this invention filled into capsules at levels up to 100 mg (on the order of typically administered liquid LS doses) possess the potential to be noninvasively delivered via incorporation into a CPAP ventilation device (or a similar noninvasive inhalation support device) over reasonably short delivery times (less than a minute).

Table 67: Emitted Dose Results by Inhaler, Flow rate, and Emission Number. N=3 Capsules per %ED.

| Inhaler/ Flow Rate | Average of %ED @ 1 | StdDev of %ED @ 1 | Average of %ED @ 2 | StdDev of %ED @ 2 | Average of %ED @ 3 | StdDev of %ED @ 3 | Average of %ED @ 4 | StdDev of %ED @ 4 | Average of %ED @ 5 | StdDev of %ED @ 5 |
|---|---|---|---|---|---|---|---|---|---|---|
| **ARCUS00** | | | | | | | | | | |
| 1.1 | 6.09% | 3.46% | 9.70% | 3.75% | 13.49% | 4.54% | 14.73% | 4.21% | 18.04% | 6.77% |
| 3.0 | 9.44% | 7.12% | 19.18% | 14.38% | 28.87% | 23.30% | 33.71% | 26.44% | 39.80% | 29.37% |
| 5.0 | 13.60% | 3.83% | 28.81% | 7.81% | 39.56% | 7.55% | 53.00% | 6.76% | 63.76% | 4.74% |
| 7.1 | 36.87% | 32.04% | 63.34% | 36.18% | 72.41% | 29.72% | 76.05% | 27.75% | 78.10% | 26.20% |
| **ARCUS2** | | | | | | | | | | |
| 3.0 | 0.92% | 0.59% | 1.68% | 0.32% | 2.41% | 0.50% | 3.05% | 0.33% | 3.50% | 0.30% |
| 5.0 | 19.01% | 6.72% | 36.27% | 13.55% | 56.81% | 18.59% | 73.96% | 13.76% | 84.37% | 6.01% |
| 7.1 | 21.18% | 16.47% | 39.90% | 25.45% | 44.91% | 33.60% | 54.64% | 31.81% | 72.03% | 21.20% |
| **LFAC2** | | | | | | | | | | |
| 3.0 | 1.85% | 0.55% | 1.95% | 0.54% | 2.21% | 0.47% | 2.55% | 0.71% | 2.77% | 0.75% |
| 5.0 | 3.23% | 2.52% | 5.66% | 1.66% | 7.42% | 0.50% | 12.35% | 3.33% | 15.72% | 5.74% |
| 7.0 | 16.06% | 1.88% | 30.22% | 14.51% | 45.04% | 11.89% | 56.14% | 7.33% | 59.04% | 9.15% |
| **LFAC0** | | | | | | | | | | |
| 3.0 | 2.41% | 1.14% | 3.03% | 0.81% | 3.70% | 0.88% | 4.74% | 0.75% | 6.18% | 1.19% |
| 5.0 | 6.91% | 0.84% | 10.33% | 3.22% | 12.07% | 3.32% | 13.61% | 3.29% | 15.05% | 3.17% |
| 7.0 | 11.75% | 4.49% | 27.03% | 6.70% | 36.06% | 15.25% | 41.93% | 18.79% | 44.38% | 18.16% |
| **LFAC00** | | | | | | | | | | |
| 3.0 | 4.00% | 1.14% | 9.38% | 5.26% | 14.48% | 9.39% | 16.47% | 9.09% | 19.75% | 8.57% |
| 5.0 | 7.28% | 2.15% | 12.45% | 2.83% | 20.81% | 2.50% | 31.69% | 10.96% | 39.46% | 14.87% |
| 7.0 | 19.31% | 10.89% | 33.61% | 20.08% | 53.85% | 22.20% | 57.90% | 24.36% | 64.26% | 21.95% |

**EXAMPLE 5** - **Emitted Dose testing utilizing a breath simulator.**

An additional set of experiments was conducted to assess the delivery efficacy of the LFAC engines in a setting mimicking passive breathing at low flow rates versus delivering at a constant flow rate (such as that used for CPAP ventilation etc.). A Breath Simulator (Copley Scientific Breath Simulator Model BRS 3000) was utilized to provide a breath profile. Powders to be used for the were tested in for emitted dose using the LFAC-00 and LFAC-2 engines and their corresponding capsules via the following method.

<u>MATERIALS AND METHODS</u>

A. Capsule Preparation

Capsules of two sizes (00 and 2) were prepared for emitted dose testing. The procedure for capsule preparation was as follows:

1. An empty capsule (either Size 00 or Size 2) was weighed. Then the capsule was filled with powder produced in Formulation development.
2. The filled capsule was weighed before emitted dose testing. The capsule was punctured using an appropriately sized inhaler.

B. Emitted Dose (ED) Apparatus Setup and Testing

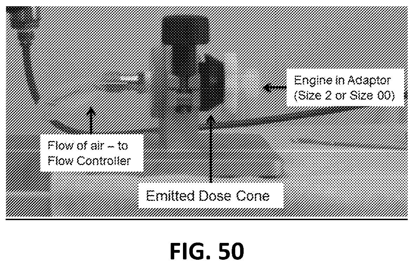

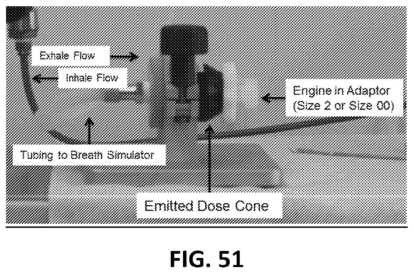

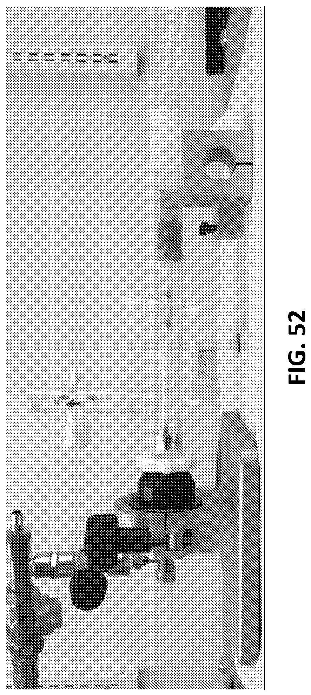

Three types of emitted dose tests were performed: constant flow emitted dose (apparatus set forth in FIG. 50), breath simulator emitted dose (apparatus set forth in FIG. 51), and assisted flow emitted dose (apparatus set forth in FIG. 52).

Constant Flow Emitted Dose was conducted as follows:

1. The emitted dose filter was weighed and inserted into the emitted dose cone.
2. The prepared capsule was inserted into an appropriately sized engine (Size 00 Engine for a Size 00 capsule, and Size 2 Engine for a Size 2 capsule). The engine was connected to the ED cone using an appropriately sized adaptor.
3. The capsule was actuated for a specified amount of time using various flowrates (10, 7 or 5 LPM) of air. The time of actuation was based on the flowrate, such that total volume of airflow experienced by the capsule during actuation was 2 L. Air flowrate was controlled by a flow controller.
4. The capsule was then removed from the engine and weighed.
5. The ED filter was removed from the ED cone and weighed.

Breath Simulator Emitted Dose was conducted as follows:

1. The emitted dose filter was weighed and inserted into the emitted dose cone.
2. The prepared capsule was inserted into an appropriately sized engine (Size 00 Engine for a Size 00 capsule, and Size 2 Engine for a Size 2 capsule). The engine was connected to the ED cone using an appropriately sized adaptor.
3. The capsule was actuated for a specified number of breath cycles at different tidal volumes (62.5, 43.75, or 31.25 mL). The number of breath cycles was based on tidal volume, such that total volume of inhalation experienced by the capsule during actuation was 2 L. Tidal volumes and number of breath cycles was controlled by a Copley Scientific Breath Simulator Model BRS 3000.
4. The capsule was then removed from the engine and weighed.
5. The ED filter was removed from the ED cone and weighed.

Assisted Flow Emitted Dose was conducted as follows:

1. The emitted dose filter was weighed and inserted into the emitted dose cone.
2. The prepared Size 00 capsule was inserted into the Size 00 engine. The engine was inserted into straight, rigid tubing which encapsulated the engine. One end of this rigid tubing was connected to the ED cone using a reducer. The other end was connected to flexible tubing which was connected to an in house air supply.
3. Positive airflow from the in-house air supply was supplied at one of the specified flowrates (12.0, 9.6, and 6.4 LPM) for the duration of two minutes in each run.
4. The engine was removed from the tubing, and the capsule was removed from the engine. The capsule was then weighed.

5. The ED filter was removed from the ED cone and weighed.

In the tables accompanying the following experiments, unless otherwise indicated, the following abbreviations have the indicated meanings:

- DOPC: 1,2-di-(9Z-octadecenoyl)-sn-glycero-3-phosphocholine
- DPPC: Dipalmitoylphosphatidylcholine
- DPPE: 1,2-dihexadecanoyl-sn-glycero-3-phosphoethanolamine
- DPPG-Na: 1,2-Dipalmitoyl-sn-glycero-3-phosphoglycerol, sodium salt
- L-leu: Leucine
- n.c.: Not calculated
- n.d.: Not detected
- POPC: 1-palmitoyl-2-oleoyl-sn-glycero-3-phosphocholine
- POPE: 1-palmitoyl-2-oleoyl-sn-glycero-3-phosphoethanolamine
- POPG-Na: 1-palmitoyl-2-oleoyl-sn-glycero-3-phospho-(1'-rac-glycerol) (sodium salt)
- SD-30: Polyglycitol.

PART A: Emitted Dose Testing Under Constant Flow of 3 powder lots at 3 different flow rates. (not according to the claims)

In the first set of experiments, three powders were tested at 3 constant flows in both Size 00 capsules and Size 2 capsules. The three powders were:

- Formulation 7: (40:24:16:18:2) DPPC:POPC:POPG-Na:SD-30:NaCl
- Formulation 8: (40:24:16:18:2) DPPC:POPC:POPG-Na:L-leu:NaCl
- Formulation 13: (56:24:18:2) DPPC:POPG-Na:SD-30:NaCl

Each of these powders was tested at the following constant flow rates for 2 L of total flow, as described above:

- 10 liters per minute airflow for 12 seconds
- 7 liters per minute airflow for 17.1 seconds
- 5 liters per minute airflow for 24 seconds

Two results were measure for each test: Emitted Percent and Percent Emitted on Filter. Emitted Percent (EP) is the percent of the total powder in the capsule that leaves the capsule over the course of the run. It is calculated by subtracting the capsule weight before the run (filled capsule weight) by the capsule weight after the run (emitted capsule weight) and dividing that value by the powder fill weight of the capsule.

Percent Emitted on Filter (PEF) is the percentage of the powder that left the capsule during the run that was on the ED filter at the end of the run. It is calculated by the capsule weight before the run by the capsule weight after the run (emitted capsule weight) and dividing that by the difference of ED filter weight after the run and ED filter weight before the run. Both of these values are reported for each of the runs. Table 68 shows the EP values and PEF values for Formulation 7 at each flowrate, for both capsule sizes:

Table 68 - Formulation 7 Constant Flow ED Test Results

| Flowrate (L/min) | EP Size 2 | PEF Size 2 | EP Size 00 | PEF Size 00 |
|---|---|---|---|---|
| 10 | 63% | 74% | 96% | 83% |
| 7 | 9% | 55% | 93% | 80% |
| 5 | 33% | 57% | 75% | 61% |

Table 69 shows the EP values and PEF values for Formulation 8 at each flowrate, for both capsule sizes:

Table 69 - Formulation 8 Constant Flow ED Test Results

| Flowrate (L/min) | EP Size 2 | PEF Size 2 | EP Size 00* | PEF Size 00* |
|---|---|---|---|---|
| 10 | 97% | 73% | 100% | 82% |
| 7 | 97% | 57% | 59% | 62% |
| 5 | 96% | 42% | 24% | 52% |
| *At 7 L/min and 5 L/min, capsule spinning was not observed for entire duration of run, so tests were re-run. Average value presented; see appendix for individual run results. | | | | |

Table 70 shows the EP values and PEF values for Lot# 224190 at each flowrate, for both capsule sizes:

Table 70 - Formulation 13 Constant Flow ED Test Results

| Flowrate (L/min) | EP Size 2 | PEF Size 2 | EP Size 00 | PEF Size 00 |
|---|---|---|---|---|
| 10 | 94% | 60% | 99% | 86% |
| 7 | 20% | 42% | 92% | 77% |
| 5 | 46% | 35% | 75% | 65% |

PART B -Emitted Dose Breath Simulator - 3 Powder Lots at 3 Breath Profiles.

In this set of experiments, three powders were tested at 3 different breathing profiles in both Size 00 capsules and Size 2 capsules. The three powders were the same as in PART A: (not according to the claims)

- Formulation 7: (40:24:16:18:2) DPPC:POPC:POPG-Na:SD-30:NaCl
- Formulation 8: (40:24:16:18:2) DPPC:POPC:POPG-Na:L-leu:NaCl
- Formulation 13: (56:24:18:2) DPPC:POPG-Na:SD-30:NaCl.

Each of these powders was tested at the breathing profiles outlined below rates for 2 L of total flow, as described in the introduction section:

- 10 L Tidal Volume: 62.5 mL/ breath; 32 breath cycles
- 7 L Tidal Volume: 43.75 mL/ breath; 42 breath cycles
- 5 L Tidal Volume: 31.25 mL/ breath; 64 breath cycles.

For all breathing profiles, inhale time was 0.372 seconds, and exhale time was 1.125 seconds. As described above, two results were measure for each test: Emitted Percent (EP) and Percent Emitted on Filter (PEF). They were calculated as described in PART A.

The tables below show the EP values and PEF values for each of the powders at each breathing profile, for both capsule sizes:

Table 71 - Formulation 7 Breath Simulator ED Test Results

| Flowrate (L/min) | EP Size 2 | PEF Size 2 | EP Size 00 | PEF Size 00 |
|---|---|---|---|---|
| 10 | 95% | 65% | 95% | 78% |
| 7 | 91% | 53% | 96% | 57% |
| 5 | 80% | 47% | 93% | 59% |

Table 72- Formulation 8- Breath Simulator ED Test Results

| Flowrate (L/min) | EP Size 2 | PEF Size 2 | EP Size 00 | PEF Size 00 |
|---|---|---|---|---|
| 10 | 97% | 68% | 99% | 66% |
| 7 | 97% | 58% | 47% | 50% |
| 5 | 65% | 53% | 16% | 42% |

Table 73 Formulation 13 Breath Simulator ED Test Results

| Flowrate (L/min) | EP Size 2 | PEF Size 2 | EP Size 00 | PEF Size 00 |
|---|---|---|---|---|
| 10 | 94% | 74% | 98% | 72% |
| 7 | 83% | 60% | 92% | 60% |
| 5 | 50% | 42% | 80% | 56% |

PART C- Emitted Dose Assisted Flow - 3 Flowrates

In this set of experiments, three powders were tested at 3 assisted air flowrates using only Size 00 capsules. The powder lot used was Formulation 7 (40:24:16:18:2) DPPC:POPC:POPG-Na:SD-30:NaCl. The powder was tested at the assisted air flowrates outlined below rates for 2 mins, as described in PART A:

- 6.4 liters per minute
- 9.6 liters per minute
- 12.0 liters per minute.

As described in PART A, two results were measure for each test: Emitted Percent (EP) and Percent Emitted on Filter (PEF). They were calculated as described in PART A. Table 74 shows the EP values and PEF values for Formulation 7 for each run:

Table 74 - Formulation 7 Assisted Flow ED Test Results

| Flowrate (L/min) | EP Size 00 | PEF Size 00 |
|---|---|---|
| 6.4 | 103% | 22% |
| 9.6 | 102% | 36% |
| 12.0 | 101% | 45% |

### EXAMPLE 6-Surface Activity Measurements.

Surface activity (expressed as surface pressure per area, with surface pressure being inversely related to surface tension) was assessed via the Langmuir trough method. A medium (KN1003) KSV NIMA Langmuir Trough (Biolin Scientific, Paramus, NJ) was used to collect all surface pressure isotherms.

Prior to isotherm measurements, the Willhelmy plate, trough, and barriers were washed and cleaned thoroughly. Briefly, a flame was used to burn residual surfactant off the platinum Willhelmy plate. The trough and barriers were cleaned 3x with 70% Ethanol with scrubbing. Following ethanol washes, the trough and plates were washed using ultrapure Millipore water.

After cleaning all components, the trough was filled barriers 175-190 ml of ultrapure Millipore water. The subphase surface was cleaned of any residual contaminants using a vacuum line and the balance and barrier positions were zeroed. Care was taken to ensure no contaminants were present prior to isotherm measurements.

Surfactant samples were deposited as a dry powder using a vibrating spatula (VWR, Franklin, MA) and allowed to equilibrate at the subphase surface for at least 15 minutes. Surface pressure isotherms were measured at 10-20 mm/min barrier speed.

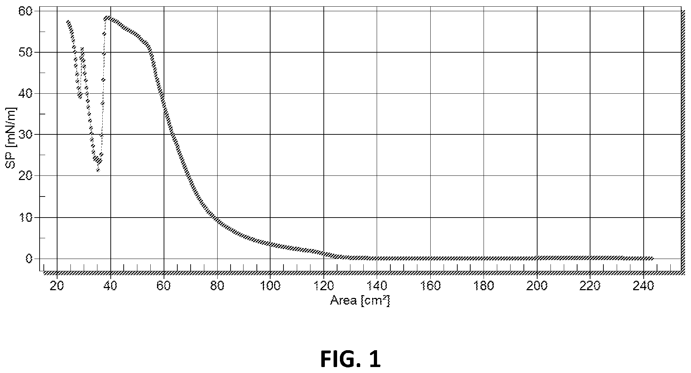

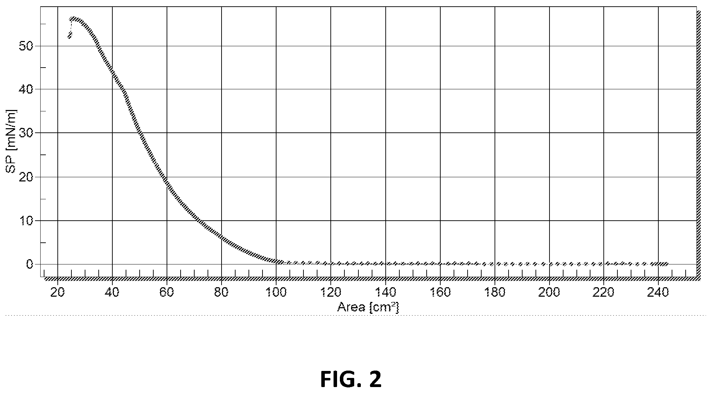

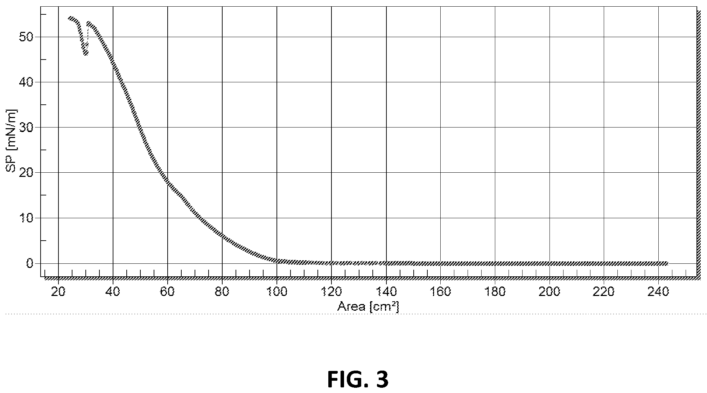

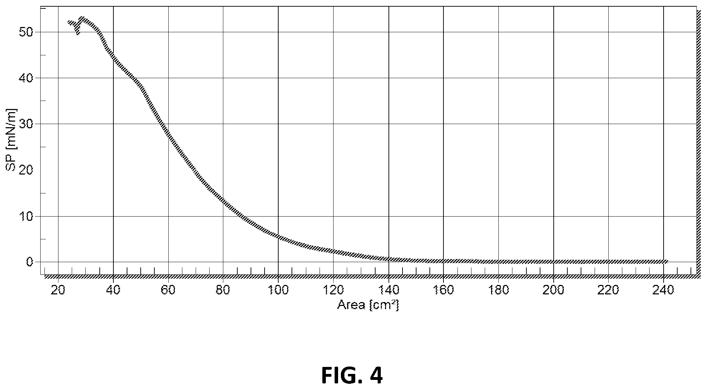

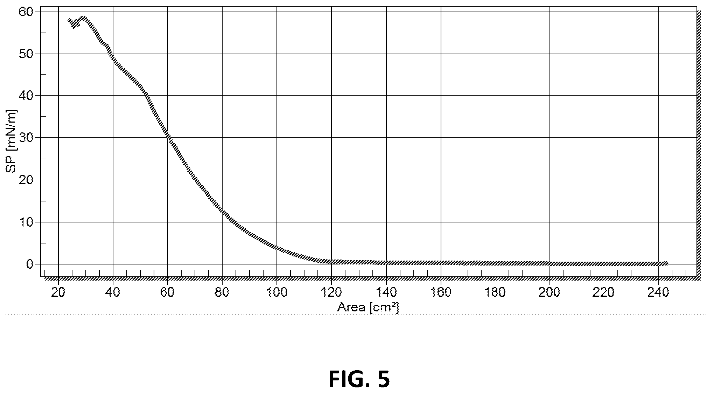

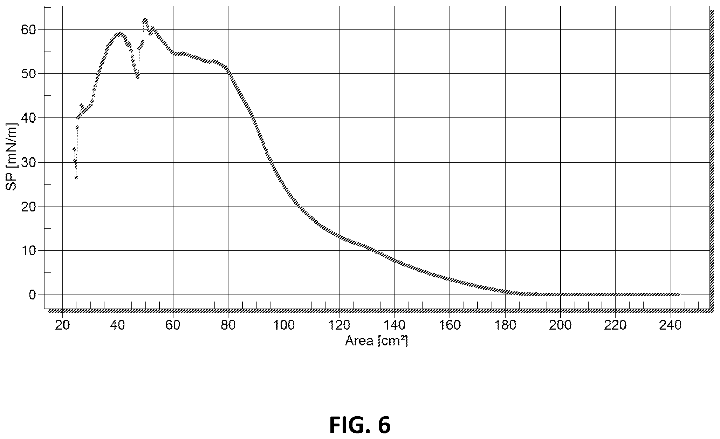

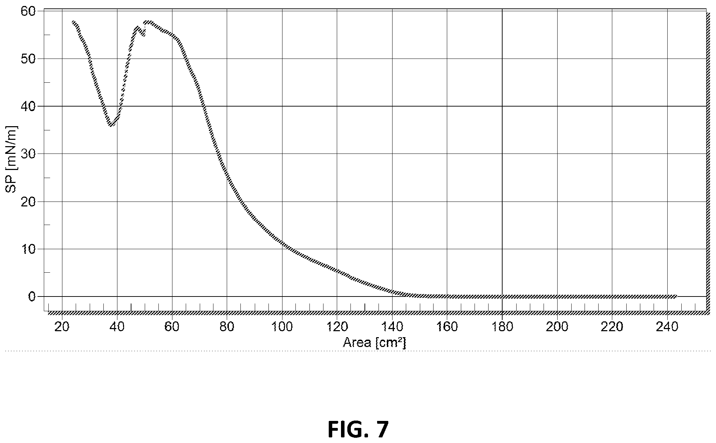

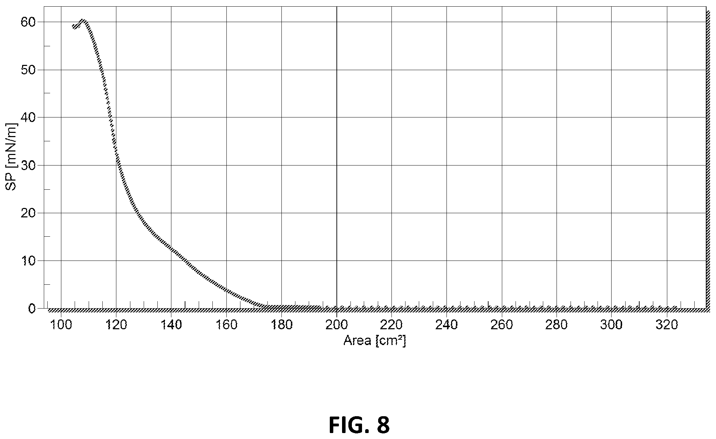

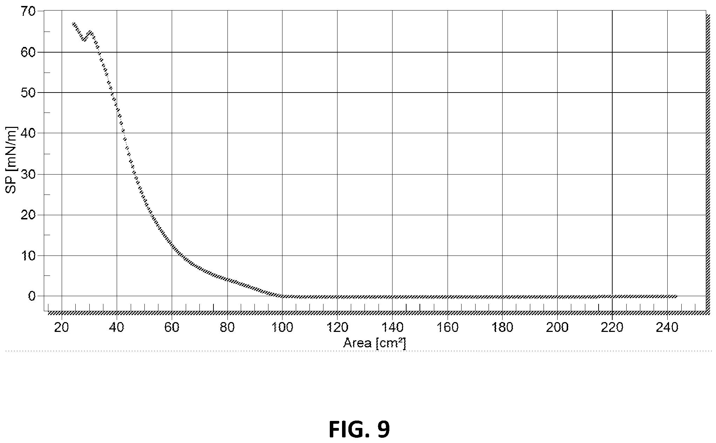

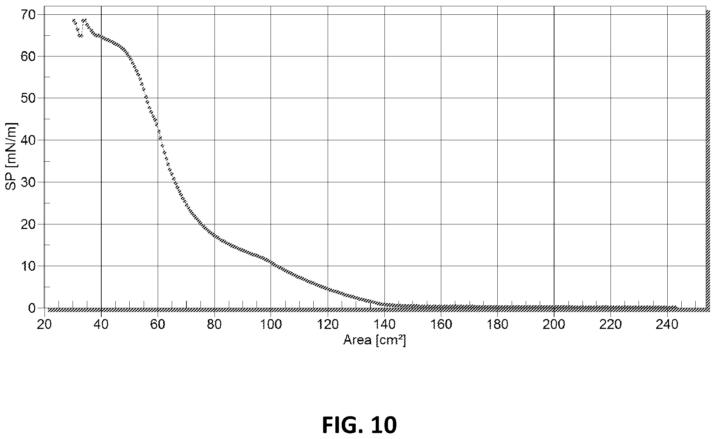

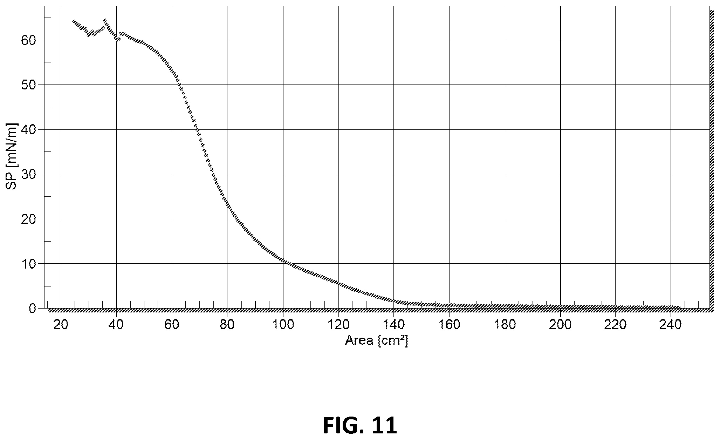

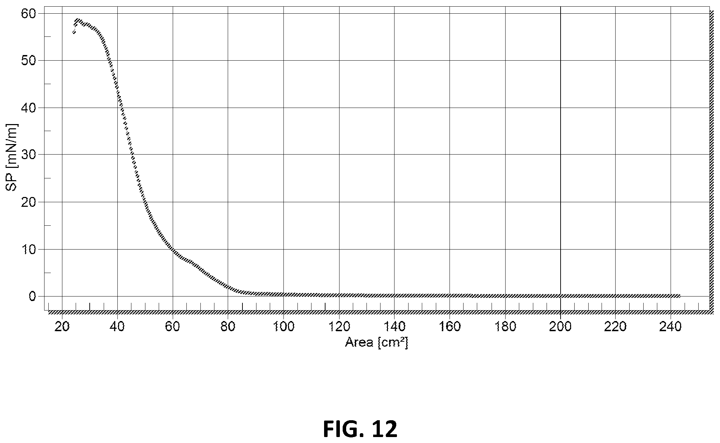

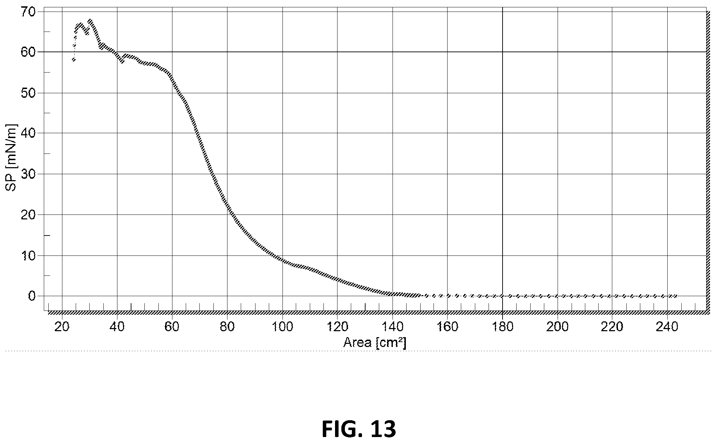

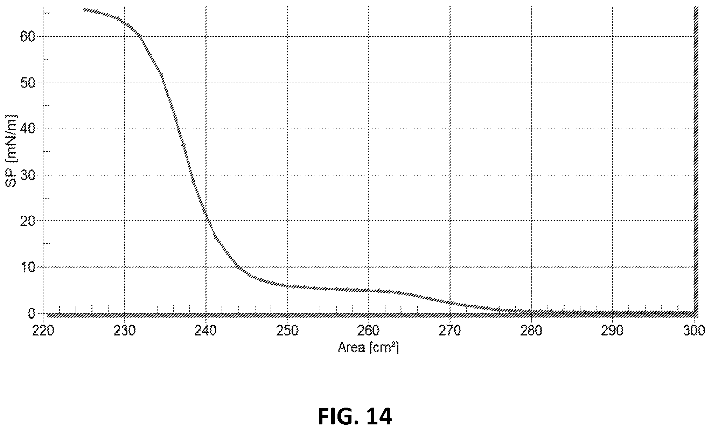

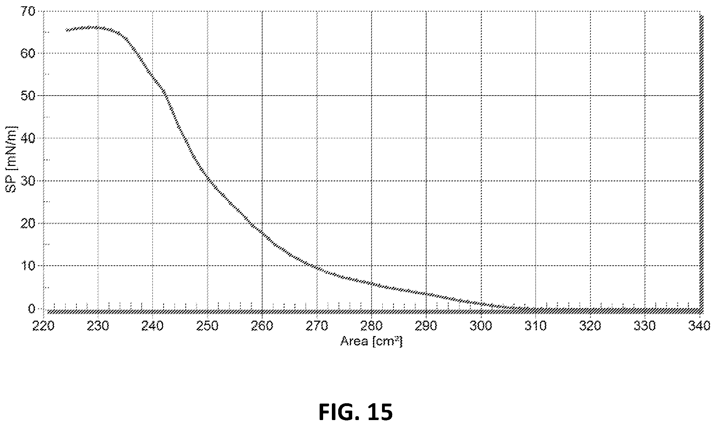

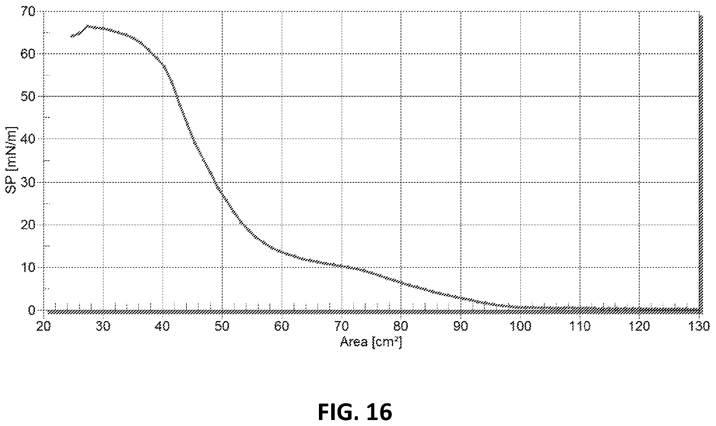

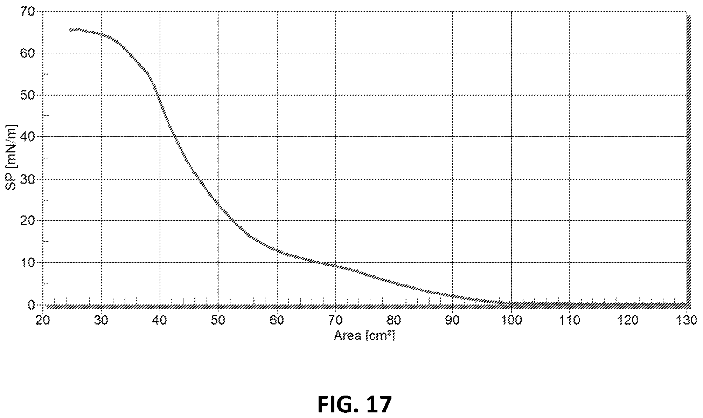

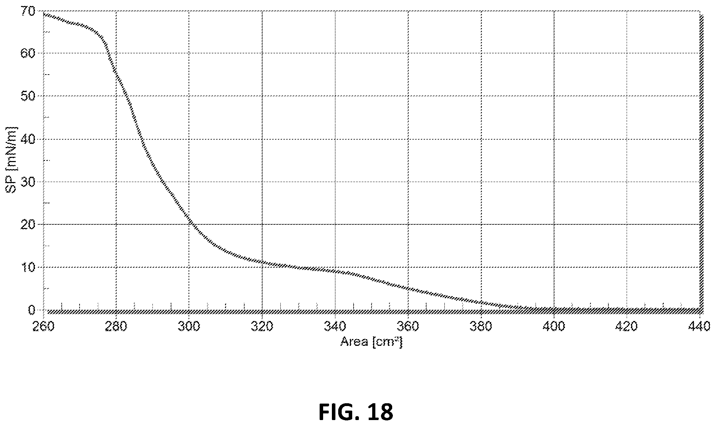

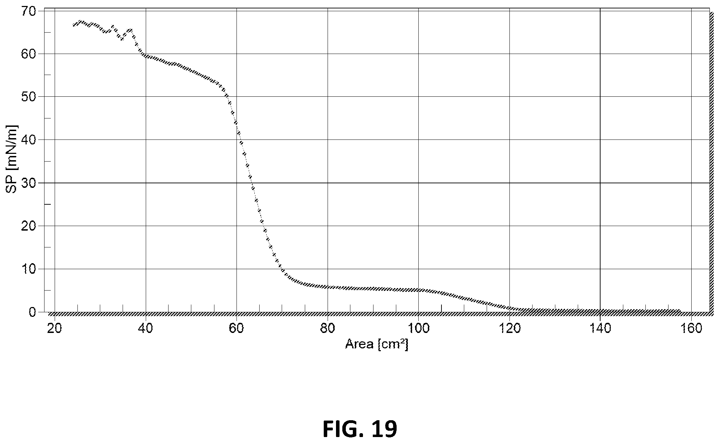

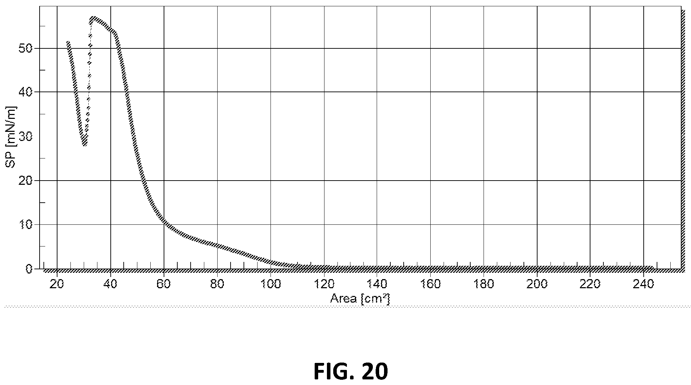

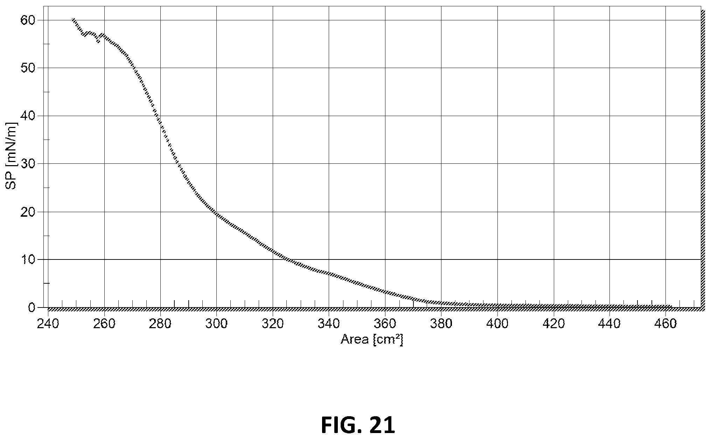

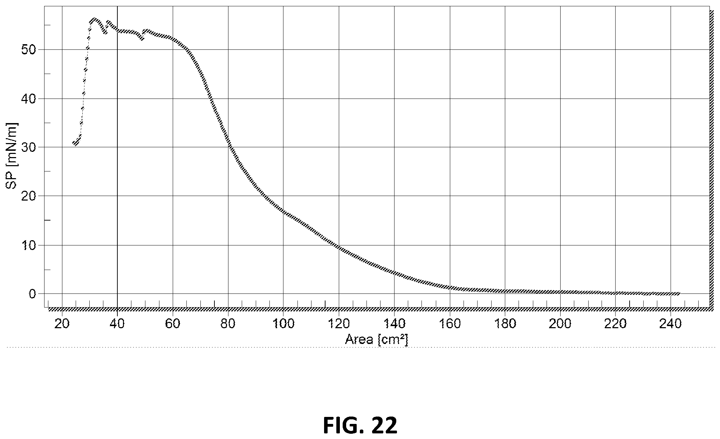

FIGs. 1-22 show isotherms of selected formulations. The isotherms for formulations containing DPPC as the sole phospholipid component in addition to various water-soluble excipients (Figures 14, 19 and 20) all displayed surface pressure versus area characteristics typically seen for pure DPPC monolayers (i.e., with decreasing area per molecule displaying a liquid-expanded phase followed by a broad, constant surface pressure transition from a liquid-expanded to liquid-condensed phase, with the surface pressure increasing sharply after this transition until a collapse pressure is reached at smaller areas per molecule consistent with a condensed, closely-packed solid phase). This behavior indicates that, upon depositing at an air-liquid interface, the phospholipid component of the particles advantageously remains at the surface whereas the water-soluble excipients partition into the aqueous phase. The remaining isotherms obtained for powders containing additional desaturated (DPPG, DPPE) and unsaturated (POPC, POPG, etc.) phospholipids in addition to water-soluble excipient and protein (albumin) components displayed surface pressure versus area behavior more analogous to that typically seen for natural LS mixtures, with a less pronounced and more gradual transition from an expanded to a condensed phase as the area per molecule was decreased. For example, FIG. 15 displays the results obtained for a 56:24:18:2 DPPC:POPG-Na:leucine:NaCl powder (7:3 DPPC:POPG-Na ratio), indicating the presence of the unsaturated phospholipid (POPG-Na) acted to fluidize the DPPC monolayer (consistent with natural LS) while still allowing for a collapse pressure in excess of 65 mN/m. As shown in Figure 16, the substitution of a fraction of the POPG-Na component from this formulation with DPPG-Na (56:8:16:18:2 DPPC:DPPG-Na:POPG-Na:leucine:NaCl) resulted in a slightly less fluidized monolayer with a more discrete transition from an expanded to condensed phase. Thus, these results indicate that the type and ratio of the phospholipid components of the invention disclosed herein can be tailored to provide a desired surface activity.

**EXAMPLE 7-Formulation Development.** (only formulations # 72, 75 and 77 are according to the claims)

Accelerated stability testing was performed on formulations having either a DPPC:POPC ratio of 7:3 with 20% excipients or 30% excipients and a DPPC:POPG-Na ratio of 7:3 with 20% excipients or 30% excipients. Also a comparison was made between the excipients Leucine and SD-30 in each family of formulations comprising a DPPC:POPC ratio of 7:3 and a DPPC:POPG-Na ratio of 7:3. Tables 75-78 summarize the composition of powders, accelerated stability conditions and the aerosol properties. All formulations were prepared as described herein.

Table 75-DPPC:POPC (7:3) with 20% excipients

| Formulation # | | Description | Condition | | gPSD ($\mu$m) | Size 00 FPF <5.6 $\mu$m (%) |
|---|---|---|---|---|---|---|
| 69 | | DPPC:POPC:SD-30:NaCl (56:24:18:2) | t=0 | | 6 | 51 |
| | | | 25° C | 2 wk | | 43 |
| | | | | 1 mth | | 58 |
| | | | 40° C | 2 wk | | 33 |
| | | | | 1 mth | | 42 |
| 70 | | DPPC:POPC:Leu:NaCl (56:24:18:2) | t=0 | | 43 | 45 |
| | | | 40 °C | 2 wk | | 19 |
| | | | | 1 mth | | 49 |

Table 76- DPPC:POPG-Na (7:3) with 20% excipients

| Formula # | Description | Condition | | gPSD ($\mu$m) | Size 00 FPF <5.6 $\mu$m (%) |
|---|---|---|---|---|---|
| 13 | DPPC:POPG-Na:SD-30:NaCl (56:24:18:2) | t=0 | | 10.8 | 37 |
| | | 25 °C | 2 wk | | 57 |
| | | | 1 mth | | 50 |
| | | 40 °C | 2 wk | | 34 |
| | | | 1 mth | | 36 |
| 15 | DPPC:POPG-Na:Leu:NaCl (56:24:18:2) | t=0 | | 10.3 | 46 |
| | | 40 °C | 2 wk | | 22 |
| | | | 1 mth | | 27 |

Table 77- DPPC:POPC (7:3) with 30% excipients

| Formula # | Description | Condition | | gPSD (μm) | Size 00 FPF <5.6 μm (%) |
|---|---|---|---|---|---|
| 67 | DPPC:POPC:SD-30:NaCl (49:21:28:2) | t=0 | | 8.3 | 49 |
| | | 25 °C | 2 wk | | 54 |
| | | | 1 mth | | 54 |
| | | 40 °C | 2 wk | | 1 |
| | | | 1 mth | | 10 |
| 68 | DPPC:POPC:Leu:NaCl DPPC:POPG-a:Leu:NaCl (56:24:18:2) | t=0 | | 3.3 | 47 |
| | | 25 °C | 2 wk | | 45 |
| | | | 1 mth | | 40 |
| | | 40 °C | 2 wk | | 21 |
| | | | 1 mth | | 23 |

Table 78- DPPC:POPG-Na (7:3) with 30% excipients

| Formulation # | Description | Condition | | gPSD (μm) | Size 00 FPF <5.6 μm (%) |
|---|---|---|---|---|---|
| 65 | DPPC:POPG-Na:SD-30:NaCl (49:21:28:2) | t=0 | | 9.8 | 48 |
| | | 25 °C | 2 wk | | 54 |
| | | | 1 mth | 9.9 | 50 |
| | | 40 °C | 2 wk | | 42 |
| | | | 1 mth | 11.1** | 51 |
| | | | 3 mth | | 69 |
| 66 | DPPC:POPG-Na:Leu:NaCl (49:21:28:2) | t=0 | | 9.3 | 48 |
| | | 25 °C | 2 wk | | 46 |
| | | | 1 mth | | 50 |
| | | 40 °C | 2 wk | | 26 |
| | | | 1 mth | | 30 |
| | | | 3 mth | | 28 |

The atomization gas flow rate was varied to determine the effects on the aerosol properties. As the atomization gas flow rate increased, the droplets became smaller resulting in lower particle sizers and higher FPF. Table 79 shows the conditions and aerosol properties of formulations of the invention.

Table 79

| Formulation # | Atomization gas flow rate | Condition | gPSD (μm) | Size 00 FPF <5.6 μm (%) |
|---|---|---|---|---|
| 65 | 20 g/min | t=0 | 9.8 | 48 |
| 65-1 | 30 g/m | t=0 | 5 | 57 |
| 65-2 | 40 g/m | t=0 | 4.2 | 65 |

The data in Tables 75-78 shows that formulations comprising POPG-Na are more stable than formulations comprising POPC. The data also shows that SD-30 excipient stabilizes formulations at accelerated temperatures versus leucine. The data from Table 79 shows that atomization conditions may be optimized to increase FPF delivered to the lungs.

Formulation 65 was chosen for its optimal stability and aerosolization properties to further modify for development of a protein containing formulation having optimum stability and FPF. Initially albumin was used to mimic a surfactant protein such as SP-B.

Accelerated stability testing was conducted with Formula 71 comprising albumin to mimic a surfactant protein such as SP-B. The formulation composition accelerated testing conditions and aerosol properties are shown in Table 80.

Table 80

| Formulation # | Description | Condition | | gPS D (μm) | Size 00 FPF <5.6 μm (%) |
|---|---|---|---|---|---|
| 71 | DPPC:POPG-Na:SD-30:Albumin:NaCl (49:21:25:3:2) | t=0 | | 8.9 | 73 |
| | | 40 °C | 2 wk | | 67 |
| | | | 1 mth | | 68 |
| | | | 3 mth | | 57 |

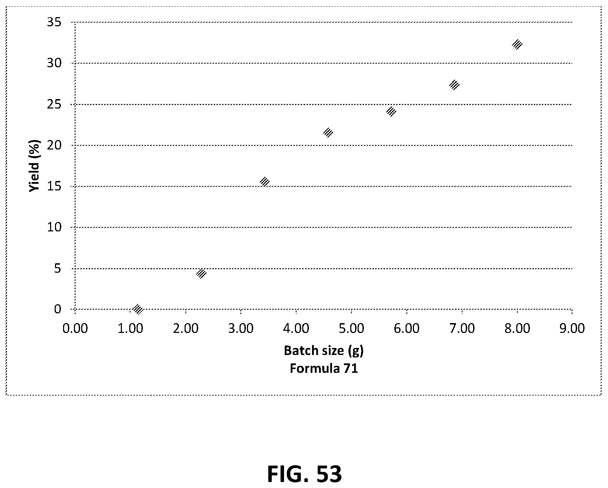

Fig. 53 is a graph showing that the yield percent increases as the batch size (g) increases for Formulation 71.

The good stability at elevated temperatures of Formula 71 indicated that this formulation would be a candidate for insertion of an SP-B surfactant protein and Formula 72 comprising DPPC:POPG-Na:SD-30:SP-B:NaCl (49:21:25:3:2) was prepared as described herein.

The presence of SP-B (specifically SEQ ID NO. 9) in Formula 72 appears to result in FPF gains as the FPF <5.6% relative to total powder was 74% and the FPF<3.4% was 66% indicating that this formulation is capable of aveolar delivery. The bulk density of Formula 72 was approximately 0.02 g/ml. The geometric sizes are shown in Table 81.

Table 81-Geometric Size

| D10 (μm) | 1.0 |
|---|---|
| D50 (μm) | 6.0 |
| D90 (μm) | 14.9 |

SP-B Protein Process Development

An SP-B protein, specifically SEQ ID NO. 9 supplied by Dr. Frans Walthers, LA BioMed, was substituted into albumin-place holder formulations. These powders were spray dried, filled into capsules, and packaged in heat-sealable pouches. Accelerated stability testing was conducted at 40 °C. The stability of these formulations indicate that the powders would be viable for long-term storage. The tables show the aerosol properties and solid state properties of each of the formulas tested.

Table 81

| Formulation # | Description | Condition | | gPSD (um) | Size 00 FPF <5.6um (%) | Size 00 FPF <3.4 um (%) |
|---|---|---|---|---|---|---|
| 72 | DPPC:POPG-Na:SP-B:SD-30:NaCl (49:21:3:25:2) | t=0 | | 6 | 74 | 66 |
| | | 40 C | 2 wk | | 74 | 61 |
| | | | 3 mth | | 73 | 58 |
| | | | 6 mth | | 67 | 52 |

## Table 82

| Formulation# | Description | Condition | gPSD (um) | Size 00 FPF <5.6um (%) | Size 00 FPF <3.4 um (%) |
|---|---|---|---|---|---|
| 72-1 | DPPC:POPG-Na:SP-B:SD-30:NaCl (49:21:3:25:2) | t=0 | 3.3 | 69 | 59 |
| 75-1 | DPPC:POPG-Na:Palmitic Acid:SP-B:SD-30:NaCl (49:21:5:3:20:2) | t=0 | 3.7 | 67 | 47 |
| 75 | DPPC:POPG-Na:Palmitic Acid:SP-B:SD-30:NaCl (49:21:5:3:20:2) | t=0 | 3.2 | 82 | 77 |
| | | t= 1M 40C | | 75 | 62 |
| | | t= 3 M 40 C | | 55 | 39 |
| 77 | DPPC:POPG:POPC:SP-B:SD-30:NaCl (49:21:7:3:18:2) | t=0 | 4.1 | 73 | 65 |
| | | t= 1M 40C | | 60 | 44 |
| | | t= 3 M 40 C | | 53 | 34 |

## Table 83

| Formulation # | Description | Condition | XRPD | TGA-120 (%) | DSC Low T1 ( C) | DSC Low T2 ( C) |
|---|---|---|---|---|---|---|
| 72 | DPPC:POPG-Na:SP-B:SD-30:NaCl (49:21:3:25:2) | t=0 | SC-D | 1.05 | 44.5 | 61.4 |

## Table 84

| Formulation # | Description | Condition | XRPD | TGA-120 (%) | DSC Low T1 ( C) | DSC Low T2 ( C) |
|---|---|---|---|---|---|---|
| 72-1 | DPPC:POPG-Na:SP-B:SD-30:NaCl (49:21:3:25:2) | t=0 | SC-D | 0.78 | 47 | 61.2 |
| 75-1 | DPPC:POPG-Na:Palmitic Acid:SP-B:SD-30:NaCl (49:21:5:3:20:2) | t=0 | SC-D | 0.86 | n.c. | 66 |
| 75 | DPPC:POPG-Na:Palmitic Acid:SP-B:SD-30:NaCl (49:21:5:3:20:2) | t=0 | SC-D | 1 | 48.5 | 66.5 |
| 77 | DPPC:POPG:POPC:SP-B:SD-30:NaCl (49:21:7:3:18:2) | t=0 | SC-D | 1.34 | 53.4 | 62.8 |

Process improvement efforts to increase throughput

Increasing total solids concentration (default value: 2 g/L) would decrease spray drying run time and reduce cost. The increase in total solids concentration did not negatively affect Formula 65-3, DPPC:POPG-Na:SD-30:NaCl (49:21:28:2) but it lowered the aerosol stability of Formula 76, DPPC:POPG-Na:POPC:SD-30:NaCl (49:21:7:21:2). The aerosol properties of the formulations tested are found in the following Tables.

Table 85

| Formulation # | Description | Condition | | gPSD (um) | Size 00 FPF <5.6um (%) | Size 00 FPF <3.4 um (%) |
|---|---|---|---|---|---|---|
| 65-3 | DPPC:POPG-Na:SD-30:NaCl (49:21:28:2) at 4 g/L | t=0 | | 4.5 | 67 | 58 |
| | | 40C | 2wk | | 72 | 61 |
| | | | 1mnth | | 71 | 58 |
| | | | 3mnth | | 66 | 53 |
| 71-1 | DPPC:POPG-Na:SD-30:Albumin:NaCl (49:21:25:3:2) at 4 g/L | t=0 | | 5.1 | 65 | 56 |
| | | 40C | 2wk | | 70 | 58 |
| | | | 1mnth | | 74 | 62 |
| | | | 3mnth | | 67 | 53 |

Table 86

| Formulation # | Description | Condition | XRPD | TGA-120 (%) | DSC | |
|---|---|---|---|---|---|---|
| | | | | | Low T1 (C) | Low T2 (C) |
| 65-3 | DPPC:POPG-Na:SD-30:NaCl (49:21:28:2) at 4 g/L | t=0 | SC-D | 1.22 | 45 | 61.8 |
| 71-1 | DPPC:POPG-Na:SD-30:Albumin:NaCl (49:21:25:3:2) at 4 g/L | t=0 | SC-D | 0.92 | n.c. | 62.8 |

Table 87

| Formulation # | Description | Condition | | Size 00 FPF <5.6um (%) | Size 00 FPF <3.4 um (%) |
|---|---|---|---|---|---|
| 76 | DPPC:POPG-Na:POPC:SD-30:NaCl (49:21:7:21:2) at 4 g/L | t = 0 | | 74 | 62 |
| | | 40C | 2wk | 39 | 28 |
| | | | 1mnth | 34 | 22 |
| | | | 3mnth | 32 | 21 |
| 78 | DPPC:POPG-Na:POPC:Albumin:SD-30:NaCl (49:21:7:3:18:2) at 4 g/L | t = 0 | | 62 | 49 |
| | | 40C | 2wk | 34 | 24 |
| | | | 1mnth | 37 | 26 |
| | | | 3mnth | 29 | 19 |

Accelerated stability testing was conducted with Formulas 73, 74 and 76. The formulation composition accelerated testing conditions and aerosol properties are shown in the Tables below.

Table 88

| Formulation # | Description | Condition | | gPSD (um) | Size 00 FPF <5.6um (%) | Size 00 FPF <3.4 um (%) |
|---|---|---|---|---|---|---|
| 73 | DPPC:POPG-Na:Palmitic Acid:SD-30:NaCl 49:21:5:23:2 | t=0 | | 4.6 | 73 | 56 |
| | | 40C | 2 wk | | 81 | 64 |
| | | | 2 mth | | 72 | 55 |
| | | | 3 mth | | 68 | 54 |
| 74 | DPPC:POPG-Na:Palmitic Acid:Albumin:SD-30:NaCl 49:21:5:3:20:2 | t=0 | | 3.1 | 78 | 62 |
| | | 40C | 2 wk | | 77 | 66 |
| | | | 2 mth | | 70 | 54 |
| | | | 3 mth | | 68 | 53 |

Table 89

| Formulation # | Description | Condition | | gPSD (um) | Size 00 FPF <5.6um (%) | Size 00 FPF <3.4 um (%) |
|---|---|---|---|---|---|---|
| 76 | DPPC:POPG-Na:POPC:SD-30:NaCl (49:21:7:21:2) | | t=0 | 6.2 | 72 | 56 |
| | | 40 C | 2 wk | | 73 | 54 |
| | | | 2 mth | | 67 | 46 |
| | | | 3 mth | | 66 | 49 |
| | | | 6 mth | | 66 | 50 |

**Claims**

1. A respirable, dry powder particle surfactant formulation for pulmonary delivery comprising:

   i) at least 30% DPPC by weight of the particle;
   ii) between 0.1% to 3% NaCl by weight of the particle;
   iii) between 1% to 10% by weight of the particle of a surfactant protein selected from the group consisting of: SP-A, SP-B, SP-C and SP-D or any active fragment thereof, wherein the active fragment contains at least a portion of a lipid associating region and maintains the functionality of the native protein; and
   iv) between 10% to 30% by weight of the particle of a hydrogenated starch hydrolysate (HSH);
   wherein all components of the dry powder particles amount to 100 weight percent; and
   wherein the fine particle fraction less than 3.4 microns (FPF<3.4 $\mu$m) of the powder is 30% or more.

2. The formulation of claim 1, wherein the fine particle fraction less than 3.4 microns (FPF<3.4 $\mu$m) of the powder is 40% or more.

3. The formulation of claim 2, wherein the fine particle fraction less than 3.4 microns (FPF<3.4 $\mu$m) of the powder is 50% or more.

4. The formulation of claim 1, wherein the fine particle fraction less than 3.4 microns (FPF<3.4 $\mu$m) of the powder is 30% to 60%.

5. The formulation of one of claims 1 to 4, further comprising at least one or more of DOPC, POPC, DPPE, DPPG or POPG.

6. The formulation of one of claims 1 to 4, further comprising between 1% to 10% by weight of the particle of a surfactant protein selected from the group consisting of: SEQ ID NOS: 1-21.

7. The formulation of claim 6, wherein the surfactant protein is 5% by weight of the particle and wherein the surfactant protein comprises the amino acid sequence of SEQ ID NO: 9.

8. The formulation of one of claims 1 to 4, wherein the HSH is polyglycitol.

9. The formulation of claim 5, selected from the formulations:

| *Formulation #* | *Description* |
|---|---|
| 72 | DPPC:POPG-Na:SD-30:SP-B:NaCl (49:21:25:3:2) |
| 75 | DPPC:POPG-Na:Palmitic Acid:SP-B:SD-30:NaCl (49:21:5:3:20:2) |
| 77 | DPPC:POPG:POPC:SP-B:SD-30:NaCl (49:21:7:3:18:2) |

wherein the ratios of the components are by weight of the particle.

10. A formulation of claim 5, comprising DPPC and further comprising POPG-Na at a ratio of 7:3 DPPC:POPG-Na.

11. A formulation of one of claims 1 to 4, wherein the surfactant protein is SP-B.

12. A formulation of claim 5, comprising DPPC:POPG-Na:SD-30:SP-B:NaCl (49:21:25:3:2) wherein the ratios of the components are by weight of the particle.

**Patentansprüche**

1. Lungengängige Trockenpulverpartikel-Tensidformulierung zur pulmonalen Zuführung, umfassend:

   i) mindestens 30 Gew.-% DPPC bezogen auf das Partikel;
   ii) zwischen 0,1 Gew.-% und 3 Gew.-% NaCl bezogen auf das Partikel;
   iii) zwischen 1 Gew.-% und 10 Gew.-% an einem Tensidprotein bezogen auf das Partikel, ausgewählt aus der Gruppe bestehend aus: SP-A, SP-B, SP-C und SP-D oder einem beliebigen aktiven Fragment davon, wobei das aktive Fragment mindestens einen Teil einer Lipidassoziierenden Region enthält und die Funktionalität des nativen Proteins beibehält; und
   iv) zwischen 10 Gew.-% und 30 Gew.-% an einem hydrierten Stärkehydrolysat (HSH) bezogen auf das Partikel; wobei alle Komponenten der Trockenpulverpartikel zusammen 100 Gewichtsprozent betragen; und wobei der Anteil an Feinpartikeln, die kleiner als 3,4 Mikrometer (FPF < 3,4 $\mu$m) sind, des Pulvers 30 % oder mehr beträgt.

2. Formulierung nach Anspruch 1, wobei der Anteil an Feinpartikeln des Pulvers, die kleiner als 3,4 Mikrometer (FPF<3,4 $\mu$m) sind, 40 % oder mehr beträgt.

3. Formulierung nach Anspruch 2, wobei der Anteil an Feinpartikeln des Pulvers, die kleiner als 3,4 Mikrometer (FPF<3,4 $\mu$m) sind, 50 % oder mehr beträgt.

4. Formulierung nach Anspruch 1, wobei der Anteil an Feinpartikeln des Pulvers, die kleiner als 3,4 Mikrometer (FPF<3,4 $\mu$m) sind, 30 % bis 60 % beträgt.

5. Formulierung nach einem der Ansprüche 1 bis 4, ferner umfassend mindestens eines oder mehrere aus DOPC, POPC, DPPE, DPPG oder POPG.

6. Formulierung nach einem der Ansprüche 1 bis 4, ferner umfassend zwischen 1 Gew.-% bis 10 Gew.-% an einem Tensidprotein bezogen auf das Partikel, das aus der Gruppe ausgewählt ist, die aus Folgendem besteht: SEQ ID NOS: 1-21.

7. Formulierung nach Anspruch 6, wobei das Tensidprotein 5 Gew.-% bezogen auf das Partikel beträgt und wobei das Tensidprotein die Aminosäuresequenz unter SEQ ID NO: 9 umfasst.

8. Formulierung nach einem der Ansprüche 1 bis 4, wobei es sich bei dem HSH um Polyglycitol handelt.

9. Formulierung nach Anspruch 5, ausgewählt aus den Formulierungen:

| Formulierung Nr. | Beschreibung |
| --- | --- |
| 72 | DPPC:POPG-Na:SD-30:SP-B:NaCl (49:21:25:3:2) |
| 75 | DPPC:POPG-Na:Palmitinsäure:SP-B:SD-30:NaCl (49:21:5:3:20:2) |
| 77 | DPPC:POPG:POPC:SP-B:SD-30:NaCl (49:21:7:3:18:2) |

wobei die Verhältnisse der Komponenten auf das Gewicht des Partikels bezogen sind.

10. Formulierung nach Anspruch 5, umfassend DPPC und ferner umfassend POPG-Na in einem Verhältnis von 7:3 DPPC:POPG-Na.

11. Formulierung nach einem der Ansprüche 1 bis 4, wobei es sich bei dem Tensidprotein um SP-B handelt.

**12.** Formulierung nach Anspruch 5, umfassend DPPC:POPG-Na:SD-30:SP-B:NaCl (49:21:25:3:2), wobei die Verhältnisse der Komponenten auf das Gewicht des Partikels bezogen sind.

**Revendications**

**1.** Formulation respirable de tensioactifs sous forme de particules de poudre sèche pour administration pulmonaire comprenant :

> i) au moins 30 % en poids de DPPC par rapport au poids des particules ;
> ii) entre 0,1 % et 3 % en poids de NaCl par rapport au poids des particules ;
> iii) entre 1 % et 10 % en poids d'une protéine tensioactive par rapport au poids des particules, ladite protéine tensioactive étant choisie dans le groupe constitué par : SP-A, SP-B, SP-C et SP-D ou un quelconque fragment actif de celles-ci, le fragment actif contenant au moins une partie d'une région d'association aux lipides et conservant la fonctionnalité de la protéine native ; et
> iv) entre 10 % et 30 % en poids d'un hydrolysat d'amidon hydrogéné (HSH) par rapport au poids des particules ;
> dans laquelle tous les composants des particules de poudre sèche représentent 100 % en poids ; et
> dans laquelle la fraction des particules fines inférieures à 3,4 micromètres (FPF < 3,4 $\mu$m) de la poudre est supérieure ou égale à 30 %.

**2.** Formulation selon la revendication 1, dans laquelle la fraction des particules fines inférieures à 3,4 micromètres (FPF < 3,4 $\mu$m) de la poudre est supérieure ou égale à 40 %.

**3.** Formulation selon la revendication 2, dans laquelle la fraction des particules fines inférieures à 3,4 micromètres (FPF < 3,4 $\mu$m) de la poudre est supérieure ou égale à 50 %.

**4.** Formulation selon la revendication 1, dans laquelle la fraction des particules fines inférieures à 3,4 micromètres (FPF < 3,4 $\mu$m) de la poudre est de 30 % à 60 %.

**5.** Formulation selon l'une des revendications 1 à 4, comprenant en outre au moins un ou plusieurs de DOPC, POPC, DPPE, DPPG ou POPG.

**6.** Formulation selon l'une des revendications 1 à 4, comprenant en outre entre 1 % à 10 % en poids d'une protéine tensioactive par rapport au poids des particules, ladite protéine tensioactive étant choisie dans le groupe constitué par : les SEQ ID n$^{os}$ : 1-21.

**7.** Formulation selon la revendication 6, dans laquelle la protéine tensioactive représente 5 % en poids par rapport au poids des particules et dans laquelle la protéine tensioactive comprend la séquence d'acides aminés de SEQ ID N° : 9.

**8.** Formulation selon l'une des revendications 1 à 4, dans laquelle le HSH est le polyglycitol.

**9.** Formulation selon la revendication 5, choisie parmi les formulations :

| N° de _formulation_ | _Description_ |
|---|---|
| 72 | DPPC:POPG-Na:SD-30:SP-B:NaCl (49:21:25:3:2) |
| 75 | DPPC:POPG-Na:acide palmitique:SP-B:SD-30:NaCl (49:21:5:3:20:2) |
| 77 | DPPC:POPG:POPC:SP-B:SD-30:NaCl (49:21:7:3:18:2) |

dans laquelle les rapports des composants sont en poids par rapport au poids des particules.

**10.** Formulation selon la revendication 5, comprenant de la DPPC et comprenant en outre du POPG-Na en un rapport DPPC:POPG-Na de 7:3.

**11.** Formulation selon l'une des revendications 1 à 4, dans laquelle la protéine tensioactive est SP-B.

**12.** Formulation selon la revendication 5, comprenant DPPC:POPG-Na:SD-30:SP-B:NaCl (49:21:25:3:2) dans laquelle les rapports des composants sont en poids par rapport au poids des particules.

**FIG. 1**

FIG. 2

FIG. 3

**FIG. 4**

FIG. 5

**FIG. 6**

**FIG. 7**

**FIG. 8**

**FIG. 9**

FIG. 10

FIG. 11

FIG. 12

**FIG. 13**

SP [mN/m]

Area [cm²]

**FIG. 14**

**FIG. 15**

**FIG. 16**

**FIG. 17**

**FIG. 18**

EP 3 474 832 B1

EP 3 474 832 B1

**FIG. 19**

FIG. 20

FIG. 21

**FIG. 22**

FIG. 23

FIG. 24

**FIG. 25**

FIG. 26

FIG. 27

**FIG. 28**

**FIG. 29**

FIG. 30

FIG. 31

FIG. 32

FIG. 33

**FIG. 34**

FIG. 35

FIG. 36

FIG. 37

FIG. 38

FIG. 39

FIG. 40

FIG. 41

**FIG. 42**

**FIG. 43**

**FIG. 44**

FIG. 45

ARCUS-2

FIG. 46

FIG. 47

LFAC-0

FIG. 48

FIG. 49

FIG. 50

FIG. 51

FIG. 52

**FIG. 53**

# EP 3 474 832 B1

## REFERENCES CITED IN THE DESCRIPTION

### Patent documents cited in the description

- US 62354382
- US 62420932
- WO 9726863 A
- US 8563683 B
- US 94687288 B
- US 4995385 A
- US 4069819 A

### Non-patent literature cited in the description

- **TATUSOVA, T. A. et al.** *FEMS Microbiol Lett*, 1999, vol. 174, 187-188
- **KARLIN** ; **ALTSCHUL**. *Proc. Natl. Acad. Sci. USA*, 1990, vol. 87 (6), 2264-8
- **K. MASTERS**. Spray Drying Handbook. John Wiley & Sons, 1984